# EUROPEAN PATENT APPLICATION

(11) **EP 2 100 962 A1**
(43) Date of publication of application: **16.09.2009**
(21) Application number: 08152646.9
(22) Date of filing: 12.03.2008
(51) Int. Cl.: C12N 15/82, A01H 5/00

(54) **Plants having improved resistance to pathogens**

(71) Applicant: Biogemma, 75001 Paris (FR); COMMONWEALTH SCIENTIFIC AND INDUSTRIAL RESEARCH ORGANISATION, Campbell, Australian Capital Territory 2612 (AU)
(72) Inventor: The designation of the inventor has not yet been filed
(74) Representative: Flesselles, Bruno F.G.

(57) **Abstract**

The invention relates to methods for increasing pathogen resistance in plants and decrease the production of mycotoxin in infected plant products by modulation of the polyamine biosynthetic pathways and to plants obtained thereof.

## Description

The invention relates to field of agriculture and to the resistance to disease and the safety of food and feed products of cultivated crops.

*Fusarium* head blight (FHB) is one of the most economically important diseases of wheat world wide. *Fusarium* spp. that cause FHB on wheat are also capable of causing this disease on other plants, and in particular small grain cereals such as barley and many also cause ear rot in maize. Globally, members of the species complex containing *F. graminearum* (teleomorph *Gibberella* zeae) are the most common cause of FHB. Members of the *F. graminearum* species complex together with many other more distantly-related *Fusarium* species, such as *F*. *pseudograminearum* and *F. culmorum,* are all economically important causes of head blight disease in various regions in the world.

Many fungal pathogens in the genus *Fusarium* (teleomorph *Gibberella*) that infect cereals can produce trichothecene mycotoxins during infection.

It has been demonstrated that production of trichothecene mycotoxins such as deoxynivalenol (DON) are important for infection and virulence on cereals. Mutants of *F. graminearum* unable to produce trichothecene toxins show reduced aggressiveness on wheat (Bai et al., 2002, Mycopathologia. 153, 91-98; Jansen et a/., 2005, PNAS. 102, 16892-16897; Proctor et al., 1995, Mol. Plant Microbe Interact. 8, 593-601). Infection by *F. graminearum* occurs at the time of anthesis and is thought to be through the anthers, followed by colonisation of other parts of the floret before entering the vasculature and pith of the rachis from where it spreads up and down the head (reviewed in Trail et al., 2005, Canadian Journal of Plant Pathology. 27, 486-498). Although trichothecene non-producing mutants can cause initial infection, they are unable to spread throughout the head, and their growth is restricted at the rachis node between the rachis and floret (Jansen *et al*., 2005, *op. cit*.). Even if the mechanism of virulence is not fully clear, there is such genetic evidences that the DON production and accumulation in the plant is tightly correlated to the spread resistance and yield loss (Jianga et al., 2006, Crop Sci 46:2590-2597).

In addition to their role in fungal virulence, many trichothecenes, such as DON, nivalenol (NIV) & T2 toxin (produced by *F. sporotrichioides,* a pathogen of maize), are harmful to humans and livestock when consumed in food/feed products derived from infected plants. Thus, the presence of trichothecene toxins in grain is coming under increasing scrutiny in both Europe and the United States of America, where legally enforceable limits on contamination by trichothecenes such as DON in food products are now in place. In mammalian systems, DON has been shown to act as a potent translational inhibitor that lead to the alteration of the synthesis of the proteins and causes acute symptoms such as vomiting and feed refusal in livestock and also chronic symptoms such as growth retardation, reduced ovarian function and immunosuppression. Processes that can reduce mycotoxin production in grain products would have important utility in improving animal feed and human food safety.

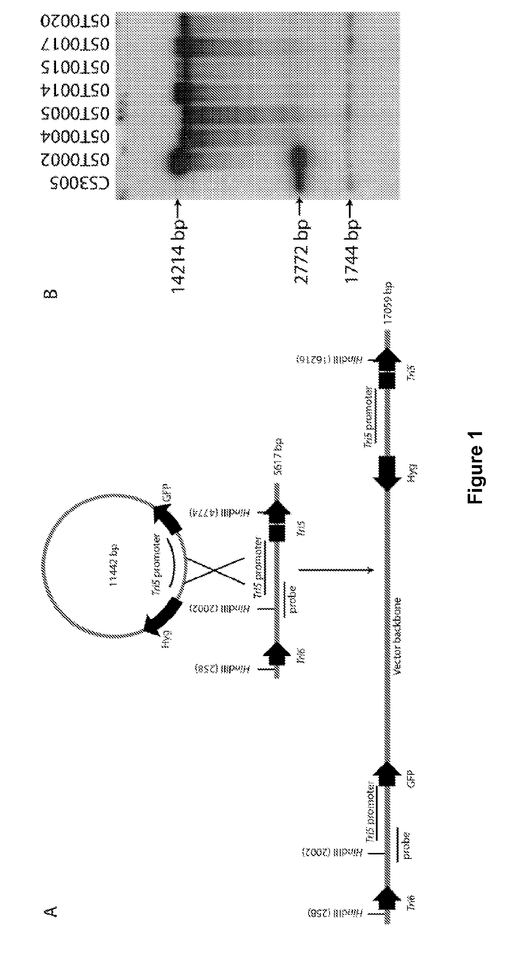

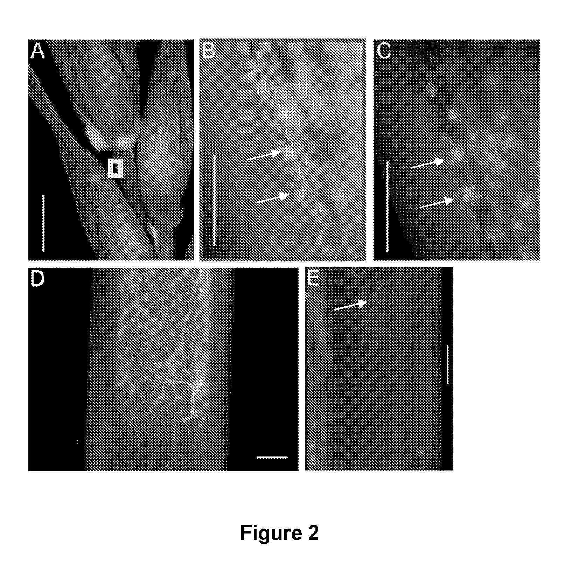

This invention is based initially on the identification of compounds that can induce the expression of the *TRI5* gene of *F. graminearum.* This gene encodes the trichodiene synthase enzyme that catalyses the conversion of farnesyl pyrophosphate to trichodiene and this is the first committed step from primary metabolism to the secondary metabolism pathway of all trichothecene mycotoxin biosynthesis. TRI5 is conserved in all trichothecene producing *Fusarium* spp. including *F. graminearum*, *F. pseudograminearum*, *F. culmorum*, *F. sporotrichioides, F. crookwellense, F. poae, F. tricinctum, F.acuminatum, F. sambucinum, F. venenatum* as well as *Myrothecium roridum* (Fekete et al., 1997, Mycopathologia. 138, 91-97; Hohn and Desjardins, 1992, Mol Plant Microbe Interact. 5, 249-56; Proctor et al., 1995, Mol. Plant Microbe Interact. 8, 593-601; Royer et al., 1999, Fungal Genetics and Biology. 28, 68-78; Trapp et al., 1998, Molecular and General Genetics. 257, 421-432). Its expression is regulated by the pathway specific transcription factor *TRI6* (Proctor et al., 1995, Applied and Environmental Microbiology. 61, 1923-1930) and regulatory protein *TRI10* (Peplow et al., 2003, Applied and Environmental Microbiology. 69, 2731-2736). The *TRI6* binding sites are conserved upstream of all *TRI5* homologous genes in Fusaria. To create an easy assay for the expression of this gene the regulatory promoter region of the *TRI5* gene was fused to the gene encoding the jellyfish green fluorescent protein and this new chimerical gene introduced into *F. graminearum* as described in Figure 1. The transformed fungal strains showed fluorescence during the infection of wheat heads at times when the trichothecene mycotoxins are known to be produced. An example of this fluorescence is shown in Figure 2. Thus these new transgenic strains appear to act as 'reporter strains' for *TRI5* gene expression and toxin production and were subsequently used to identify compounds that induce toxin production.

The invention is based on the demonstration that the production of trichothecene mycotoxins including DON is stimulated *in vitro* by a range of metabolites, most of which contain either one or multiple amine groups.

During *in vitro* growth of the GFP-expressing *F. graminearum* strain, induction of DON was observed when such identified inducers, including putrescine, arginine and cadaverine, were applied in the media, alone or combined with others. Moreover, during the infection of wheat heads by *Fusarium graminearum,* induction was observed for several of the identified inducers including putrescine, arginine, cadaverine and lysine. In addition, *Fusarium* infection of wheat heads led to the induction of wheat gene transcripts encoding enzymes responsible for the synthesis of arginine and polyamines which in turn are strong inducers of trichothecene synthesis in the fungus. Induced cereal gene sequences include those encoding arginine decarboxylase, ornithine decarboxylase and argininosuccinate synthase, in particular.

The invention describes that the production of trichothecene toxins can be reduced by the selection of plants that accumulate lower amounts of the mycotoxin inducers, in particular in the parts of the plant where the production of mycotoxins takes place. Specifically, the reduction of inducing compounds in the host plant can be obtained *via* the manipulation of host genes, e.g., arginine decarboxylase, ornithine decarboxylase and argininosuccinate synthase, that are responsible for the production of amine/polyamine inducers of mycotoxins. These manipulations can lead to increased resistance to infection, reduced toxin level in plants, and thus to improved food and feed safety, and increased yield via plant resistance to *Fusarium* pathogens.

Alternatively, it is possible to reduce the level of infection by *Fusarium* and/or the level of mycotoxins by reducing the availability of inducers to the fungi. These can be reduced by limiting their synthesis by the plant, increasing their degradation, or by using competitor chemicals that would saturate the *Fusarium* amine sensing or transport systems.

Several patent applications describe methods that result in detoxification of trichothecenes (WO200060061, WO2005021740 or WO200020573), identify an insensitive molecular target site (US2002088022 or WO200105976) or make cells less sensitive to trichothecene toxins (WO2006070815 or US2003165991).

The inventors have now identified potent stimulators of trichothecene production by *Fusarium* pathogens. The use of this information makes it possible to reduce the production of DON or other trichothecenes by the manipulation of plant gene expression in plant tissues.

US2005026953 indicates that it is possible to reduce the production of DON by the application of a chemical inhibitor alkenylpiperidine. The use of gene technology to reduce the induction of other mycotoxins by other pathogenic fungi such as the silencing of plant lipoxygenases to reduce induction of aflatoxins by *Aspergillus spp.* is described in WO9726364. However, the production of aflatoxins by *Aspergilli* and the production of trichothecenes by *Fusaria* do not appear to be related either in their biosynthetic pathways, their regulation, their effect on humans and their role in plant pathogenesis.

The production of trichothecenes by *F. graminearum* appears to be somewhat specific to host infection. Autoclaved grain and axenic cultures are often used for the analysis of trichothecene production. However, the levels of accumulated toxin observed in most *in vitro* culture conditions are orders of magnitude lower than those measured during the infection of live wheat heads and other tissues (Mudge et al., 2006, Physiological and Molecular Plant Pathology. 69, 73-85; Voigt et al., 2007, European Journal of Plant Pathology. 117, 1-12). In some instances, complex sequential nutritional regimes have been reported to partially induce DON production in culture (Miller and Blackwell, 1986, Canadian Journal of Botany. 64, 1-5), but factors responsible for high level induction of DON have not been identified. There is also evidence that H₂O₂, a product of the host oxidative burst sometimes elicited during plant infection, may enhance DON production in culture (Ponts et al., 2006, Fems Microbiology Letters. 258, 102-107) but the levels of DON induced by H₂O₂ in vitro are still very low when compared to those that accumulate in infected plants.

To find *in vitro* conditions that allow trichothecene production to levels similar to *in planta*, the inventors have used novel approaches such as nutritional profiling. The use of large scale nutritional profiling had never been applied to study mycotoxin production. This approach was used to identify a series of molecules that can induce trichothecene production *in vitro.* Many of the identified inducing compounds were either precursors or products in the polyamine biosynthetic pathway of plants and this pathway appears indeed to be activated early on during the infection of wheat heads.

The polyamine biosynthetic pathway is well known, as several patent applications propose to manipulate it in transgenic plants. One could cite JP2006020601, JP2006067947, WO2006057306, US2006225154, US2004086985, JP2003199582, WO2005018307, JP2006325554, W0200109358, W0200218547, W0200111062, WO200223974, W02003084314.

However, these patent applications relate, for most of them to abiotic stress tolerance and none of them cite nor suggest that such a strategy can be used to obtain a reduction in mycotoxin production or an increased resistance to trichothecene-producing *Fusarium* pathogens.

The invention thus relates to a method for inducing resistance and/or tolerance to a trichothecene-producing pathogen infection in a plant, comprising the step of inhibiting or downregulating expression of an endogenous polyamine synthesis gene in said plant, said inhibition or downregulation resulting in said plant to have increased resistance to said trichothecene-producing pathogen infection as compared to a nearly isogenic plant in which said endogenous polyamine synthesis gene is not inhibited or downregulated.

The invention also relates to a method for reducing trichothecene level in a plant infected by a trichothecene-producing pathogen, comprising the step of inhibiting or downregulating expression of an endogenous polyamine synthesis gene in said plant, said inhibition or downregulation resulting in said plant to have reduced trichothecene level when infected by trichothecene-producing pathogen as compared to a near isogenic plant in which said endogenous polyamine synthesis gene is not inhibited or downregulated.

The invention can also be performed by inhibiting or downregulating expression of an endogenous polyamine synthesis gene in a part of a plant including rachis, stem, spikelet or spike, thereby inducing resistance to a trichothecene-producing pathogen infection or reducing trichothecene level in said part of a plant.

In a preferred embodiment, said trichothecene is deoxynivalenol (DON). In a preferred embodiment, said trichothecene-producing pathogen is a *Fusarium* from *Fusarium* spp, and is in particular chosen among *F. graminearum, F. pseudograminearum, F. sporotrichioides, F. poae, F. sambucinum, F. venenatum, F. culmorum, F. cerealis, and F acuminatum.* In another embodiment, said trichothecene-producing pathogen is *Myrothecium roridum.*

In the context of the present invention, nearly isogenic plants are plant whose genome is at least 90 % identical, more preferably at least 95 % identical. In a preferred embodiment, the genomes of near isogenic plants only differ at one or a few specific loci. In the context of the present invention, said loci are the loci corresponding to the genes involved in the polyamine biosynthetic pathway. Inhibition or down-regulation of these genes will make the plant more resistant to infection by the pathogen.

The present invention makes it possible to induce resistance and/or tolerance in a plant that does not have, *per se,* a level of resistance and/or tolerance to pathogen infection. It makes also it possible to increase resistance and/or tolerance to pathogen infection in plants which already have a basal level of resistance and/or tolerance.

As used herein, the terms "altering", "increasing", "increased", "reducing", "down-regulating", "reduced", "inhibited" or the like are considered relative terms, *i.e.* in comparison with the wild-type or unaltered state, preferably in nearly isogenic plants. The "level of a protein" refers to the amount of a particular protein, which may be measured by any means known in the art such as, for example, Western blot analysis or other immunological means. The "level of an enzyme activity" refers to the amount of a particular enzyme measured in an enzyme assay. It would be appreciated that the level of activity of an enzyme might be altered in a mutant but not the expression level (amount) of the protein itself. Conversely, the amount of protein might be altered but the activity would the same if a more or less active protein is produced. Reductions in both amount and activity are also possible such as, for example, when a gene encoding the enzyme is inactivated. In certain embodiments, the reduction in the level of protein or activity is by at least 40% or by at least 60% compared to the level of protein or activity in the unmodified plant, or by at least 75%, at least 90% or at least 95%.

Resistance and/or tolerance to pathogen infection represents the ability for the plant to limit the growth of the pathogen and/or to limit the production of trichothecenes by the pathogen during infection. Resistance and/or tolerance can be monitored by a reduction in necrosis, bleaching and spread of the fungus (growth), or measurement of lower amount of mycotoxins including DON and derivates by usual methods in the infected plant. As will be seen, these effects are usually observed.

In the context of the invention, a polyamine synthesis gene (also mentioned as gene of the polyamine biosynthetic pathway) is a gene coding for an enzyme that is active during the synthesis of polyamines, and/or makes use or produces a polyamine, such as L-ornithine, putrescine, L-arginine, agmatine, citrulline, N-carbamoylputrescine, spermine and spermidine, including genes involved in the synthesis of precursors thereof. This term may also include, by extension, a gene coding for an enzyme that is active during the synthesis of, and/or makes use or produces cadaverine, such as lysine decarboxylase. Regulation of these latter genes is also part of this invention. Polyamines are small, ubiquitous, nitrogenous compounds that have been implicated in a variety of stress responses in plants (Bajaj et al., (1999) Mol. Breeding 5, 493-503). Their biosynthesis pathway is well known and described in the literature.

In a specific embodiment, said gene is chosen in the group consisting of arginine decarboxylase (ADC, EC 4.1.1.19), ornithine decarboxylase (ODC, EC 4.1.1.17), agmatinase (EC 3.5.3.11), argininosuccinate synthase (ASS, EC 6.4.3.1), and argininosuccinate lyase (EC 4.3.2.1). It can also be a lysine decarboxylase, producing cadaverine from lysine.

In a preferred embodiment, said plant is a monocotyledon. In the most preferred embodiment, said plant is a cereal, in particular chosen in the group consisting of wheat, maize, rice, rye, barley, triticale, sorghum and oat. In a specific embodiment, said cereal is wheat. In another embodiment, said cereal is maize. In yet another embodiment, said cereal is rice.

In the context of the present invention, a gene of the polyamine biosynthetic pathway is down-regulated or inhibited. Consequently, the production of the enzyme is decreased, as compared to a wild-type plant, and the eventual production of the polyamines is also decreased.

There are numerous ways, known in the art, to downregulate or inhibit a specific gene in a plant.

The inhibition of a gene involved in the biosynthesis pathway of polyamines can be obtained by any means known in the art. Thus, one can proceed with the insertional mutagenesis by inserting a transposable element or a T-DNA within the gene to be inhibited. One can also make a physical or chemical mutagenesis, in particular by the use of EMS, X-rays or ultraviolet.

The mutated plants are then screened for example by PCR, using primers located in the target gene. However, one can also use other methods of screening, such as Southern Blots or screening through the AIMS method described in WO9927085 (to detect insertions), using probes which are specific of the target genes. One can also use methods for detecting point mutations or small insertions / deletions with specific endonucleases (such as *Ce*/I, or *Endo*l) as described in W02006010646.

In another embodiment, the inhibition or down-regulation is achieved by transforming the plant with a vector containing a sense or antisense construct. Both methods are known to allow inhibition or down-regulation of a target gene. It also uses the method of RNA interference (RNAi), which is particularly effective for the inhibition of genes in plants. This method is well known to the skilled person and consists of the transformation of the plant with a construct producing after transcription, a double duplex RNA, one of the strands of which being complementary to the mRNA of the gene target.

Genetic engineering approaches to altering, in particular specifically reducing, gene activity in plants such as wheat are well known in the art. These methods include the introduction of gene constructs for expression of a suitable antisense molecule that is complementary to the RNA of the target gene and can hybridize with it. Antisense molecules are thought to interfere with the translation or processing or stability of the RNA of the target gene, thereby inactivating expression of the gene. Methods of devising antisense sequences are well known in the art and examples of these can be found in US5190131, EP467349, EP223399 and EP240208, which are incorporated herein by reference. The use of antisense methods in plants has been reviewed by Bourque (1995, Plant Science 105,125-149) and Senior (1998, Biotechnology and Genetic Engineering Reviews 15,79-119). Bourque lists a large number of examples of gene inactivation using antisense sequences in plant systems. She also states that attaining 100% inhibition of an enzyme activity may not be necessary as partial inhibition will more than likely result in measurable change in the system. Senior states that antisense methods are now a very well established technique for manipulating gene expression in plants.

Antisense molecules for ODC, ADC or other polyamine biosynthesis or modification genes can be based on the sequences given therein or derived from homologous DNA or mRNA sequences obtained from other species, for example barley. The antisense sequences may correspond to all or part of the transcripts of any of these genes or for sequences that affect control over their expression, for example their splicing. The antisense sequence may correspond to the targeted coding region of the target gene, or the 5'-untranslated region (UTR) or the 3'-UTR or combination of these. It may be complementary in part to intron sequences, which may be spliced out during or after transcription, preferably only to exon sequences of the target gene. In view of the generally greater divergence of the UTRs, targeting these regions provides greater specificity of gene inhibition. In particular embodiments, the length of the antisense sequence is at least 19 contiguous nucleotides, at least 50, at least 100, at least 200, at least 500 or at least 1000 nucleotides corresponding to the complement of the gene RNA sequence. The full-length sequence complementary to the entire gene transcript may be used. In a particular embodiment, the length of the antisense sequence is 100-2000 nucleotides. In further embodiments, the degree of sequence identity of the antisense sequence to the complement of the targeted transcript is at least 85%, at least 90% or 95-100%. The antisense RNA molecule may of course comprise unrelated sequences which may function to stabilize the molecule.

Another molecular biological approach that may be used is co-suppression. The mechanism of co-suppression is not well understood but is thought to involve post- transcriptional gene silencing (PTGS) and in that regard may be very similar to many examples of antisense suppression. It involves introducing an extra copy of a gene or a fragment thereof into a plant in the sense orientation with respect to a promoter for its expression. The size of the sense fragment, its correspondence to target gene regions, and its degree of sequence identity to the target gene are as for the antisense sequences described above. In some instances the additional copy of the gene sequence interferes with the expression of the target plant gene. Reference is made to WO9720936 and EP465572 for methods of implementing co-suppression approaches.

A further method that might be employed to introduce genetic variation into the wheat plant is duplex or double stranded RNA mediated gene silencing (RNAi). In this method a DNA is introduced that directs the synthesis of an at least partly double stranded RNA product(s) with homology to the target gene to be inactivated. The DNA therefore comprises both sense and antisense sequences that, when transcribed into RNA, can hybridize to form the double-stranded RNA region. In a preferred embodiment, the sense and antisense sequences are separated by a spacer region that comprises an intron which, when transcribed into RNA, is spliced out. This arrangement has been shown to result in a higher efficiency of gene silencing. The double-stranded region may comprise one or two RNA molecules, transcribed from either one DNA region or two. The presence of the double stranded molecule triggers a response from an endogenous plant system that destroys both the double stranded RNA and also the homologous RNA transcript from the target plant gene, efficiently reducing or eliminating the activity of the target gene. Reference is made to AU99/29514 and WO9953050 for methods of implementing this technique. In particular embodiments, the length of the sense and antisense sequences that hybridise are at least 19 contiguous nucleotides, at least 30, at least 50, at least 100, at least 200, at least 500 or at least 1000 nucleotides. The full length sequence corresponding to the entire gene transcript may be used. In a particular embodiment, the lengths are in the range 100-2000 nucleotides. In further embodiments, the degree of sequence identity of the sense and antisense sequences to the targeted transcript is at least 85%, at least 90% or 95-100%. The RNA molecule may of course comprise unrelated sequences which may function to stabilize the molecule. The RNA molecule may be expressed under the control of a RNA polymerase II or RNA polymerase III promoter.

The double-stranded RNA molecule may also comprise sequences from more than one gene, joined together, and thereby target multiple genes.

This RNAi method is particularly well suited in wheat. Indeed, due to the hexaploid nature of wheat, it may thus be difficult to identify mutants in all three genomes for a specific gene. The RNAi technology makes it possible to design a construct that would be directed against a region of the target gene, that is both specific of the target gene (for example ADC or ODC), and similar among the copies of the gene on each genome. Alternatively, as indicated above, a RNAi construct containing sequences from all three genes of the genomes can also be used, which makes it possible to inhibit the expression of these genes while transforming the plant with only one construct.

The RNAi method is also well suited for other plants such as maize, barley in rice. Indeed, when multiple genes coding for an enzyme are present in the genome of these plants (for example on different chromosomes), or only an EST (expressed sequence tag) is available to the person skilled in the art, this person can easily design an RNAi vector that will inhibit said gene. Indeed, as indicated above, there is no need of the full sequence of a gene to be able to successfully inhibit it with the RNAi method. Furthermore, due to the proximity of some of the cereal genomes (rice and maize, wheat and barley, for example), it may also be possible to successfully inhibit a gene in an organism, while using a RNAi vector comprising a sequence from another organism.

Note that ESTs can routinely be obtained from a given organism by using sequence information from another organism, an alignment program such as BLAST and subprograms blastn or tblastx, and the information present in databases (such as GeneBank, EMBL or TIGR).

There is thus no need to have the full sequences of the genes for each organism to be able to practice the invention (inhibit the expression of the genes) in different organisms.

The genetic variation responsible for the desired inactivation of gene expression in wheat may comprise a nucleic acid molecule encoding one or more ribozymes. Ribozymes are RNA molecules with enzymatic or catalytic function that can cleave other RNA molecules at specific sites defined by one or often two hybridizing sequences. The cleavage of the RNA inactivates the expression of the target gene. The ribozymes may also act as an antisense molecule, which may contribute to the gene inactivation. The ribozymes contain one or more catalytic domains, preferably of the hammerhead or hairpin type, between the hybridizing sequences. Other ribozyme motifs may be used including RNAseP, Group I or II introns, and hepatitis delta virus types. Reference is made to EP 321201 and US 6,221,661. The use of ribozymes to inactivate genes in transgenic plants has been demonstrated.

Another method that might be employed to alter in particular and specifically to reduce gene activity in plants such as wheat comprises use a transcription factor. The inhibition or down-regulation is achieved by transforming the plant with a vector containing a sense or antisense construct including a transcription factor that would repress the activity of the targeted genes.

The invention also provides isolated nucleic acid molecules comprising RNA or DNA, preferably DNA, which encode the gene-inhibiting molecule. In certain embodiments, the nucleic acid molecules encode antisense, sense (co-suppression), double-stranded RNA or ribozyme molecules which target the polyamine biosynthetic gene sequence and which inactivate its expression in the plant. The invention also provides genetic constructs comprising or encoding the isolated nucleic acid molecule, comprising one or more regulatory elements such as promoters, enhancers and transcription termination or polyadenylation sequences. Such elements are well known in the art. The genetic constructs may also comprise intron sequences that aid expression of the transgene in plants, particularly in monocotyledonous plants such as wheat. The term "intron" is used in its normal sense as meaning a genetic segment that is transcribed but does not encode protein and which is spliced out of an RNA before translation.

Introns may be incorporated in a 5'-UTR or a coding region if the transgene encodes a translated product, or anywhere in the transcribed region if it does not.

The invention further provides vectors, for example plasmid vectors, comprising the genetic constructs. The term "vector" includes an expression vector, being capable of *in vitro* or *in vivo* expression, and a transformation vector, capable of being transferred from one cell or organism to another. The vectors comprise sequences that provide for replication in cells, for example in prokaryotic cells such as *E.coli* or *Agrobacterium.* In a particular embodiment, the vector is a binary vector comprising a T-DNA sequence, defined by at least one T-DNA border sequence that can be introduced into the plant cells.

The invention further provides cells comprising the vectors, for example *Agrobacterium* or plant cells which may be regenerable cells such as the cells of the scutellum of immature embryos. Alternatively, the cells may be transformed plant cells comprising the transgene.

In a specific embodiment, said genetic construct comprises a promoter that is induced by the pathogen infection. Using such a promoter would ensure that the construct inducing the downregulation / inhibition of the target gene would only be translated after infection by the pathogen, therefore allowing the plant to produce and use the polyamine during its normal growth. As demonstrated below, the genes of the polyamine biosynthetic pathway are induced upon *Fusarium* infection. Their native promoters are therefore good candidates for such a purpose.

Alternatively, those derived from known pathogen inducible genes of cereals described elsewhere (Golkari et al., 2007, Plant Biotechnol J. 5(1):38-49; Kong et a/., 2007, Genome 50: 1038-1048; Boddu et al., 2006, Mol. Plant-Microbe Interact. 19: 407-417; Pritsch et al., 2000, Mol. Plant-Microbe Interact. 13: 159-169; Pritsch et al., 2001, Physiol. Mol. Plant Pathol. 58: 1-12; Bernardo et al., 2007, Functional & Integrative Genomics 7: 69-77) may be used. Other examples of pathogen-inducible promoters include those from pathogenesis-related proteins (PR proteins), which are induced following infection by a pathogen; e.g., PR proteins, SAR proteins, beta-1,3-glucanase, chitinase, and the like (Redolfi et al. (1983) Neth. J. Plant Pathol. 89:245-254; Uknes et al. (1992) Plant Cell 4:645-656; Van Loon (1985) Plant Mol. Virol. 4:111-116; WO9943819). Of particular interest is the inducible promoter for the maize An2 gene whose expression is induced by the pathogen *Fusarium graminearum* (see Harris et al., Plant Molecular Biology (2005) 59:881-894), the inducible promoter for the maize PR1 gene whose expression is induced by many pathogens including *Fusarium* sp (US20010025380 and Cordero et al., (1992) Physiological and Molecular Plant pathology 41:189-200). One also can also cite the AoPRT-L promoter described in WO9966057.

Interesting promoters are also the ones that have capacity to be induced locally at or near the site of pathogen infection (Che et al., (1996) Plant Journal 10:955:966; Warner et al., (1993) Plant Journal 3:191-201; Marineau et al. (1987) Plant Mol. Biol. 9:335-342; Matton et al. (1989) Molecular Plant-Microbe Interactions 2:325-331; Somsisch et al. (1986) Proc. Natl. Acad. Sci. USA 83:2427-2430; Somsisch et al. (1988) Mol. Gen. Genet. 2:93-98; Yang (1996) Proc. Natl. Acad. Sci. USA 93:14972-14977. Zhang et al. (1994) Proc. Natl. Acad. Sci. USA 91:2507-2511; Siebertz et al. (1989) Plant Cell 1:961-968; US 5750386).

A wound-inducible promoter may also be considered in the context of the invention, as pathogens may find entry into plants through wounds or insect damage. Such wound-inducible promoters include promoters of the potato proteinase inhibitor pin II gene (Ryan (1990) Ann. Rev. Phytopath. 28:425-449; Duan et al. (1996) Nature Biotechnology 14:494-498), wun1 and wun2 (US 5428148), win1 and win2 (Stanford et al. (1989) Mol. Gen. Genet. 215:200-208), systemin (McGurl et al. (1992) Science 225:1570-1573), WIP1 (Rohmeier et al. (1993) Plant Mol. Biol. 22:783-792; Eckelkamp et al. (1993) FEBS Letters 323:73-76), and MPI gene (Corderok et al. (1994) Plant J. 6(2):141-150).

In another embodiment, said genetic construct comprises a promoter that is inducible by a chemical (for review, see Moore et al., 2006, Plant J. 45:651-83, Padidam M. 2003, Curr Opin Plant Biol. Apr;6(2):169-77, Wang et al., 2003, Transgenic Res. 2003 Oct;12(5):529-40 and Zuo and Chua 2000, Curr Opin Biotechnol. Apr;11(2):146-51). Some examples of couples of chemically inducible systems and chemical inducer used in plants are, the alcA promoter from *A*. *nidulans*, inducible by ethanol (Roslan et al., 2001, Plant J. Oct;28(2):225-35) or the ecdysone receptor from *C*. *fumiferana*, inducible by an ecdysone agonist (Koo et al., 2004, Plant J. Feb;37(3):439-48). In this case, said chemical shall be applied to the plant upon *Fusarium* infection. This would induce inhibition and/or downregulation of the targeted genes for the polyamine biosynthesis and hence, control of the pathogen infection.

In another embodiment, the promoter is tissue specific and particularly induces expression in the rachis, spikelets and/or stem cavity.

In a preferred embodiment, said cereal is rice and said gene is chosen among the genes having a sequence represented by any of SEQ ID N° 1, SEQ ID N° 2 (these two sequences being ADC), SEQ ID N° 14, SEQ ID N° 15, SEQ ID N° 16 (these three sequences being ODC), SEQ ID N° 23 and SEQ ID N° 24 (these two sequences being ASS).

In another preferred embodiment, said cereal is wheat and said gene is chosen among the genes having a sequence represented by any of SEQ ID N° 3, SEQ ID N° 4, SEQ ID N° 5, SEQ ID N° 6, SEQ ID N° 7, SEQ ID N° 8, SEQ ID N° 9, SEQ ID N° 10 (these eight sequences being ADC), SEQ ID N° 17, SEQ ID N° 18, SEQ ID N° 19, SEQ ID N° 20, SEQ ID N° 21 (these five sequences being ODC), SEQ ID N° 25, SEQ ID N° 26 and SEQ ID N° 27 (these three sequences being ASS).

In another preferred embodiment, said cereal is barley and said gene is chosen among the genes having a sequence represented by any of SEQ ID N° 11, SEQ ID N° 12, SEQ ID N° 13 (these three sequences being ADC), SEQ ID N° 22 (this sequence being ODC), and SEQ ID N° 28 (this sequence being ASS).

In another preferred embodiment, said cereal is maize and said gene is chosen among the genes having a sequence represented by any of SEQ ID N° 29, SEQ ID N° 55, SEQ ID N° 56, SEQ ID N° 57 (these four sequences being ODC), SEQ ID N° 58, SEQ ID N° 59, SEQ ID N° 60, SEQ ID N° 61, SEQ ID N° 62, SEQ ID N° 63, SEQ ID N° 64, SEQ ID N° 65 (these eight sequences being ADC), SEQ ID N° 66, SEQ ID N° 67, SEQ ID N° 68, SEQ ID N° 69, SEQ ID N° 70, and SEQ ID N° 71 (these six sequences being ASS).

The invention also relates to a plant preferably a cereal presenting an inhibition or downregulation of the expression of an endogenous polyamine synthesis gene.

In a first embodiment, said cereal is rice and said endogenous polyamine synthesis gene is an ADC chosen among SEQ ID N° 1 and SEQ ID N° 2.

In another embodiment, said cereal is rice and said endogenous polyamine synthesis gene is an ODC chosen among SEQ ID N° 14, SEQ ID N° 15 and SEQ ID N° 16.

In another embodiment, said cereal is rice and said endogenous polyamine synthesis gene is an ASS chosen among SEQ ID N° 23 and SEQ ID N° 24.

In another embodiment, said cereal is wheat and said endogenous polyamine synthesis gene is an ADC chosen among EQ ID N° 3, SEQ ID N° 4 SEQ ID N° 5, SEQ ID N° 6, SEQ ID N° 7, SEQ ID N° 8, SEQ ID N° 9 and SEQ ID N° 10.

In another embodiment, said cereal is wheat and said endogenous polyamine synthesis gene is an ODC chosen among SEQ ID N° 17, SEQ ID N° 18, SEQ ID N° 19, SEQ ID N° 20 and SEQ ID N° 21.

In another embodiment, said cereal is wheat and said endogenous polyamine synthesis gene is an ASS chosen among SEQ ID N° 25, SEQ ID N° 26 and SEQ ID N° 27.

In a first embodiment, said cereal is barley and said endogenous polyamine synthesis gene is an ADC chosen among SEQ ID N° 11, SEQ ID N° 12 and SEQ ID N° 13.

In another embodiment, said cereal is barley and said endogenous polyamine synthesis gene is an ODC and is represented by SEQ ID N° 22.

In another embodiment, said cereal is barley and said endogenous polyamine synthesis gene is an ASS represented by SEQ ID N° 28.

In a first embodiment, said cereal is maize and said endogenous polyamine synthesis gene is an ADC chosen among SEQ ID N° 58, SEQ ID N° 59, SEQ ID N° 60, SEQ ID N° 61, SEQ ID N° 62, SEQ ID N° 63, SEQ ID N° 64 and SEQ ID N° 65.

In another embodiment, said cereal is maize and said endogenous polyamine synthesis gene is an ODC chosen among SEQ ID N° 29, SEQ ID N° 55, SEQ ID N° 56 and SEQ ID N° 57.

In another embodiment, said cereal is maize and said endogenous polyamine synthesis gene is an ASS chosen among SEQ ID N° 66, SEQ ID N° 67, SEQ ID N° 68, SEQ ID N° 69, SEQ ID N° 70, and SEQ ID N° 71.

Inhibition or downregulation of an endogenous polyamine synthesis gene may be achieved by the methods described above. It is preferred when said plant is such that the inhibition or downregulation of the endogenous polyamine synthesis gene is not permanent but may be induced by an external stimulus such as a pathogen infection or application of a chemical.

In particular, the invention relates to a transgenic plant containing a transgene comprising a genetic construct encoding a gene-inhibiting molecule such as described above (RNAi, antisense, co-suppression, ribozyme, and the like). This gene-inhibiting molecule is under the control of a promoter which may be inducible as described above.

In a specific embodiment, the plant is such that inhibition or downregulation of the endogenous polyamine synthesis gene is induced by the pathogen infection and in particular by *Fusarium* infection.

In another specific embodiment, said inhibition or downregulation is restricted to the rachis, the spikelets and/or the stem cavity. This may be achieved by use of specific promoters of said tissues of the plant.

The invention also relates to a cell of a plant, preferably a cell of a plant, or a part of a plant. Spikelets and seeds and a cell of a plant are among preferred part of plants according to the invention. Preferably, said plant is a cereal.

Transgenic plants are obtained by traditional methods for genetic transformation of plants and can be obtained by any one of the techniques known to one skilled in the art : methods of direct transfer of genes such as direct microinjection into plant embryoids, vacuum infiltration (Bechtold et al. 1993, C R Acad Sci III. 316(10) : 1194-1199) or electroporation (Chupeau et al., 1989, Biotechnology vol.7: pp. 503-508) or the bombardment by gun of particules covered with the plasmidic DNA of interest (Fromm et al., 1990, Nature, 319(6056):791-3 ; Finer et al., 1992, Plant Cell Report 11 : 323-328). Agrobacterium mediated transformation methods may also be used *Agrobacterium tumefaciens*, in particular according to the method described in the article by An et al., (1986, Plant Physiology, 81 :86-91), or *Agrobacterium rhizogenes,* in particular according to the method described in the article by Guerche et al., (1987, Biochimie. Jun-Jul;69(6-7):621-8). According to a preferred mode, it is possible to use the method described by Ishida et al., (1996, Nature Biotechnol. 14, 745-50) for the transformation of maize. According to another method, the wheat is transformed according to the method described in WO 00/63398.

In another embodiment, the invention relates to a method for reducing trichothecene production by a trichothecene-producing pathogen, in particular a *Fusarium,* comprising the step of limiting concentration of polyamines and/or polyamine amino acid precursors accessible to said pathogen, in particular said *Fusarium* during its growth. This method is particularly useful *in vivo*, and can also be performed *in vitro*, by using a growth medium deficient in said polyamines and/or polyamine amino acid precursors. It is to be noted that limiting the concentration of polyamines and/or polyamine amino acid precursors accessible to said pathogen, in particular said *Fusarium*, during its growth will also limit the ability of said pathogen, in particular said *Fusarium*, to grow if no other source of both carbon and nitrogen is available.

Indeed, the inventors have demonstrated that the presence of these compounds, and in particular putrescine and L-arginine is higher in infected plants, and that these compounds induce trichothecene production by the pathogens. Other interesting compounds are guanine, citrulline, ornithine, agmatine, cadaverine, N-amylamine, ethanolamine, β-phenylethylamine, tyramine, L-methionine, L-lysine and D-glucosamine.

Alternatively, the invention also relates to a method for inducing trichothecene production by a DON-producing pathogen, in particular a *Fusarium,* comprising the step of making polyamines and/or polyamine amino acid precursors accessible to said pathogen, in particular said *Fusarium,* during its growth. This method is useful *in vitro*, by adding polyamines and/or polyamine amino acid precursors in the growth medium. This makes it possible to improve the yield of mycotoxin production by the fungi.

When performed *in vivo*, limitation of the concentration of polyamines and/or polyamine amino acid precursors accessible to said pathogen, in particular said *Fusarium*, during its growth may be performed by growing said pathogen, in particular said *Fusarium*, on a plant presenting an inhibition or downregulation of the expression of an endogenous polyamine synthesis gene. Said plant is described above, and both the spread of said pathogen, in particular said *Fusarium*, and its ability to produce mycotoxins will be reduced when on said plant, as compared to when the pathogen is on a nearly isogenic plant which does not present said inhibition or down-regulation.

In another embodiment, one can perform the method of the invention *in planta* by applying an inhibitor of an enzyme of the polyamine biosynthetic pathway to said cereal, in the presence of said pathogen, in particular said *Fusarium*. One could use an inhibitor as described in US 4376116, an inhibitor of ornithine decarboxylase, such as α-monofluoromethylornithine (DFMO), or an S-adenosylmethionine decarboxylase (SAMDC) inhibitor (Matters et al, Clin. and Exp. Metastasis, 22(4), 2005 , 331-339(9), Mehrotra et al, Contraception, 57(1), 1998, 55-60).

Reduction of the level of the available polyamines and/or polyamine amino acid precursors may also be obtained by the use of another non-pathogenic microorganism that would use said compounds competitively with said pathogen, in particular said *Fusarium.*

In another embodiment, it is possible to reduce the production of trichothecene by a DON-producing pathogen, in particular a *Fusarium*, by adjunction of a compound that would abolish the effect of said polyamines and polyamine amino acid precursors to said pathogen, in particular said *Fusarium* (induction of trichothecene production and increased growth). Such compounds may in particular be chosen among nitrate and cysteine.

In another embodiment, an inhibitor of a polyamine/amino acid transporter of a *Fusarium* is used.

### FIGURES

**Figure 1****:** Insertion of *TRI5-GFP* at the *TRI5* locus. (A) Vector, target locus and predicted resultant genomic locus. (B) Southern analysis of transformants for site specific integration of the construct. Genomic DNA was cut with *Hind*III. The position of the probe used is shown in A. All transformants except 05T0002 show homologous integration at the *TRI5* locus.
**Figure 2****:** *In planta* expression of GFP driven by the *TRI5* promoter (transformant 05T0020). A, bright field of florets. The upper shown floret was inoculated. Hyphae can be seen emerging from the spikelet rachis junction (bar = 5 mm). B, bright field close up (bar = 0.5 mm) of boxed region in A, showing hyphae emerging from the rachis. C, fluorescent image of B, showing strong GFP expression in the hyphae emerging from the rachis (bar = 0.5 mm). D, mycelia in the pith cavity of a stem cross section about 5 cm below stem head junction (bar = 0.5 mm). E, hyphae at the very front on the infection about 5 cm below the stem head junction as the fungus goes down the stem expressing GFP (bar = 0.5 mm). A, B, C cultivar INDIA44 4 dpi. D, E cultivar Kennedy 14 dpi.

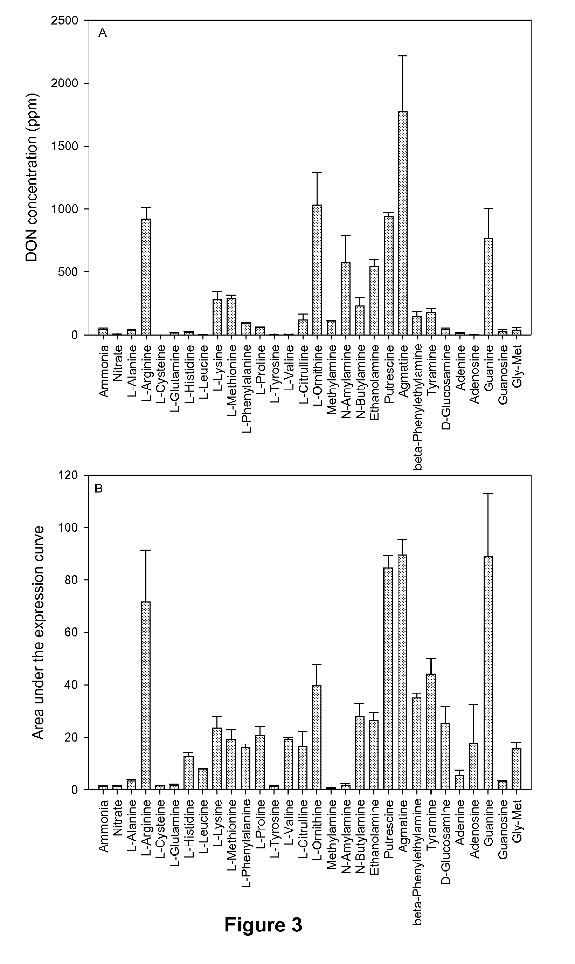

**Figure 3****:** A, DON production in selected nitrogen sources from the phenotype microarray plates with the wild type isolate CS3005. Toxin was measured after seven days culture. Data are the mean of three biological replicates. Error bars are standard errors of the mean. B, Total area under the expression curve for the same nitrogen sources as in A. The correlation coefficient (R2) between the DON concentration and total area under the expression curve was 0.66.

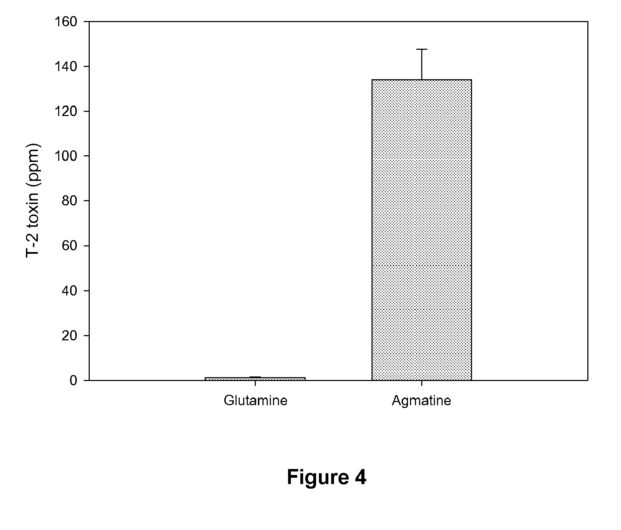

**Figure 4****:** T-2 toxin production by *F. sporotrichioides* in defined media containing either glutamine or agmatine as the nitrogen source. Cultures were grown for 7 days at 28°C in the dark.

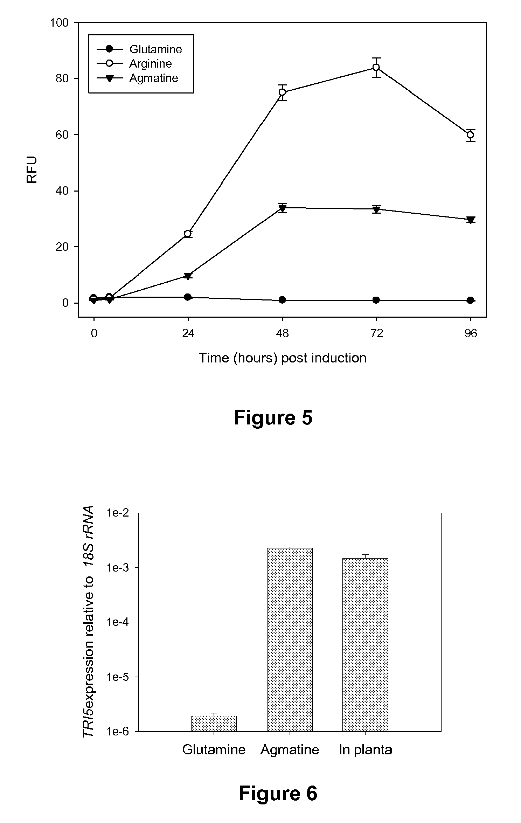

**Figure 5****:** Fluorescence from the *TRI5*-*GFP* isolate in cultures grown for 2 days in glutamine followed by either glutamine, arginine or agmatine addition to the media to a final equivalent concentration of 5 mM. Values are the mean relative fluorescence units (RFU) of twelve biological replicates, with error bars representing the standard error of the mean.
**Figure 6****:** *TRI5* gene expression relative to fungal *18S rRNA* in culture 4 days post-inoculation (dpi) in glutamine or agmatine as nitrogen sources and *in planta.* In planta expression is 4 dpi (the maximum level observed in a seven day time course - data not shown). Error bars represent the standard error of the mean of four biological replicates.

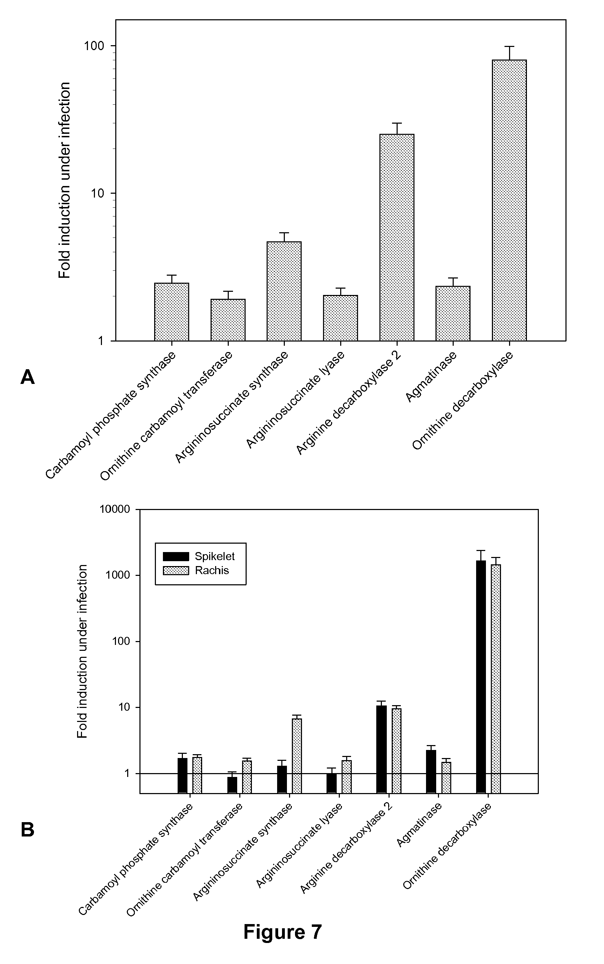

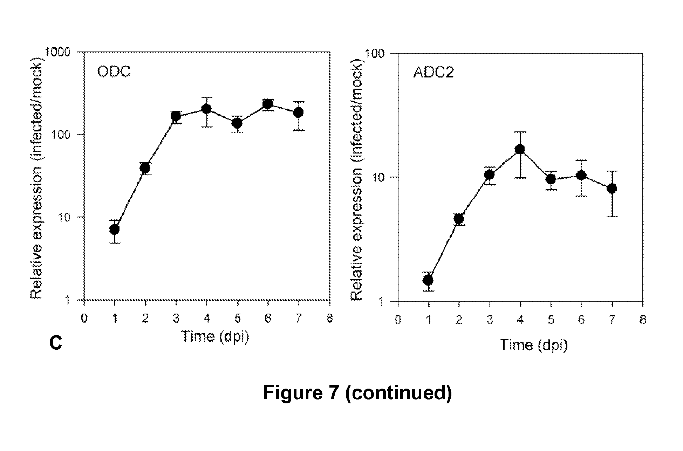

**Figure 7****:** A, B and C are independent experiments conducted at different times. A, data for gene expression in whole heads at 5 days post-inoculation. B, data for gene expression in the rachis and the spikelets separately at 5 days post-inoculation. C, Time course induction of ODC and *ADC2* gene expression during infection of wheat by *F. graminearum.* Data in A and B is the mean of eight biological reps for both mock and infected samples. Data in C is the mean of four biological reps for both mock and infected samples. Error bars are the standard error of the mean.

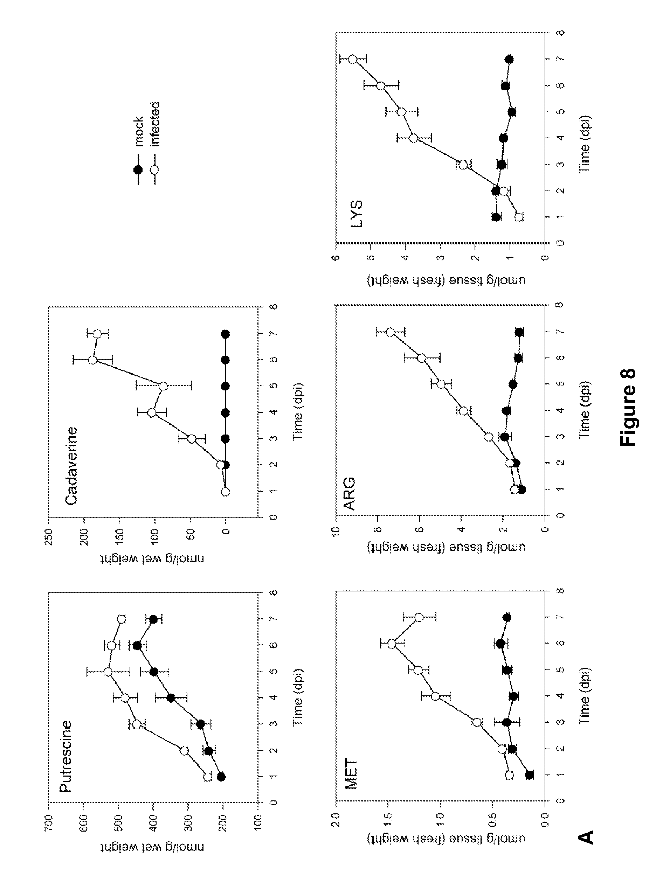

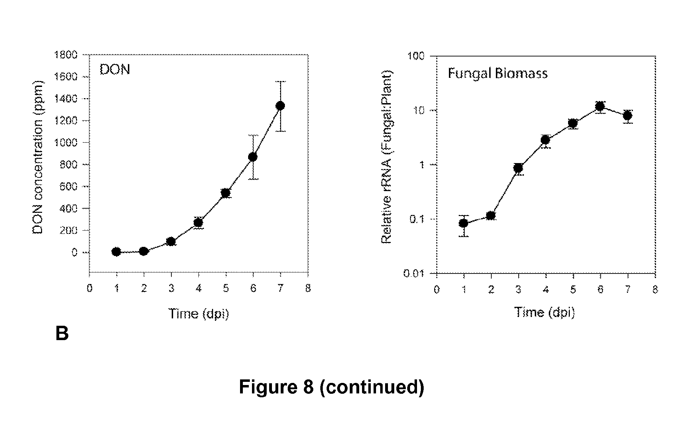

**Figure 8****:** (A) Induction of free amino acid and polyamine inducers during Fusarium Head Blight disease of wheat (MET = methionine, ARG = arginine, LYS = lysine). (B) Increases in both deoxynivalenol (DON) and fungal biomass during Fusarium Head Blight disease of wheat in same experiment as described in (A). All data points consist of four biological replicates with error bars representing the standard error of the mean.

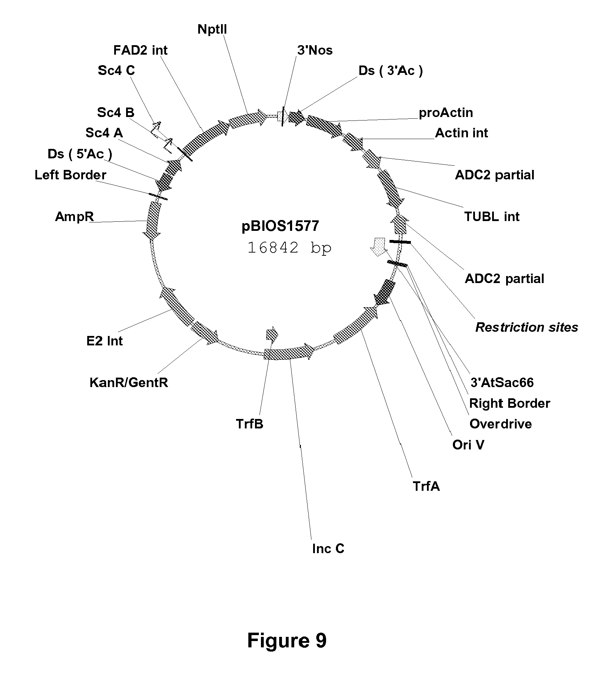

**Figure 9****:** plasmid pBIOS 1577. a partial sequence of the wheat *ADC* gene (*ADC2* partial) has been introduced in sense and antisense orientation in the vector, separated by the rice tubulin intron (TUBL int), under the control of the Actin promoter and intron (proActin and Actin int).

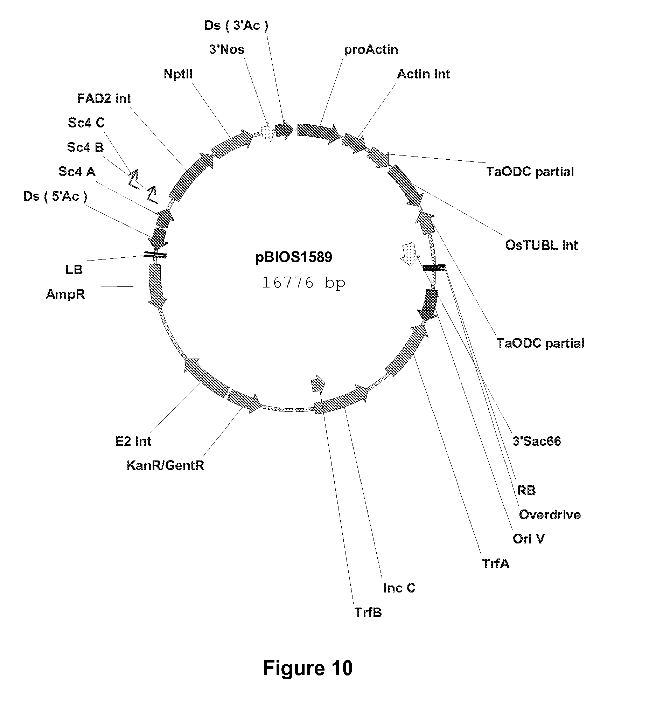

**Figure 10****:** plasmid pBIOS 1589. a partial sequence of the wheat *ODC* gene (*TaODC* partial) has been introduced in sense and antisense orientation in the vector, separated by the rice tubulin intron (*OsTUBL* int), under the control of the Actin promoter and intron (proActin and Actin int).

### EXAMPLES

The invention is based initially on the identification of compounds that can induce the expression of the *TRI5* gene of *Fusarium graminearum.* This gene encodes the trichodiene synthase enzyme that catalyses the conversion of farnesyl pyrophosphate to trichodiene and this is the first committed step from primary metabolism to the secondary metabolism pathway of trichothecene mycotoxin synthesis. TRI5 is conserved in all trichothecene producing *Fusarium* spp. including *F. graminearum*, *F. sporotrichioides, F. poae, F. sambucinum, F. venenatum* as well as *Myrothecium roridum* (Fekete et al., 1997, Mycopathologia. 138, 91-97; Hohn and Desjardins, 1992, Mol Plant Microbe Interact. 5, 249-56; Proctor et al., 1995, Mol. Plant Microbe Interact. 8, 593-601; Royer et al., 1999, Fungal Genetics and Biology. 28, 68-78; Trapp et al., 1998, Molecular and General Genetics. 257, 421-432). Its expression is regulated by the pathway specific transcription factor *TRI6* (Proctor et al., 1995, Applied and Environmental Microbiology. 61, 1923-1930) and regulatory protein *TRI10* (Peplow et al., 2003, Applied and Environmental Microbiology. 69, 2731-2736). The *TRI6* binding sites are conserved upstream of all *TRI5* homologous genes in *Fusaria.* To create an easy assay for the expression of this gene the regulatory promoter region of the *TRI5* gene was fused to the gene encoding the jellyfish green fluorescent protein (GFP) and this new chimerical gene introduced into *F. graminearum.* The transformed fungal strains showed fluorescence during the infection of wheat heads at times when the trichothecene mycotoxins are known to be produced. Thus these new transgenic strains appear to act as 'reporter strains' for *TRI5* gene expression and toxin production and were subsequently used to identify compounds that induce toxin production and characterize the mechanisms of action of production of these mycotoxins.

### EXAMPLE 1: experimental conditions

### Construction of reporter strains

The strategy adopted was to first develop transgenic strains of *F*. *graminearum* (Fg) that contained the reporter GFP gene regulated by the *TRI5* promoter (promoter of the gene encoding the Fg trichodiene synthase, said gene determining the first committed step in the biosynthesis of trichothecene mycotoxins) as tools to identify conditions that would induce gene expression necessary for toxin production in the fungus.

### Vector construction

The *TRI5* promoter-GFP fusion vector was constructed by overlap PCR using primers to the *TRI5* promoter (5'-GGCCTCGAGGCTCACGGGCTACAGTGAAT-3', SEQ ID N° 30 and 5'-TGCTCACCATGATGGCAAGGTTGTACTGGT-3', SEQ ID N° 31) and eGFP (5'-TTGCCATCATGGTGAGCAAGGGCGAGGAG-3', SEQ ID N° 32 and 5'-GGCCTCGAGCGCCTTAAGATACATTGATGAGTTT-3', SEQ ID N° 33). The PCR construct consisted of 1.5 kb of *TRI5* promoter amplified from *F. graminearum* CS3005, followed by eGFP and the SV40 terminator amplified from peGFP-N1 (Clontech). Individual fragments were amplified with Phusion DNA polymerase (Finnzymes), gel purified, then fused using the same polymerase with the two external primers. The PCR product was digested with *Xho*l and ligated into the *Sal*l site of pPZPhygHindX containing the hygromycin phosphotransferase gene under the *Aspergillus nidulans TrpC* promoter and terminator (Elliott and Howlett, 2006, Mol Plant Microbe Interact. 19, 588-96).

### Fungal transformation

The *F. graminearum* isolate CS3005 was transformed with the *TRI5-GFP* vector (Figure 1).

Fungal protoplasts were generated from approximately 1 × 10⁷ conidia germinated overnight in 200 mL of TB3 (0.3% yeast extract, 0.3% casamino acids, and 20% sucrose) media at 20°C with shaking at 150 rpm. Germlings were collected on miracloth and washed with 1 M sorbitol. Germlings were resuspended in 20 mL of 1 M sorbitol containing 5 U chitinase (Sigma), 500 mg Driselase (Sigma) and 200 mg lysing enzymes (Sigma), and incubated 1-2 hours at 28°C to release protoplasts. Protoplasts were collected by centrifugation at 4°C, washed three times with ice cold STC (20% sucrose, 50 mM Tris/HCI pH 8, 50 mM CaCl2). To a 200 µL aliquot of STC containing 2×10⁷ protoplasts 10 µg circular plasmid DNA was added, incubated for 20 minutes at room temperature, then 1 mL of 40% PEG4000, 10 mM Tris HCl pH 8, 50 mM CaCl2 was added, incubated for 20 minutes at room temperature and then 5 mL of TB3 media was added and the tubes incubated overnight at room temperature with gentle agitation. 3 mL of the transformation mixture was embedded in 30 mL regeneration media (0.2% yeast extract, 0.2% casamino acids, 0.55% low melt agarose, 27% sucrose) and set in 14 cm Petri dishes, incubated overnight and then overlaid with 30 mL of regeneration media containing hygromycin at 400 µg mL⁻¹. After 7-14 days of growth at room temperature in the dark, transformants were transferred to individual plates containing hygromycin (200 µg mL⁻¹), allowed to grow, single spored then checked for insertion of the construct by PCR and Southern hybridisation. The hybridisation probe was amplified using primers binding in the promoter region used for the reporter construct (5'-AGGTTTCAACCTCCGAGGAA-3', SEQ ID N° 34, 5'-TATCTGGCATGCCTGTCATC-3', SEQ ID N° 35).

### Plant growth and inoculation

Seeds of the susceptible bread wheat cultivars Kennedy and INDIA 44 were grown in controlled environment rooms with photoperiod length of 14 h, 25°C and 50% relative humidity at 500 mmol m-2 s. Night-time temperature was set at 15°C, with 90% relative humidity. Three seeds were sown per pot (14 cm diameter, 17 cm height) containing steamed soil (50% peat and 50% sand by volume). Heads were spray inoculated at mid anthesis with a conidial suspension at 1×10⁶ spores per mL-1. Water was used for the mock inoculation. After spraying heads were covered with plastic bags containing a small volume of water to assist in humidification. After two days plastic bags were replaced with glassine paper bags.

### Reporter characterisation

Transformants comprising the reporter strain containing the eGFP driven by the *TRI5* promoter have been screened on wheat heads for GFP expression and a number of transformants showed excellent fluorescence *in planta.*

No fluorescence was observable in plate culture. Temporally and spatially restricted fluorescence was observed with transformants in grain cultures. *In planta* GFP was visible as earlier as two days post inoculation (dpi). Fluorescence was still visible 10 dpi but was greater at earlier time points (3-6 dpi). There also appears to be some spatial restriction to GFP expression, with the greatest signal observed from the area surrounding the spikelet node.

### Toxin assays

Deoxynivalenol (DON) was measured using the DON Plate Kit ELISA from Beacon Analytical Systems according to the manufacturer's instructions, except an additional low concentration standard was used (0.02 ppm). Culture filtrates were initially diluted 1:25 giving a detection range of 0.5-12.5 ppm. Further sequential 1:10 dilutions were made for samples outside of this range as required.

T-2 toxin was measured using the Veratox T-2 ELISA kit from Neogen (Lansing, MI, USA) according to the manufacturer's instructions, except an additional low concentration standard was used (0.0025 ppm). Culture filtrates were initially diluted 1:50 into 50% methanol to give a detection range of 0.125-2.5. Further dilutions were made as for DON detection.

### Microscopy and fluorometry

Hand sections and whole tissue mounts were examined using a Leica Microsystems MZ16FA fluorescent stereomicroscope (Wetzlar) fitted with GFP plus filter set. GFP fluorescence was quantified in microtitre plates using a Fluoroskan Ascent FL microplate fluorometer (Thermo Scientific) using excitation at 485 nm and emission at 520 nm.

### Gene expression analysis

Plant gene expression was quantified using reverse transcriptase-quantitative polymerase chain reaction (RT-qPCR), as described by (Mudge et al., 2006, Physiological and Molecular Plant Pathology. 69, 73-85). Primers are listed in Table I.

**Table I: Primers used for RT-qPCR and the accessions of genes used to design the primers. Accessions are from GenBank (PR5 and Peroxidase) or from the DFCI Wheat Gene Indicies database (http://compbio.dfci.harvard.edu/tgi/cgibin/tgi/gimain.pl?gudb=wheat)**

| Gene Name | Accession | Primer forward | Primer reverse |
|---|---|---|---|
| PR5 | AF442967 | ACAGCTACGCCAAGG ACGAC, SEQ ID N° 36 | CGCGTCCTAATCTA AGGGCAG, SEQ ID N° 37 |
| Peroxidase | X56011 | GAGATTCCACAGATGC AAACGAG, SEQ ID N° 38 | GGAGGCCCTTGTTT CTGAATG, SEQ ID N° 39 |
| Carbamoyl phosphate synthase | TC249129 | TTGGAAAATTGTTGGT GTTG, SEQ ID N° 40 | CCCATTCATATGGA GCATC, SEQ ID N° 41 |
| Ornithine carbamoyl transferase | TC249067 TC249077 | ATGAAGCCCTGATGG AGATG, SEQ ID N° 42 | ATGGCACCGTCTGT TACCTC, SEQ ID N° 43 |
| Argininosuccina te synthase | TC265654 | ACGCTGAAGGTTTCAT CAGG, SEQ ID N° 44 | TAGATGCCCTTCTC CAGCAT, SEQ ID N° 45 |
| Arginine decarboxylase 2 | TC241040 | AGGAGGAGGAGCTCG ACATT, SEQ ID N° 46 | GCCGAACTTGCCCT TCTC, SEQ ID N° 47 |
| Agmatinase | TC236883 | GGGAAGAGATTTGGT GTGGA, SEQ ID N° 48 | TCACACCTTCCCCA AGTTTC, SEQ ID N° 49 |
| Ornithine decarboxylase | TC240001 | GGTACGCGAGTACTG GATCG, SEQ ID N° 50 | TCGACGTGTACGTC TTCTCG, SEQ ID N° 51 |

### Amino acid and polyamine measurements

Free amino acids were extracted from individual wheat heads by grinding the heads under liquid nitrogen and resuspending 500 mg of the powder in 2 mL of methanol, incubated at -20°C overnight and then 8 mL of water added and centrifuged to pellet solid matter. 500 µL of supernatant was dried down under vacuum. This extract was used with the Waters (Milford, MA, USA) AccQ-tag amino acid detection kit, with quantification by HPLC according to the manufacturer's instructions. For polyamine analysis wheat tissue is ground to fine powder in liquid nitrogen, extracted in perchloric acid, centrifuged and the supernatant mixed with a saturated solution of sodium carbonate and then dansyl chloride. Dansylated polyamines were extracted in toluene, the organic phase is collected, dried and re-dissolved in acetonitrile; 20 µl are injected into the HPLC as described by Marce et al. (Journal of Chromatography B 666 (1995) 329-335).

### Example 2: Construction and testing of the TRI5-GFP isolate of Fusarium graminearum

The *F*. *graminearum* isolate CS3005 was transformed with the *TRI5-GFP* vector as described above (Figure 1.A) and 7 putative transformants were selected for further analysis. Six of these putative transformants showed integration at the *TRI5* locus when tested by Southern blot analysis (Figure 1.B) and these were used in further experiments.

The *TRI5-GFP* transformants showed no GFP fluorescence during culture on PDA while in autoclaved wheat grain culture they showed diffuse GFP throughout the cultures and strong GFP expression in the hyphae surrounding a limited number of grains. Selected transformants were tested in point inoculations of wheat cv Kennedy heads. Transformants 05T0005, 05T0017 and 05T0020 were consistent in their expression of GFP during infection. GFP expression *in planta* was strongest at sites of re-emergence from the rachis and during colonisation of the pith cavity of the stem (Figure 2). Weak fluorescence could also be seen in the hyphae collaring the spikelet-rachis junction, although these were typically partially obscured by non-fluorescent hyphae. The induction of *TRI5* expression during infection and the infection patterns of the *TRI5-GFP* transformants generated in this study were consistent with previously published observations on the spatial location of trichothecene action (Jansen et al., 2005, PNAS. 102, 16892-16897) indicating that the *TRI5-GFP* strains may be used as tools for high-throughput screening of inducers of *TRI5* and subsequently DON biosynthesis.

### Example 3: Nutrient profiling for the ability of F. graminearum to grow in various carbon and nitrogen sources

The ability of *F*. *graminearum* to grow in a variety of carbon and nitrogen sources was assessed using the phenotype microarray technology. Amongst the carbon sources D Fructose allowed the most growth but a number of other carbon sources, particularly the simple sugars, supported good growth. Similarly with nitrogen source profiling, *F. graminearum* was able to use a number of different compounds as sole nitrogen sources, including some that may be biochemically difficult to access in nature such as D enantiomers of amino acids. Both the Australian CS3005 and American Ph-1 strains of *F*. *graminearum* were assayed for their ability to access nitrogen sources, and although generally these isolates were similar in their growth across the plate, some differences were observed between these strains. Most notable was the considerably poorer ability of the Australian CS3005 isolate to grow on biuret and methylamine. Neither isolate could grow on D-lysine, Hydroxylamine, ethylenediamine, N-acetyl-D-galactosamine, N-acetyl-D-mannosamine, thymine, thymidine, uracil, uridine or alloxan as a sole nitrogen source.

### Example 4: Nutrient profiling for induction of DON production in F. graminearum

Using the *TRI5-GFP* reporter strain (05T0017) and parent (CS3005) as a control, the role of carbon and nitrogen sources were assessed for their ability to induce *TRI5* promoter activity and GFP expression. To test carbon sources, the *TRI5-GFP* transformant 05T0017 was grown in the PM1 and PM2 carbon source plates but no fluorescence was observed with any of the 190 carbon sources tested in these plates. Growth of the reporter strain in PM3 nitrogen source test plates revealed a key role for several specific nitrogen sources in regulating *TRI5* expression. Even though many nitrogen sources supported growth of the fungus, most did not induce *TRI5* expression. The exceptions were agmatine, putrescine, arginine and guanine, which promoted strong GFP fluorescence and to a lesser extent citruline, ornithine, N-amylamine, ethanolamine, β-phenylethylamine, tyramine and D-glucosamine. Expression of GFP seemed to appear after most of the growth of the fungus had occurred. For example in agmatine, fluorescence was maximal at 4 dpi, but at 3 dpi the well showed near maximal optical density and microscopically was essentially full of hyphae.

The culture filtrate for 30 selected nitrogen sources was assayed for DON content at the end of the seven days incubation. As shown in Figure 3, the production of toxin (Figure 3.A) correlated with the occurrence of GFP fluorescence (Figure 3.B). With some nitrogen sources, e.g. arginine, ornithine, putrescine and agmatine, the DON concentrations that were measured in the media were extremely high and either equal to, or greater than 1000 ppm. It should be noted the basal media used in the PM3 plates is very similar to that used in the widely used two stage DON inducing media (Miller and Blackwell, 1986, Canadian Journal of Botany. 64, 1-5) except for the nitrogen source. Well A2 containing ammonia as the nitrogen source is equivalent to the second stage media of Miller and Blackwell (1986, *op. cit*.), but relatively small amounts of DON were present in this media (44±9 ppm) compared to other wells. It should also be noted that the fluorescence read out for *TRI5* GFP expression is in some circumstances dependent on the growth and biomass of the fungus and may not truly represent the activity of the fungus and its potential for DON production. This is exemplified by cultures using methylamine as a nitrogen source where growth was very limited and only a low fluorescence signal was obtained, however, analysis of media showed toxin was still produced (109±9 ppm).

The role of agmatine in inducing trichothecene production appears to be a general phenomenon, as the production of T-2 toxin by *F. spropotrichioides* was also higher in agmatine (134±14 ppm, n=3) compared to glutamine (1.3±0.3 ppm, n=3) containing media after 7 days growth in culture (Figure 4).

To investigate whether *TRI5-GFP* could be induced by either arginine or agmatine in mature hyphae pre-grown on other nitrogen sources, the induction was analysed after initial growth in the non-inducing nitrogen source glutamine, followed by addition of glutamine, arginine or agmatine. As shown in Figure 5, induction by arginine and agmatine occurred within 24 hours of their application, but did not reach maximal levels until 48-72 hours after addition of the inducing nitrogen source. The addition of additional glutamine did not have any effect on *TRI5-GFP* expression.

### Example 5: TRI5 gene expression level in response to agmatine is similar to that observed in p/anta

Although DON production was strongly induced *in vitro* by the identified compounds it is difficult to compare directly the levels of DON production in liquid culture to that *in planta* where the majority of the tissue is of plant origin. However, on a fresh weight basis DON levels in excess of 500 ppm in heavily infected heads have been observed (Mudge et al., 2006, Physiological and Molecular Plant Pathology. 69, 73-85). Analysis of fungal gene expression relative to fungal specific reference gene in both culture and *in planta* provides a better comparison of fungal mycotoxin biosynthetic potential. To address this, RT qPCR was carried on *TRI5* gene transcripts, relative to the fungal *18S rRNA.* As shown in Figure 6, the level of *TRI5* gene expression, when the fungus was grown in agmatine as the nitrogen source was very similar to that observed during infection.

### Example 6: Abolition and synergism of TRI5 induction by nitrogen source combinations

The role of nitrogen in regulating the production of secondary metabolites is well documented in many other fungi. We were interested to see if there is a hierarchy of *TRI5* activation when multiple nitrogen sources are present. Interactions between nitrogen sources and their effects on *TRI5* transcript levels were investigated in media where two nitrogen sources were present during growth (Table II). Nitrate and cysteine present in the media at the time of inoculation completely inhibited the induction of *TRI5-GFP* by nitrogen sources e.g. agmatine, putrescine, that when supplied alone were highly inductive. Synergistic effects were also observed, particularly between methionine and agmatine. It should be noted that there was some concentration dependence observed with the inducing nitrogen sources. For example the agmatine/control combination (where agmatine was at a concentration of 5 mM) showed considerably less fluorescence than the agmatine/agmatine combination (final concentration 10 mM).

**Table II: Interaction of selected nitrogen sources in induction of TRI5-GFP. Cultures were grown in the presence of two nitrogen sources for four days then the fluorescence measured. Data are the mean of four replicate cultures relative to the maximal level observed (Methionine/Agmatine, 100%) with the standard error of the mean shown in brackets. The control is no nitrogen source.**

| | **C** | **A** | **N** | **Ag** | **P** | **G** | **M** | **Cy** |
|---|---|---|---|---|---|---|---|---|
| **Control (C)** | 0(0) | | | | | | | |
| **Ammonium (A)** | 0(1) | 0(1) | | | | | | |
| **Nitrate (N)** | 0(0) | 0(0) | 0(0) | | | | | |
| **Agmatine (Ag)** | 5(1) | 15(9) | 0(1) | 26(23) | | | | |
| **Putrescine (P)** | 0(1) | 9(6) | 0(0) | 69(11) | 61(0) | | | |
| **Glutamine (G)** | 0(0) | 0(0) | 0(0) | 47(4) | 37(11) | 0(0) | | |
| **Methionine (M)** | 18(5) | 24(6) | 0(0) | 100(18) | 79(7) | 71(5) | 30(3) | |
| **Cysteine (Cy)** | 0(0) | 0(0) | 0(0) | 2(6) | 5(4) | 0(0) | 3(3) | 0(0) |

### Comments about the data

The induction of *TRI5* gene expression and DON production induced in these experiments is notable for two important reasons. Firstly the levels of *TRI5* mRNA measured in the fungus when induced by strong inducers like agmatine and arginine (Figure 6) were equivalent to those observed in the fungus when growing in infected wheat plants indicating that these inducers either mimic the inducing factors present in infected wheat heads or they are the actual inducers present in infected grains. Secondly, the concentrations of DON and T2 toxin produced in the presence of inducers like agmatine (Figure 4) are extremely high and equivalent or greater than those observed in infected plant material again demonstrating that these inducing compounds either mimic or reproduced the inducing conditions in infected plants.

### Example 7: Analysis of the expression of genes of polyamine biosynthesis pathway in wheat during infection

Analysis of the expression of genes of the polyamine biosynthesis pathway in wheat during infection (Figure 7) showed that genes encoding ornithine decarboxylase (E.C. 4.1.1.17), arginine decarboxylase (E.C. 4.1.1.19) and argininosuccinate synthase (E.C. 6.3.4.5) were the most strongly induced in expression during infection (Figure 7). This indicates that the levels of these enzymes and the levels of synthesis of arginine and polyamines are likely to increase during the infection of wheat and that these may in turn induce trichothecene production and particularly DON production in the infecting *Fusarium* fungus.

To verify that metabolites capable of inducing trichothecene toxins are also induced following challenge of wheat by *F. graminearum* infected heads were sampled to see if the strong inducers were increased. Following inoculation, there were substantial increases in the concentrations of the polyamines, putrescine and cadaverine as well as the amino acids arginine, methionine and lysine at times that coincided with DON induction and fungal biomass increases (Figure 8.A and B).

### Example 8: Characterization of Wheat ADC2 and ODC sequences.

Putative function (text) searches of both rice and *Arabidopsis* genomes were performed to identify putative *ADC* genes. *Arabidopsis* contains two *ADC* sequences (*AtADC1*, AT2G16500 and *AtADC2*, AT4G34710) and three loci were identified in rice (Os04g01690, Os06g04070 and Os08g33620) with two of these known to be expressed (Os04g01690 and Os06g04070). These genomics sequences were used to find wheat orthologous sequences, by a blast to a wheat EST databank. Two groups of sequences were identified encoding for the wheat equivalents of *ADC1* and *ADC2.* Five wheat EST (EMBL:CK163110 ; EMBL:CD8420; EMBL:CD930750 and two private sequences) with significant homologies to the rice *ADC2* were identified and clustered on a unique sequence of 1351 nt (SEQ ID N° 7).

This sequence can be transcribed to the amino acid sequence depicted in SEQ ID N° 52.

Two wheat EST (EMBL:BF474072 and EMBL:BI479919) were used to determine the *ADC1* wheat sequence. The alignment between the wheat contigs for *ADC1* and *ADC2* shows only poor homology.

A 450 nt fragment of the *AtADC2* contig was choosen for designing RNAi constructs (SEQ ID N° 53). This sequence is divergent enough to *ADC1* to allow the specific inhibition of the wheat *ADC2* gene on the three genomes by the RNAi construct.

Three rice ODC sequences were identified with putative function searches in the rice genome. These were located on chromosome 9 (Os09g37120.1), on chromosome 2 (Os02g28110.1) and on chromosome 4 (os04g04980.1).

These three sequences were used to find wheat homologous sequences. As most of the wheat sequences identified by these different alignments were similar, it was concluded that the ODC sequence is highly conserved in wheat. One of the sequences (EMBL:CN012161, SEQ ID N° 17) was identified to be homologous to the three rice sequences; this sequence is issued from a *Fusarium* infected library. It was chosen to define the ODC fragment sequence to be use for RNAi (SEQ ID N° 54). The RNAi construct including this fragment may inhibit the expression of the three wheat ODC paralogues.

### Example 9: Wheat RNAi transformation constructs for ADC and ODC :

The synthetic fragment of *ADC2* was cloned in a pUC57 vector (GenBank accession Y14837), leading to the pUC57 TaADC2 vector. The *BamH*l / *Sal*l fragment of 451 nt (TaADC2) from pUC57-ADC2 was then introduced in the pENTR1A vector (Invitrogen) linearised with *Bam*Hl / *Xho*l, to create the pBIOS1561.

An LR clonase reaction between the pBIOS1561 and the pBIOS1560, allows the creation of pBIOS 1577 (Figure 9). pBIOS1560 is a vector used to create RNAi vectors. A PCR reaction on pBIOS1577 controlled that the tubulin intron is in the right orientation to be properly spliced. The binary plasmid pBIOS1577, was then introduced in the *A*. *tumefaciens* hypervirulent strain EHA105, and used for transformation experiments.

The synthetic fragment of ODC was cloned in a pUC57 vector, leading to the pUC TaODC vector. The *Xba*l / *Eco*lCRl fragment of 450 nt from pUC57-ODC was introduced into the pENTR1A vector opened by Xbal / *Xmn*l to create the pBlOS1588. In a similar way an LR clonase reaction between pBlOS1588 and pBIOS1560, led to the binary vector pBlOS1589 (Figure 10) to be introduced in the EHA105 *Agrobacterium* strain for transformation experiments.

### Example 10: Wheat transformation protocol

The method is essentially similar to the one described in WO 00/63398. Wheat tillers, approximately 14 days post-anthesis (embryos approximately 1mm in length) were harvested from glasshouse grown plants to include 50cm tiller stem, (22/15°C day/night temperature, with supplemented light to give a 16 hour day). All leaves were then removed except the flag leaf and the flag leaf was cleaned to remove contaminating fungal spores. The glumes of each spikelet and the lemma from the first two florets were then carefully removed to expose the immature seed. Only these two seed in each spikelet were generally uncovered. This procedure was carried out along the entire length of the inflorescence. The ears were then sprayed with 70% IMS as a brief surface sterilization.

*Agrobacterium tumefaciens* strains containing the vector for transformation were grown on solidified YEP media with 20mg/l kanamycin sulphate at 27°C for 2 days. Bacteria were then collected and re-suspended in TSIM1 (MS media with 100mg/l myo-inositol, 10g/l glucose, 50mg/l MES buffer pH5.5) containing 400µM acetosyringone to an optical density of 2.4 at 650nm.

Agrobacterium suspension (1µl) was inoculated into the immature seed approximately at the position of the scutellum: endosperm interface, using a 10µl Hamilton, so that all exposed seed were inoculated. Tillers were then placed in water, covered with a translucent plastic bag to prevent seed dehydration, and placed in a lit incubator for 3 days at 23°C, 16hr day, 45µEm-2s-1 PAR.

After 3 days of co-cultivation, inoculated immature seed were removed and surface sterilized (30 seconds in 70% ethanol, then 20 minutes in 20% Domestos, followed by thorough washing in sterile distilled water). Immature embryos were aseptically isolated and placed on W4 medium (MS with 20g/l sucrose, 2mg/l 2,4-D, 500mg/l Glutamine, 100mg/l Casein hydrolysate, 150mg/l Timentin, pH5.8, solidified with 6g/l agarose) and with the scutellum uppermost. Cultures were placed at 25°C in the light (16 hour day). After 12 days cultivation on W4, embryogenic calli were transferred to W425G media (W4 with 25mg/l Geneticin (G418)). Calli were maintained on this media for 2 weeks and then each callus was divided into 2mm pieces and re-plated onto W425G.

After a further 2 weeks culture, all tissue was assessed for development of embryogenic callus: any callus showing signs of continued development after 4 weeks on selection was transferred to regeneration media MRM 2K 25G (MS with 20g/l sucrose, 2mg/l Kinetin, 25mg/l Geneticin (G418), pH5.8, solidified with 6g/l agarose). Shoots were regenerated within 4 weeks on this media and then transferred to MS20 (MS with 20g/l sucrose, pH5.8, solidified with 7g/l agar) for shoot elongation and rooting.

The presence of the T-DNA, and the number of copies are quantified by Quantitative PCR.

For ODC RNAi, 41 transformants (T0) were obtained, regenerated and cultivated in greenhouse.

For *ADC2* RNAi, 55 transformants (T0) were obtained, regenerated and cultivated in greenhouse.

### Example 11: Analysis of wheat transformants in the presence of pathogenic fungus

### Inoculation of wheat transformants and control by Fusarium

### a- glasshouse and culture chamber

The artificial infection occurs using an inoculum of 10⁶ spores / ml in water for *Fusarium culmorum* or 5x10⁵ spores / ml for *Fusarium graminearum.* The inoculation is performed on the spike by Ecospray (LCF Labo chimie France, Aix en provence) on plants at a mid flowering stage. After inoculation the spike is covered by a transparent cellophane packet. 24h after the first inoculation a second inoculation is performed on these spikes to cover the flowering period. The cellophane packet is removed 7 days after the second inoculation.

Five spikes are inoculated by plant, 6 plants by repetition and 3 repetitions for each event. The first notation is made 15 days post inoculation, followed by notations every 7 days for a period of 4 weeks. The percentage of infected spikelets is noted, with a spikelet considered to be infected if at least two glumes are infected (white coloured).

### b- in field

The above described spore solution for inoculation is completed by addition of 0,1 % of tween 20. The inoculation is performed by a nitrogen spray (2bar) at the density of 100ml/m², at a mid flowering stage, at the end of the day to limit the evaporation.

The notation is done on 10 plants by line, 2 and 3 weeks after inoculation. The percentage of infected spikelets is noted as previously described.

### Inoculation of wheat transformants

Transformed plants may also be growth and inoculated by the reporter strain *TRI5-GFP* as described in Example 1, and the fungus growth may be followed by microscopy and fluorometry (Example 1).

Transformed plant may also be inoculated by wild type fungus, and a toxin quantification may be done by an ELISA test as described in Example 1 or by HPLC.

## Claims

1. Method for inducing and/or increasing resistance to a trichothecene-producing pathogen infection in a plant comprising the step of inhibiting or downregulating expression of an endogenous polyamine synthesis gene in said plant, said inhibition or downregulation resulting in said plant to have increased resistance to said pathogen infection as compared to a nearly isogenic plant in which said endogenous polyamine synthesis gene is not inhibited or downregulated.

2. Method for reducing trichothecene level in a plant infected by a trichothecene-producing pathogen, comprising the step of inhibiting or downregulating expression of an endogenous polyamine synthesis gene in said plant, said inhibition or downregulation resulting in said plant to have reduced trichothecene level when infected by said pathogen as compared to a nearly isogenic plant in which said endogenous polyamine synthesis gene is not inhibited or downregulated.

3. Method according to claim 1 or 2, wherein said pathogen is *Fusarium*.

4. Method according to any of claim 1 to 3, wherein said plant is a cereal.

5. Method according to claim 4, wherein said cereal is chosen in the group consisting of wheat, maize, rice, rye, barley, sorghum and oat.

6. Method according to any of claims 1 to 5, wherein said gene is chosen in the group consisting of arginine decarboxylase (ADC, EC 4.1.1.19), ornithine decarboxylase (ODC, EC 4.1.1.17), agmatinase (EC 3.5.3.11), argininosuccinate synthase (EC 6.4.3.1), argininosuccinate lyase (EC 4.3.2.1).

7. Method according to any of claims 1 to 6 wherein said gene is inhibited or downregulated by mutation of said gene by insertion of a transposable or a T-DNA element or by physical mutagenesis.

8. Method according to any of claims 1 to 5, wherein said gene is inhibited or downregulated by transformation of said cereal with a genetic construct.

9. The method of claim 8, wherein said genetic construct comprises a promoter that is induced by *Fusarium* infection.

10. Method according to any of claims 1 to 9, wherein said plant is rice and said gene is chosen among the genes having a sequence represented by any of SEQ ID N° 1, SEQ ID N° 2, SEQ ID N° 14, SEQ ID N° 15, SEQ ID N° 16, SEQ ID N° 23 and SEQ ID N° 24.

11. Method according to any of claims 1 to 9, wherein said plant is wheat and said gene is chosen among the genes having a sequence represented by any of SEQ ID N° 3, SEQ ID N° 4 SEQ ID N° 5, SEQ ID N° 6, SEQ ID N° 7, SEQ ID N° 8, SEQ ID N° 9, SEQ ID N° 10, SEQ ID N° 17, SEQ ID N° 18, SEQ ID N° 19, SEQ ID N° 20, SEQ ID N° 21, SEQ ID N° 25, SEQ ID N° 26 and SEQ ID N° 27.

12. Method according to any of claims 1 to 9, wherein said plant is barley and said gene is chosen among the genes having a sequence represented by any of SEQ ID N° 11, SEQ ID N° 12, SEQ ID N° 13, SEQ ID N° 22 and SEQ ID N° 28.

13. Method according to any of claims 1 to 9, wherein said plant is maize and said gene is chosen among the genes having a sequence represented by any of SEQ ID N° 29, SEQ ID N° 55, SEQ ID N° 56, SEQ ID N° 57, SEQ ID N° 58, SEQ ID N° 59, SEQ ID N° 60, SEQ ID N° 61, SEQ ID N° 62, SEQ ID N° 63, SEQ ID N° 64, SEQ ID N° 65, SEQ ID N° 66, SEQ ID N° 67, SEQ ID N° 68, SEQ ID N° 69, SEQ ID N° 70, and SEQ ID N° 71.

14. A plant presenting an inhibition or downregulation of the expression of an endogenous polyamine synthesis gene.

15. The plant of claim 14, wherein said inhibition or downregulation is induced by *Fusarium* infection.

16. The plant of claim 14, wherein said inhibition or downregulation is restricted to the rachis, the spikelets and/or the stem cavity.

17. The plant of any of claims 14 to 16, which is a cereal.

18. The plant of claim 17, which is chosen in the group consisting of wheat, maize, rice, rye, barley, sorghum and oat.

19. A part of the plant of any of claims 14 to 18, wherein said part is a cell or a seed.

20. Method for reducing trichothecene production by a trichothecene-producing pathogen, comprising the step of limiting concentration of polyamines and polyamine amino acid precursors available to said trichothecene-producing pathogen during its growth.

21. Method according to claim 20, wherein said limitation is performed in the growth medium *in vitro*.

22. Method according to claim 20, wherein said limitation is performed *in planta*, by growing said trichothecene-producing pathogen on a plant presenting an inhibition or downregulation of the expression of an endogenous polyamine synthesis gene.

23. Method according to claim 20, wherein said limitation is performed *in planta* by growing said trichothecene-producing pathogen on a plant and applying an inhibitor of an enzyme of the polyamines biosynthetic pathway to said cereal.

24. Method according to claim 20, wherein said limitation is performed by supplying a compound abolishing the effect of said polyamines and polyamine amino acid precursors to said trichothecene-producing pathogen.

25. Method according to any of claims 20 to 24, wherein said pathogen is *Fusarium*.

26. Method according to claim 25, wherein said limitation is performed by applying an inhibitor of a *Fusarium* polyamine/amino acid transporter to said *Fusarium*.

27. Method according to any of claims 20 to 26, wherein said polyamines and polyamine aminoacid precursors are chosen in the group consisting of ornithine, arginine, putrescine and agmatine.


SEQUENCE LISTING

<110>  BIOGEMMA
       COMMONWEALTH SCIENTIFIC AND INDUSTRIAL RESEARCH
       ORGANISATION

<120>  PLANTS HAVING IMPROVED RESISTANCE TO PATHOGENS

<130>  BGM 45 - EP PRIO

<160>  71

<170>  PatentIn version 3.3

<210>  1
<211>  1872
<212>  DNA
<213>  Oryza sativa

<400>  1
   atggcgaaga agaactacgg tcaagtgtac aacatcctcg ggtggggcga cccctacttc      60
   accgtgaact cccatggcca cctcgccgtc aagccccatg gccgcgacac catgtccggc     120
   caggacatcg acgtccactc cgtcatccac agagctctcg ccaccaccat caccaccaac     180
   gacggcgaca agaagcccca gttcccgatg atcctccgct tccccgacgt gctcaagaac     240
   cgcctcgact ccctccacgc cgccttccat ggcgccgtcg acagcaccgg ctacgcgtcc     300
   aggtaccagg gcgtgttccc catcaaggtg aaccagaaca aggccgtcgt ccaggacctc     360
   gtcaccttcg gccatggcta cagctacggc ctcgaggctg gctccaagcc ggagctgctc     420
   atcgccatga gctgcctcgc caaggccaag cccggagctt acctggtctg caatggctac     480
   aaggacgccg actacgtcgc gctcgcgctc tccgcgcgcg ccatgggcct caacgccatc     540
   atcgtgctgg agatggagga ggagctcgac atcgtcgtcg agcagagcgc caggctcggc     600
   gtcgagccgg tcatcggcgt ccgcgccaag cttctcacca agattccggg acacttcggc     660
   tccacggccg gcaagcacgg caagttcggg atgctcgccg acaagatcta cgaggtggcc     720
   ggcaagctga agaagatggg gaagctgcac tggctgaagc tgctgcacta ccatgtcggc     780
   tccatgatcc cgaccaccga catcgtgtac aacgcggcgg ccgaggccgc aggaatctac     840
   tgcgcgctgg tgaaggagca cggcgcgacg gggatgacca cgctggactg cggcggcggg     900
   ctcggcgtcg actacgacgg gacgcggtcg ggcagctccg acatgtcggt ggcgtacggg     960
   ctcgagcagt acgcgtccag catcgtgcag gcggtgcggc tcacgtgcga cgacaatggc    1020
   gtcccccacc cggtgctctg caccgagagc ggccgcgcca tggcgtcgca ccactccatg    1080
   atcatcctcg aggcgctctc ggcgatcccg gagccgcagg acgaggagga cacccatcac    1140
   cggctgctca gcaagatcca ggacctctcc tccaagcagc caagaactgc tcataccgtc    1200
   aatggcggcg gcggcgtgga cgccatgcac tcgcacgccg tggagctgaa gaagcacggg    1260
   atcgagatgt acaagctggc gaagaagctg tccaagaggg tgaccggcga cgccaacggc 1320
   atctacaact accacatgaa cctctccgtc ttctccctcg tccccgactt ctggggcatc 1380
   ggccagctct tcccgatgat gccggtgagc cggctgaacg agaagccgac catcaacggc 1440
   acgctggtcg acatcacctg cgacagcgac ggcaaggtag agaagttcat ccgcgacgcc 1500
   gtgacgctgc cgctgcaccc actcgacgac gcggcggcgg agcacggcgg ctactacgtg 1560
   gcggcgctgc tgtcgggggc gtaccaggag gccctggcgt gcaagcacaa cctgttcagt 1620
   ggccccacgc tcgtgcgcgt cgagagcgcc ggcggcggcg gcgcgttcaa gatcgtctcc 1680
   gttgagctcg gcccgacggc ggaggaggtc atcggcacga tgaggtacga cgtgaagaac 1740
   gacattagcg atgtgatcga gaaggttgcg acggagaatg gcgtgtggcc gatggttgag 1800
   ccgctgatga agaaagggct gaccaccatg ccctacctca acgattacaa gccccccaaa 1860
   accacctttt aa 1872

<210> 2
<211> 2109
<212> DNA
<213> Oryza sativa

<400> 2
atgcctgcgc tcgccgtgga cgccgctgca cctgtcgcgc acgccttcgc gtgcgacgcg 60

gcgcgcttcc ccgcgccgct gctgggcccc gccgccgcgg ctgcggcggt ggcggagaag 120

ccggacgcgg ccgcgtggtc ggccgacctc tcgtccgcgc tgtacaacgt ggacggctgg 180

ggcgcgccgt acttcttcgt caacgacgac ggcgacgtcg ccgtgcgccc gcacggcgcc 240

gcaaccctgc ccgggcagga gatcgacctc gccaaggtgg tggccaaggc cgccggcccg 300

cgctccggcg gcgggctcgg cctgccgctc cccctgctgg tgcggttccc cgacgtgctc 360

cgccaccgcg tggaggccct gaacgcggcg ttcgactacg ccgtgcgctc caccggctat 420

ggcgggaggt accagggcgt gtaccccgtc aagtgcaacc aggaccgcca cgtcgtggag 480

gacattgtgg agttcggcga gccgttccgc ttcggcctgg aggccggctc caagcccgag 540

ctgctgctcg ccatgagctg cctcgccgcg cgcggcaacc cggacgccct gctcatctgc 600

aacggctaca aggacgacga gtacgtctcg ctcgcgctca tcgcgcgcac catggggctc 660

aacacggtca tcgtgctcga gcaggaggag gagctcgaca ttgtggtcga cgctagccgc 720

cgcctcggcg tgcgcccggt ggtcggcatg cgcgccaagc tgcgcaccaa gcacgccggc 780

cacttcggct ccacgtccgg ggagaagggc aagttcggcc tcaacgccgc gcagattctc 840

tccgtcgtcg ccaagctcaa gacccttggt atgctcgact gcctccagct cctgcacttc 900

cacattggct cccagatccc gaccactgct ctgctcggcg atggcgtcgg ggaggccgcg 960

cagatctact gcgagctcgc ccgcctgggc gcggccatgc gcgtcatcga cgttggtgga 1020

ggcctcggca ttgactacga cgggagtcac tcggcgcaga ccgacatgtc cgtggcatac 1080

agcttggagg agtacgcggc ggccgtggtg gccgccgtcg gccgcgtctg tgaccgcaag 1140

ggcgtcgcgc atcccatcat ctgcagcgag agcggccgcg cgctggtgtc gcaccactct 1200

gtcctggtgt tcgaggcctt ctcggcttcg gcgcccggcc gcattgaccc ggccacgggc 1260

tacctgctcg acgagctcac cgacgactgc cacgccgact accgcaacct catggccgcc 1320

gcggtgcgcg gcgacttcga cacctgcgct ctgtacgccg accagctgaa gcggcgctgc 1380

gccgatcagt tcaaggacgg cgttcttggc ctggagcacc tcgccgcggt ggacagcctc 1440

tgcgagatcg tcgcccgcgg catgggcgcc gccgagccgc cgcgcacgta ccacatcaac 1500

ctgtccgtgt tcacctccct gccggacatg tgggcgatcg ggcagatgtt cccgatcatc 1560

ccaatccagc gcctcggtga gcgccctgcg gtcgacggcg tcctctccga cctcacctgc 1620

gacagcgacg gcaaggtcga ccacttcatc ggcgggaggc acagcctgcc gctgcacgag 1680

ctccccgtcc acggcacccg cggctactac ctcggcatgt tcctcggcgg cgcctaccag 1740

gaggccctcg gcggcctcca caacctgttc ggcgggccga gcgtggtgcg cgtgtcgcag 1800

agcgacgggc cgcactgctt cgccgtcacg cgcgcggcgg cggggccgtc gtgcgccgac 1860

gtgctccgct cgatgcagca cgagcccgag gtgatgttcg aggtgctcaa gcagaggacc 1920

gacggcgcca ccgccgccgc gctcgccagg gcgttcggcg caatgccgta cctgtcgttc 1980

gacccggagg ccgcggcgat ggccagcggg gagagcagcg gcatgagcag cgactcggag 2040

gggtcggccg ccggcgcggc ggaggaggat gacgacgagt gggagttcat gcgggggctc 2100

accgtgtga 2109

<210> 3
<211> 906
<212> DNA
<213> Triticum aestivum

<400> 3
atgcaagcct tctctgatct tcactagtaa ggtgcttcca cgatggccgc ccagaactac 60

ggtgaattgt acaacatcct cggctggggc gaaccctact tcaccgtgaa cagggacggc 120

cacctgtgtg tcaagaccca cggccacgag acgaagccag ggcaagagat agacgtggtg 180

tccgtagctg aagctgcaaa ggagaagggc gtccaattcc cgatgatcct ccgcttcccc 240

gacgtgctca agcaccgcct cgactcgctc cacgtcgcat tcgagaaggc catcaagcac 300

accgggtaca cggcgccata tcagggcgtg tacccagtga aggtgaacca gaacagggcc 360

atcatcaacg actttgttag ctttggccac cgtcacagct acgggctgga ggcgggctcc 420

aagccagaat tgctcatcgc aatgagctac cttaccaaag ccaagcctgg agccttcctg 480

gtatgcaatg ggtacaagga cgcggactac gtggcgctgg cgctgtcggc gcgtgccatg 540

ggcctgaatg tcatcatcgt gttggagatg gaggaggagt tggacatcat catcgagcac 600

agcagcaagc tcggggtaga accggtgctg ggcgtgcgtg ccaagctgct caccaagata 660

ccaggccact tcgggtcgac ggccggaaag cacggcaagt tcggcctgct tgcggacaag 720

atctacgagg tggccaagaa gctcaaggat ctcaacaagc tgcactggct caagttgctg 780

cacttccaca tcggctccat gatcccgacc acggacatag tgtccagtgc agccagggag 840

gcctccggca tctactgcac cctggtgaag cagtatggcg cggagatgac gacgctggac 900

tgcggc 906

<210> 4
<211> 963
<212> DNA
<213> Triticum aestivum

<400> 4
tagcgtggtc gcggccgagg tctctccgtc ttctcgctgc ttcctgacgt gtggggcatc 60

aaatggcgct tcccgatgat gcctgtgagc cggctcaatg aggagcctac ccgaatgagc 120

acactcatcg acctaacctg cgacagcgac ggcaagatcg acaagttcat aggaaacgcc 180

gagacgctcc cgcttcaccc actagaccct gagcgcggtg gctgctacta cctcgccgtg 240

ctgctttccg gcgcatacca ggaagccctt tctaacaagc acaacctgtt cggcggcccg 300

agcatagtgt gggtcgagaa aagcaccgcc gctgccaccg gcttccaaat tgccacagcc 360

gacctgggcc cgacgacgga ggagctcatc agcaccatga ggtacaatgt caggcaggac 420

atcgtgggcg tcatccagag gcgggccata gagatagggg tgtggggaat ggtgggggag 480

gcagtgcaca ctgggcttac caccatgccg tacctcatca tggaagatca cgctgcctct 540

tcatggaagc accaaggagg actagatgga aaagacgagt ccatcgtcaa gatggtgttt 600

cggcaagttt cccgtctcct ctgcttgccc ttcatgtggt ccatcccatc aaaacgaatc 660

ggatcagcag cctgatttcg atcgtttccc ctcaaaaaat aaagagtaat gctacaccca 720

ccaacactta cgtgagcttt ttacgtaaag gctaatatgg atgtgtttta ttggatcgag 780

attagattag tttgacatgg aaagttacgt agatgtatca ttattcaaaa ataaaccgcc 840

ccctaaatta ttgttctagc attataatag tatttgttat ttactcatat attgtaagag 900

aatctttcta tatatctata tatatatatc tattacgctg aaaacctcgg ccgcgaccac 960

gct 963

<210>  5
<211>  680
<212>  DNA
<213>  Triticum aestivum

<400>  5
ggttcccgac tgctggggca tccagcagct gttcccgatg atgcccgtga gccggctcca      60

cgagaggccg accaacatgg ccacgctcat tgacctcacc tgcgacagcg acggtaaggt     120

cgaaaagttc atccgtggcg cccagacact gccgctgcac ccgctggacc ccaagctcgg     180

tggctactac gtggccgtgc tcctgtccgg cgcgtaccag gaagcccttt ctagcaagca     240

caacctgttc ggtggcccaa gcattgtgcg ggtcgacgaa agcggcggca tcgtcacagc     300

ggacctgggc gctacaacgg aggagctcat cagtaccatg aggtacgatg tcaatcagga     360

catcctcgac gtgatccacg agcgggccga agagcatggg gtgtggaaga tggtggggga     420

gatcgtgaag gccgggctta ccaccatgcc ctacctcatc gaataccagg tcccgctccc     480

aggaaccgcc tagctttcgt gtttggacga gactatatat atctcaaata agcgtggccc     540

ctattcgtgt ttgcacgggc tgatatatgc atgagcattc tcctgcagtg ctctaaatta     600

tatcgactat catctcttag cattcttctg atatatgtat ttatatttaa gaataaaggt     660

tttaaggcat cgatctatct                                                 680


<210>  6
<211>  1353
<212>  DNA
<213>  Triticum aestivum


<220>
<221>  misc_feature
<222>  (1053)..(1053)
<223>  n is a, c, g, or t

<220>
<221>  misc_feature
<222>  (1248)..(1248)
<223>  n is a, c, g, or t

<220>
<221>  misc_feature
<222>  (1269)..(1269)
<223>  n is a, c, g, or t

<400>  6
atttcctgac tacagcacaa agaaacagaa aaatttctga cttttttccc cagtggatta      60

gctttctccg gttgttaatt ccgtgacgag atgcctgcgc tcgctgtcga cgccgccgcc     120

cccgtggccc acgccttcgc cgcgtcctgc gacgcgtcgc cgcgcttcgc cgcgccgctg     180

ctgggccccg ccgccgccgt cgtcgctgcc acggccgccg agaagcccga gatggccgcg     240

tggtcggccg acctctcgtc ggcgctctac aacgtggacg gctggggcgc gccctacttc 300

ttcgtcaacg acgacggcga cgtcgcggtg cgcccgcacg gcgccgccac gctgcccggc 360

caggagatcg acctcgccaa ggtcgtcgcc cgggcggccg gcgcgcgcgc cggcggcggg 420

ctcggcctcg cgctgccgct gctcgtgcgc ttccccgacg tgctgcgcca ccgcgtcgag 480

accctcaacg ccgccttcgc ctacgccgtg cgctccacgg gctacgccgg gcggtaccag 540

ggcgtctacc ccgtcaagtg caaccaggac cggtacgtgg tcgaggacat cgtcgagttc 600

ggcgcgccct tcgggttcgg cctcgaggcc ggctccaagc ccgagctgct gctcaccatg 660

agctgcctcg tcgcccgcgg cagccccgac gccctgctca tctgcaacgg ctacaaggac 720

ctcgagtacg tctcgctcgc gctcatcgcc cgcaccatgg gcctcaacac cgtcatcgtg 780

ctcgagcagg aggaggagct cgacattgtc gtcgaggcca gccgccgcct caacatccgc 840

ccggtggtcg gcatgcgcgc caagctgcgc accaagcacg ccggccactt cggcgccacc 900

tccggcgaga agggcaagtt cggcctcaac gccgcgcaga ttctctccgt ggtggccaag 960

ctcaaggccc tcgccatgct cgactgcctc cagctcctgc acttccacat tggctcccag 1020

atccccacca ccgccctgct cgccgacggc gtnggcgagg ccgcgcagat ctactgcgag 1080

ctcgcccgcc tcggcgcggc catgcgcgtc attgacgtcg gcggcggcct cggcatcgac 1140

tacgacggga cgcactcggc gcagaccgac atgtcggtgg cctacagcct ggaggagtac 1200

gcggcggccg tggtcgccgc cgtcggccgc gtctgcgacc gcaagggncg tgcagcaccc 1260

tgtcatctng cagcgagagc cggccgcccc ctggtgtccc accattcggt cctcattttc 1320

agggccttcc cggaaggggg ccgggttcca aaa 1353

<210> 7
<211> 1555
<212> DNA
<213> Triticum aestivum

<400> 7
ttcgggtcga ccacgcgtcc gcctcttttg gcatcagtga tcgtcggaat ctcatcatcc 60

tagattgatt tcgattgtta taattactga cttttttgcc gtaattttct tcttcgctgt 120

tcgtcgatcg atctacagca cttttcgcct catacaattc ttttcttctt cgatttttcc 180

atcaccagaa acgaatagca gagaatttcc tgactacagc acaaagaaac agaaaaattt 240

ctgacttttt tccccagtgg attagctttc tccggttgtt aattccgtga cgagatgcct 300

gcgctcgctg tcgacgccgc cgcccccgtg gcccacgcct tcgccgcgtc ctgcgacgcg 360

tcgccgcgct tcgccgcgcc gctgctgggc cccgccgccg ccgtcgtcgc tgccacggcc 420

gccgagaagc ccgagatggc cgcgtggtcg gccgacctct cgtcggcgct ctacaacgtg 480

gacggctggg gcgcgcccta cttcttcgtc aacgacgacg gcgacgtcgc ggtgcgcccg 540

cacggcgccg ccacgctgcc cggccaggag atcgacctcg ccaaggtcgt cgcccgggcc 600

gccggcgcgc gcgccggcgg cgggctcggc ctcgccctgc cgctgctcgt gcgcttcccc 660

gacgtgctgc gccaccgcgt cgagaccctc aacgccgcct tcgcctacgc cgtgcgctcc 720

acgggctacg ccgggcggta ccagggcgtc taccccgtca agtgcaacca ggaccggtac 780

gtggtcgagg acatcgtcga gttcggcgcg cccttcgggt tcggcctcga ggccggctcc 840

aagcccgagc tgctgctcac catgagctgc ctcgtcgccc gcggcagccc cgacgccctg 900

ctcatctgca acggctacaa ggacctcgag tacgtctcgc tcgcgctcat cgcccgcacc 960

atgggcctca acaccgtcat cgtgctcgag caggaggagg agctcgacat tgtcgtcgag 1020

gccagccgcc gcctcaacat ccgcccggtg gtcggcatgc gcgccaagct gcgcaccaag 1080

cacgccggcc acttcggcgc cacctccggc gagaagggca agttcggcct caacgccgcg 1140

cagattctct ccgtggtggc caagctcaag gccctcgcca tgctcgactg cctccagctc 1200

ctgcacttcc acattggctc ccagatcccc accaccgccc tgctcgccga cggcgtcggc 1260

gaggccgcgc agatctactg cgagctcgcc cgcctcggcg cggccatgcg cgtcattgac 1320

gtcggcggcg gcctcggcat cgactacgac gggacgcact cggcgcagac cgacatgtcg 1380

gtggcctaca gcctggagga gtacgcggcg gccgtggtcg ccgccgtcgg ccgcgtctgc 1440

gaccgcaagg gcgtgcagca ccctgtcatc ttgcacgaga gccggccgcc ccctggtgtc 1500

ccaccattcg gtcctcattt tcagggcctt cccggaaggg ggccgggttc caaaa 1555

<210> 8
<211> 1413
<212> DNA
<213> Triticum aestivum

<220>
<221> misc_feature
<222> (5)..(5)
<223> n is a, c, g, or t

<220>
<221> misc_feature
<222> (23)..(23)
<223> n is a, c, g, or t

<220>
<221> misc_feature
<222> (29)..(29)
<223> n is a, c, g, or t

<220>
<221> misc_feature
<222> (40)..(40)

<223>  n is a, c, g, or t

<220>
<221>  misc_feature
<222>  (46)..(46)
<223>  n is a, c, g, or t

<220>
<221>  misc_feature
<222>  (49)..(49)
<223>  n is a, c, g, or t

<220>
<221>  misc_feature
<222>  (55)..(55)
<223>  n is a, c, g, or t

<220>
<221>  misc_feature
<222>  (75)..(75)
<223>  n is a, c, g, or t

<220>
<221>  misc_feature
<222>  (78)..(78)
<223>  n is a, c, g, or t

<220>
<221>  misc_feature
<222>  (95)..(95)
<223>  n is a, c, g, or t

<220>
<221>  misc_feature
<222>  (203)..(203)
<223>  n is a, c, g, or t

<400>  8
   gaccnggacg ccactccggg ttncaaccnc caaaaaaatn acacangtng gcccngagcc      60
   gcactccata cggtngangg atctgccgtc cacangacca gaccgacact ccacaacaca     120
   tgggtgtctc cgggaccgca actcggattc cccgccggcc ccgagccgcg ctccacctga     180
   gctgatgacg ctgccgccca cgngaccaga tcaaacactc cacgacaatg ctcttcggag     240
   ccgccgctcc gacttcctca ccggccccga ggccgcgcac acgcctggcc gacgaagaac     300
   ccacaaagcc acccgagcca ccgcgagttg acgaggacca gacctccaag gcgtcgcccc     360
   caagaggaaa gcgacaagaa tgccgccgac gcccgttccg acctgcggcc tgtacgcgga     420
   ccagctgaag cggcgctgcg ccgagcagtt caaggagggg gtcctcgggc tggaacacct     480
   cgccgccgtg gacgggctct gcgagatcgt cgcccgcggc atgggcgcgg cggaggggcc     540
   gcgcaggtac cacatcaacc tgtcggtgtt cacctcgctg ccggacatgt gggccatcga     600
   gcagctcttc cccatcatcc ccatccagcg cctccaggag cggcccgccg tggacggcgt     660
   gctctcggac ctgacctgcg acagcgacgg caaggtggac cagttcatcg gcggcaggag     720
   cagcctcccc ctgcacgagc tccccaccca cggcacccgc ggctactacc tgggcatgtt 780
   cctgggcggc gcctaccagg aggcgctggg cggcctgcac aacctgttcg gcgggccgag 840
   cgtggtgcgc gtctcccaga gcgacggccc gcactgcttc gccgtgacgc gcgccgcgcc 900
   gggcccgtcc tgcgccgacg tgctccgcgc catgcagcac gagccggagg tgatgttcga 960
   ggtgctcaag cagaggacca ccgacgacgg cgccaccgcc gcggccctcg ccagggcgtt 1020
   cgggtcaatg ccgtacctgt cgttcgacgc cgaggaggcc tcggccatga gcggcgcgga 1080
   gtcgagcggc atgagcagcg actcggaggg gtcggctgcc ggcgccgccg aggatgatga 1140
   cgaggagtgg gagttcatgc gcgggctcac cgtgtgaggg cgagctcttt ctatatctat 1200
   caccggtcga accggccggt gatgttgtat cgtgtcactc atggtcgtag cgaggtcgtc 1260
   gggcgtctcg tcgccgttta gtttcaccgc cggagtttgt tttggcgcca tcttttttat 1320
   ttcccatgcg tcagtccact gtttctttca acttttttct cttggaataa ataacaaaag 1380
   gaaactatca gtactatgtt tctttcaaaa aaa 1413

<210> 9
<211> 595
<212> DNA
<213> Triticum aestivum

<220>
<221> misc_feature
<222> (494)..(494)
<223> n is a, c, g, or t

<220>
<221> misc_feature
<222> (515)..(515)
<223> n is a, c, g, or t

<220>
<221> misc_feature
<222> (541)..(541)
<223> n is a, c, g, or t

<220>
<221> misc_feature
<222> (548)..(548)
<223> n is a, c, g, or t

<220>
<221> misc_feature
<222> (560)..(560)
<223> n is a, c, g, or t

<220>
<221> misc_feature
<222> (579)..(579)
<223> n is a, c, g, or t

<220>
<221>  misc_feature
<222>  (582)..(582)
<223>  n is a, c, g, or t

<220>
<221>  misc_feature
<222>  (591)..(591)
<223>  n is a, c, g, or t

<400>  9
gaggacctcc gactacggcg ccaccatcgc ggccctcgcc agggcgttcg ggtcaatgcc      60

gtacctgtcg ttcgacgccg aggaggcctc ggccatgagc ggcgcggagt cgagcggcat     120

gagcagcgac tcggaggggt cggcggaggg cgccgccgag gatgatgacg aggagtggga     180

gttcatgcgc gggctcaccg tgtgaccacg gcgagcttat actctcctgt atctgtcgcc     240

ggtccaaccg gccggcgatg tttactttac cagccagcaa gcggcagctg ctgccggtgt     300

tgtatcgtgt tactcatggt cgtagcgagg tcgtcgggcg tctcgtcgcc gtttagtttc     360

gccggagttt gttttggcgc catcttttta tttcccatgc gtcggtccac tgtttctttc     420

aaccttttct tcttgggaat aaataacaaa aggaaactag cagtagtatg tttctttcga     480

ataagaaaaa aganatcaaa agggggggcc ggtancaatt cccctatagg agtctatacg     540

ncgctcantg gcgtctttan agtctgacgg aaaacctgng tnccaactaa nccct          595

<210>  10
<211>  350
<212>  DNA
<213>  Triticum aestivum

<400>  10
gcacgaggga ggaggcggtg gccatgagcg gcgcggagtc gagcggtatg agcagcgact      60

cggaggggtc ggccgccggc gccgccgagg atgatgacga ggagtgggag ttcatgcgcg     120

ggctcaccgt gtgagggcga gctctttcta tatctatcgc cggtccaacc ggccggtgat     180

gttgtatcgt gtccatggtc gtagtgaggt cgtcgggcgt ctcgtcgccg tttagtttcg     240

ccggagttcg ttttggcgcc atctttttta tttcccatgc gtcggtccac tgttttcttc     300

ttggatggaa taaataacaa aaggaaacta gctgtactat gtttctttcg               350

<210>  11
<211>  2122
<212>  DNA
<213>  Hordeum vulgare

<400>  11
cacgcgtccg cccacaacca aatatataca agtctcctcc aatcttcgct ggtgctttga      60

cgatggccac caagaactat ggtgaattgt acaacatgct cggctggggt gaaccttact     120

tcatcgtgaa cacggatggc cacctatgtg tcaagaccaa cggccacgag acaaagccag 180
ggcaagagat agacgtggtg tccgttgttg aagctgcaaa ggaagagggc gtccagtttc 240
cgatgatcct ccgcttcccc ggcgtgctca agcaccgcgt cgactcgctc cacgttgcat 300
tcgagaacgc catcaagcac accgggtaca cggcgccata tcagggcgtg tacccagtga 360
aggtgaatca gaacaaggcc atcatcaacg actttgttac cttcggccac cgttatagct 420
acgggctaga ggcgggctcc aagccggaat tgctcatcgc aatgagctac cttaccaaag 480
ccaagcccgg agccttcttg gtgtgcaatg gctacaagga cgcggagtat gtggcgctgg 540
cgctctcggc gcgtgccatg ggcctcaatg tcatcatcgt gttggagatg gaggaggagc 600
tggacatcgt catcgaccag agcaacaagc tcggggtgga gccggtgctg ggcgtgcgtg 660
ccaagcttct caccaagatt ccaggccact tcgggtcgac ggccggcaag catggcaagt 720
tcggcctcct cgcggagaag atctacgagg tggccaagaa gctccagggt ctcaacaagc 780
tgcactggct caagctgcta cactttcaca tcggctccat gatcccgacc acggacattg 840
tgtccaatgc agccagggag gcctccggca tctactgcac tctggtgaac cagtatggcg 900
cgcagatgac cacgctggac tgcggcggag ggctcggcgt cgactacgac ggcacccggt 960
caggtagctc cgacatgtcg gtggcatatg ggttggagga gtatgcgtcc agcattgtgc 1020
aggcggtgcg gctcacgtgc gagtacaatg gtggcgttcc ccaccctgtt ttgtgcaccg 1080
agagcggccg cgccatggtg tcgcatcact cgatgatcat cctcgaggcc ctctcggcga 1140
tccctgagcc caaggacgac gatacccccg agcagctgca cggcaagatc cagcatcttt 1200
catccaatgt gccgtcgccg agagcactcg tggccacgga cctccagaag cacgccaccg 1260
acatccagaa gcacgggatc gagatgtaca agctggccaa gaagctctcc aagcggatcg 1320
ccggggacgc caacaccatc tacaactacc acatgaacct ctccatcttc tcgctcgttc 1380
ccgacttctg gggcatcaag cacctcttcc cgatgatgcc cgcgagccgg ctcaacgaga 1440
agccgactca aatgggcacg ctcatcgaca tcacatgcga cagcgacgga aaggttgaca 1500
agttcatcgg caatgccgac acgctgccgc tgcacacgct ggaccctgag agcgacggct 1560
actacgtggc cgtgctcctg tccggcgcgt accaggaagc cctttcttgc aagcataacc 1620
tgttcggcgg cccaagcata gtccgggtcg agaacaacac cgacggtggt ggtggcttcc 1680
acatcgccac agccgacctt ggcccgacga cggaggagct catcagcacc gtggggtacg 1740
atgtcaagga ggacattcgc ggcgtgatcg aaaaatgggc cagggagaag gaggtgtggg 1800
agaaggtggg ggtgttcgtg gaggctgggc ttacctccat gccctacctc gtcgaataca 1860
aggtcccgcg aaccgcctaa tttcatcgtg attcatattt catgctattt cttttatcca 1920

cactttggct ttaaataagc gtagccggca tgcctgcctc attcgtacgt gtaggacagg 1980

ctttgggttc cgccattcca cgccatgctt tgccattttc tattgaagaa taaataaagt 2040

tcatctcccc ttctcatttg tttactctta tttatgttca gaaaaataaa taaaggttat 2100

aataactaaa ttgaattgat tt 2122

<210> 12
<211> 751
<212> DNA
<213> Hordeum vulgare

<400> 12
ccgcaaccgt ctcccctcgc tcgctcgctc gtccatctcc catatctcgc cgcagcgcgc 60

gcatcagcac agcagagatc cctccaccgc ccggcgagcg aaagcaaaac ctagcgccgc 120

ctcggctcgc cgtccccgcc gtcttcttcg catccgactg aacgccggcg tggggtgccc 180

agcctccaca accggcgacg cgaccctccc cgccgcgcgc tccccacccg cctgctcctt 240

ttgcgggggt agccggggct ccggcctcgg cggctttcta agcccccttc cttacaacag 300

gatccaaaat cccgtcctag atttcttcta cctccccttc cagaacttcc tcttttggca 360

tcagtgatcg ccggaatctc cccctccccc ccagtagatt tcgtcgattc cttgttgtaa 420

tttttgcctc cattttcttc ttcttcttcc ttcgatcgat cgatctacag ccggtttcgc 480

ctcatacaga ttcttttctt cttcgagatt tgtccattcc atcgccgcta acctccgtcg 540

ccgccaccag aaacaaacag cacagaaatt aacgtataca gcacaaagaa acaaaaaaaa 600

ttcctgactt ttccccccct tcccagtgga ttagctttct ccggtttgtg acgagatgcc 660

tgcgctcgct gtggacgctg ccgcccccgt ggcccacgcc ttcgccgcgt gcgacgcgtc 720

gccgcgtttc gccgctcccc tgctgggccc c 751

<210> 13
<211> 1917
<212> DNA
<213> Hordeum vulgare

<400> 13
gaccgctacg tcgtcgagga catcgttgag ttcggtgcgc ccttcgggtt cggcctcgag 60

gccggctcca agcccgagct gctgctcacc atgagctgcc tcgtcgcccg cggcagcccc 120

gacgccctgc tcatctgcaa cggctacaag gacctcgagt acgtctcgct cgcgctcatc 180

gcccgcacca tgggcctcaa caccgtcatc gtgctcgagc aggaggagga gctcgacatt 240

gtcgtcgagg ccagccgccg cctcaacatc cgccccgtcg tcggcatgcg cgccaagctg 300

cgcaccaagc acgccggcca cttcggcgcc acctccggcg agaagggcaa gttcggcctc 360

aacgccgcgc agattctctc cgtggtggcc aagctcaagg ccctcggcat gctcgactgc 420

ctccagctcc tgcacttcca cattggctcc cagatcccga ccaccgctct gctcgccgac 480

ggcgtcggcg aggccgcgca gatctactgc gagctcgccc gactcggcgc ggccatgcgc 540

gtcattgacg ttggtggcgg cctcggcatc gactacgacg ggacgcactc tgcggagacc 600

gacatgtcgg tggcctacag cctggaggag tacgcggcgg ccgtggttgc cgccgtcggc 660

cgcgtctgcg accgcaaggg cgtgcagcac cctgtcatct gcagcgagag cggccgcgcg 720

ctggtgtcgc accactcggt cctcatcttc gaggccttct ccgcgacggc gccggcttcc 780

aacatgatgg acccggccac cgcctacctg ctcgacgagc tcaccgacga ctgccgcaac 840

gactaccgca acctcatggc gtccgccgtg cgcggcgact tcgacacctg cggcctgtac 900

gccgaccagc tgaagcggcg ctgcgccgag cagttcaagg agggggtcct cggcctggag 960

cacctcgccg ccgtggacgg gctctgcgag atcgtcgccc gcggcatggg cgcggcggag 1020

gggccgcgca ggtaccacat caacctgtcg gtgttcacct ccctgccgga catgtgggcg 1080

atcgagcagc tgttccccat catccccatc cagcgcctcc aggagcgccc agccgtggac 1140

ggcgtgctct cggacctgac ctgcgacagc gacggcaagg tggaccagtt catcggcggc 1200

aggagcagcc tcccactgca cgagctcccg agccacggca cccgcggcta ctacctgggc 1260

atgttcctgg gcggcgccta ccaggaggcg ctgggcggcc tgcacaacct gttcggcggg 1320

ccgagcgtgg tgcgcgtgtc gcagagcgac gggccacact gcttcgccgt gacgcgcgcc 1380

gcgccggggc cgtcctgcgc cgacgtgctc cgcgccatgc agcacgagcc ggaggtgatg 1440

ttcgaggtgc tcaagcagag gaccaccgac gacggcgcga ccgcggcggc cctcgccagg 1500

gcgttcgggt caatgccgta cctgtcgttc gacgccgagg aggcggcggc gatgagcagc 1560

gcggagtcga gcggcatgag cagcgactcg gaggggtcgg cggccggcgc cgccgaggac 1620

gatgacgagg agtgggagtt catgcgcggg ctcaccgtgt gacggcgagc tcttttctgt 1680

acctattatc accggcccaa ccggccggtg atgtttacca gccagcggca gcagccggtg 1740

ttgtatcgtg tccatggtcg tagtgaggtc gtcgggcgtc tcgtcgccgt ttagtttcgc 1800

cgccggagtt tgttttggcg ccaccttttt cattcccatg cgtcagtcca ctgtttcgtt 1860

cgtccctttt tttctcttgg aataacaaaa ggaaactatt actactaaaa aaagctt 1917

<210> 14
<211> 1374
<212> DNA
<213> Oryza sativa

<400> 14
atggcagcag cagaaggata tcagatggag gccgccgtgc tcgcggcgcc cgtggtgaag 60

gaccgggagg tgctccgcct ccggcgagac gcggccggcc gtcgagacgt taccggactc 120

gtgcggacca tcgtcgccgc ggcaccgccg cccgacagta cgacccgcac cgcggcggcg 180

ggcgcgttca acgtgatcga cctcggcgag gtggcgcggc tgttcgcggc gtggtggcgc 240

gggctccgcg gcgtgcgtcc ctactacgcc gtcaagtgca accccaaccc ggcgctgctc 300

ggcgcgctcg cggggctcgg cgccggcttc gactgcgcca gccgcgccga gatggaggcc 360

gtgctggcgc tcggcgtcgc ggccgaccgc gtcgtctacg cgaacccgtg caagctggag 420

ccccacctgg agtacgcggc cggcgtcggc gtcgacctca ccaccttcga ctccgaggag 480

gaggtcggca agatcaagcg ctgccacccg gggtgccggc tgctgctacg gatcaaggcc 540

cccgacggcg acgatggcgg cggcgccatg ctaaacttgg gaaccaagta cggcgcgcac 600

cgggacgagg tggtgccgct cctcgccgcc gcgcggcgag ccgggatggc cgtcgtcggc 660

gtctcgttcc acgtcgggag cgccgtgtcc cgcgtcggca tctacgccgc cgccatcgag 720

gcggcccgcg ttcgacgccg ccgccgcgct cggcatgccg cgcatgcacg tgctagacat 780

cggcggggga ttcaaggccg gcggcggcgg cggcgaatcc acgttccagg aggcaagcgc 840

cgtgatcagc gcggccctcg cgaggcactt cggtggcgac gacatgccca gcggcgtcga 900

ggtgatagcc gagccggggc gctacttcgc cgagacggcg ttcgcgctcg ccgcccgcat 960

cttcgggaag cgcacgcgcg gcgaggtgcg cgagtactgg atcgacgacg gcatgttcgg 1020

caccctgtgc tgcgtgcact tcgagaacta cgtgccgcgc ccggcgccgg tgaccgccac 1080

cgccgacgac ggcgacgagc aggcggcgac catgggcggc gagatgatga tcacgtcgac 1140

gaacacgtcg acgaagacgc acccgtcgac ggtgttcggc cccacgctgg actcgttcga 1200

cgaggtggtg aggggttatc agctgccgga gctgtgcacc ggggactggc tggtgttcga 1260

cgacgtcggc gcgtacacca ccgtgtgcag ctcagacttc aacgggttct ccacgtccaa 1320

catgaagacc tacctcgcct actcctgcta gatcactggg aaagtgccac ctaa 1374

<210> 15
<211> 1240
<212> DNA
<213> Oryza sativa

<400> 15
atggctggag gaagaccctt ggagtcggtg ctcgtggccc ccggggtgaa gggaaagaag 60

gtactcgcat tcaagcgcga cgggctcaag aaaaacgagg cagtgacggg cctcatccac 120

gacatcgtcg ccagctccag cgccaggagc gcgttccacg tcctggacct cgccaaggtg 180

gtcgacctct acgcgggatg gcgccgcgcg ctccccggcg tgaggccctt ctacgccgtc 240

aagtgcaacc cggacacggc gctgctcggc gcgctcgccg cgctcggcgc cggcttcgac 300

tgcgccagcc gcgccgagat cgaggccgtc ctcgcgctcg gcgtcccgcc cgccgccatc 360

gtctacgcta acccgtgcaa gcccggcgcg cacgtcgcgt tcgccgccga ggccggcgtc 420

aacgtcacca cgtacgattc cgaggaggag gtggccaagg tgaagcgctg ccaccccagt 480

tgcgagctgc tgctccgcat caaggcgccg gactgcggcg gcgtcaaggt gcgacctcgg 540

cctcaagtac cgcgcgcaac ccggaacaag gtgctgccgc tgctgcgcgc cgcgccgccg 600

cgcgggcctc ggcgtggccg gcgtgtcgtt ccacgtcggc agcggcgcgt cccgcgcggc 660

cgtctaccgc ggcgccatcg aggccgcgcg cgcggcgtcc aacgcgccgc ggggctcggg 720

atgccgccga tgcgcgtcct gaacatcgcc ggcgggttcg tccgccggcg ccgcgttcgg 780

cgacgcgccg ccggtaattc aaccgcgcgc tcgggcgcta ctttggcgac actcctcccc 840

accgtcgagt tgatccggcg agcccggccg gtacttcgcc gagaccgcct tcacgctcgc 900

cgcgcgcgtc atcgggaagc gcaggcgcgg cgacgtgcgc gagtactgga tcgacgacgg 960

cgtctacggc tcgctcaact gcatcctcct ggacagctac gtgccgcgcc cgaggccgct 1020

cgccggcgcg cgccccggcg aggagacgca cgcctcgacg gtgttcgggc cgacgtgcga 1080

ctccatcgac acggtggtga ccgggtacca gctgccggag atgagcgtcg acgactggct 1140

tgtgttcgac gacatgggcg cctacaccac tgcggccggc tccagcttca acggctttgc 1200

cacgtcagcc ataaacacct acctggcata ctccagctag 1240

<210> 16
<211> 1227
<212> DNA
<213> Oryza sativa

<400> 16
atgcaggccg tgctcatggc ccccggcgtc aaggacaaga aggtgctcgc cttcaagcgc 60

ggcaagggca aggacgccga cgccggcgtc acggcgctca tccgcgacat cgtcgccggc 120

ggcgcccgga gcgcgttcca cgtcttcgac ctcgccaagg tcgtcgacct ccaccgcggg 180

tggcgccgcg cgctccccga cgtgcgcccc tgctacgccg tcaagtgcaa ccccgacggc 240

gccatgctcg ccgcgctcgc cgcgctcggc gccggcttcg actgcgccag ccgcgccgag 300

atcgaggcag tgctcgcgct cggcgtccgc cccgccacca tcgtctacgc caacccgtgc 360

aagcccgagg cgcacctcga gtacgccgcc gaggtcggcg tcaacctcac cacctacgac 420

tccgaggagg aggtggccaa ggtccggcga tgccacccgc gctgcgagct gctgctccgc 480

atcaaggcgc ccgactccgg cgacgccaag gtcgacctgg ggctcaagta cggcgccaac 540

ccggacgagg tgctaccgct cctccgcgcc gcgcagcgcg agggcgtcgc cgtcgccggc 600

gtctccttcc acgtcgggag cggcgcgtcc cgcgccgacg tctaccgcgg cgccatcgag 660

gcggcgcgcg aggcgttcga cgccgcggcg gcgctcggca tgccgccgat gcgcgtcctc 720

gacatcggcg gcggcttcat ggccggccgg accttcgacg aggcggcggc ggtcatcaac 780

cgcgcgctgg agcgccactt cggcgacctc ccctgcgtcg aggtgatcgg cgagcccggg 840

cggtacttcg ccgagacggc gttcaccctc gccgcgcgcg tcatcgggaa gcgcacccgc 900

ggcgagctcc gggagtactg gatcgacgac ggcctctacg gctccctcaa ctgcatcctc 960

atggaccact acgtgccgcg cccgaggccg ctcgccgccg ccgccgccgg cgaggacacg 1020

acggcggcga cgacgcacgc ctcgacggtg ttcggcccga cgtgcgactc cctcgacacg 1080

gtggtcaccg gctaccagct gccggagatg agcgtcggcg actggctcgt gttcgacgac 1140

atgggcgcct acaccaccgc ggctggctcc aacttcaacg gcttcgccac gtcagcgata 1200

aagatccacc tggcatactc cagctag 1227

<210> 17
<211> 756
<212> DNA
<213> Triticum aestivum

<400> 17
gattcggccg agggtggacc tctggaccaa gtacggcgcg cacgaggacg aggtcatgcc 60

gctcctccgc gccgcccagc gcgaggggct caacgtggcc ggcgtgtcgt tccacgtcgg 120

gagcggcgcg tccaacacgg acgtgtaccg gggcgccatc gaggccgcgc gcggggtctt 180

cgacgcggca gccgggctcg gcatgccgcc catgcgcgtg ctcgatatcg gcggcgggtt 240

catggccggc ccggcgttcg acgaggcggc cgcggtcatc aacgcggcgc tcgagcgcta 300

cttcggggag cttccgtgcg tggaggtgat aggcgagccg gggaggtact tcgccgagac 360

cgccttcacg atggcggcgc gggtcatcgg gaagcgcact cgcggtgagg tacgcgagta 420

ctggatcgac gacggcctct acggcaccct cagctgcatc cccatggacc actacgtgcc 480

gcacccgagg ccgctcgccg tgccgcgcgc cggcgagaag acgtacacgt cgacggtgtt 540

cgggccgacg tgcgactccc tcgacacggt ggtgaccggg taccagctgc cagagatgag 600

cgtgggggac tggctcgtgt tcgacgacat gggcgcctac accaccgcct ccggctccaa 660

cttcaacggc ttctccacct cggacatcaa gacctacttg gcctactcca gctgatatgg 720

aactagtcat gtggccacgt actacgtagc acacgc 756

<210> 18
<211> 797
<212> DNA
<213> Triticum aestivum

<400> 18
attcggcacg agggcgcgag gggctcaacg tggctggcgt gtcgttccac gtcggcagcg 60

gcgcgtccaa cacggacgtg taccggggcg ccattgaggc cgcgcgcggg gcctacgacg 120

cggcagccgg gctcggcatg ccgcccatgc gcgtgctcga tatcggcggc gggttcatgg 180

ccggcccggc gttcgacgag gcggccgcgg tcatcaacgc ggcgctcgag cgttacttcg 240

gggagcttcc ctgcgtggag gtgataggcg agccggggag gtacttcgcc gagaccgcct 300

tcacaatggc ggcgcgggtc atcgggaagc gcactcgcgg cgaggtacgc gagtactgga 360

tcgacgacgg cctctacggc accctcagct gcatccccat ggaccactac gtgccccaac 420

cgaggccgct cgccgtgccg cgcgccggcg agaagacgta cacgtcgacg gtgttcgggc 480

cgacgtgcga ctccctcgac acggtggtga ccgggtacca gctgccagag atgagcgtag 540

gggactggct cgtgttcgac gacatgggcg cctacaccac cgcctccggc tccaacttca 600

acggcttctc cacctcggac atcaagacct acttggccta ctcaagctga tatggaactt 660

atgatgtggc cacgtactac gtagcacacg ccattcgatt tgttttcgca gtacaatgaa 720

gagctgtaat taagctcgta cacaatgtat gtgcttttgt ttgttctatt ttttatttta 780

attaggtata tatatgt 797

<210> 19
<211> 720
<212> DNA
<213> Triticum aestivum

<400> 19
ccacgcgtcc gacgccaaga ggcattcccc gagccagcga tcttctcgcc gcacttcaca 60

cagttgctcg tgcacgtgcc acgccccgag aaaccgtaac ttaccacgca gagctaggct 120

ctcattgttt aagtgagata ttcacagtcg catccgtcgt cgagatggtt gggagcagcc 180

cgatgcaggc ggtgctggtg gcgccggggg tgaaggacaa gaaggtgctc gcgttccgcc 240

gggacgcgct caaggagaag gacgccgtcg ccgcgctcat gcgcaccatc gcggccggcg 300

gcgtgcggag cgccttctac gtcttcgacc tcgcccgggt cgtcgacctg taccgcggct 360

ggcgccgcgc gctccccggc gtgcgcgcct gctacgccgt caagtgcaac ccggagccgg 420

cgctactcgg cgcgctggcc gcgctcgggg gccggcttcg actgcgccca gccgcaggga 480

gatcgaggct gtgctcgctc tcggcgtgca gcccggcagc atcgtgtacg ccaacccgtg 540

caagccggag gcccacctcg agtacgcggc tgaggtgggc gtccacctca ccacctatga 600

ctccgaggag gaggtgggta agggtcaggc gcttgcaccc gggcttggac ctcatcctcc 660

gcatcaaggc cccgaaggcg ggaaggcccg gggtggccct cggcaccaag ttcggggcgg 720

<210> 20
<211> 934
<212> DNA

<213>  Triticum aestivum

<400>  20
gattcggccg agggtggacc tcggcaccaa gtacggcgcg cacgaggacg aggtcatgcc      60

gctcctccgc gccgcccagc gcgaggggct caacgtggcc ggcgtgtcgt tccacgtcgg     120

gagcggcgcg tccaacacgg acgtgtaccg gggcgccatt gaggccgcgc gcggggtctt     180

cgacgcggca gccgggctcg gcatgccgcc catgcgcgtg ctcgatatcg gcggcgggtt     240

catggccggc ccggcgttcg acgaggcggc cgcggtcatc aacgcggcgc tcgagcgcta     300

cttcggggag cttccctgcg tggaggtgat aggcgagccg gggaggtact tcgccgagac     360

cgccttcacg atggcggcgc gggtcatcgg gaagcgcact cgcggtgagg tacgcgagta     420

ctggatcgac gacggcctct acggcaccct cagctgcatc cccatggacc actacgtgcc     480

ccaaccgagg ccgctcgccg tgccgcgcgc cggcgagaag acgtacacgt cgacggtgtt     540

cgggccgacg tgcgactccc tcgacacggt ggtgaccggg taccagctgc cagagatgag     600

cgtaggggac tggctcgtgt tcgacgacat gggcgcctac accaccgcct ccggctccaa     660

cttcaacggc ttctccacct cggacatcaa gacctacttg gcctactcaa gctgatatgg     720

aactaatcat gtggccacgt actacgtagc acacgccatt cgatttgttt tcgcagtaca     780

atgaagagct gtaattaagc tcgtacacaa tgtatgtgct tttgtttgtt ctatttttct     840

aattttaatt aggcatatac atatatgtat ccaatgtgga tcgttattct tttgtttcct     900

aaaatgaaac tttgttatca acaatttttt ttcc                                 934


<210>  21
<211>  771
<212>  DNA
<213>  Triticum aestivum

<400>  21
ccgcccgcgc ggcgttcgac gcggccgcgc agctcggcat gccgcccatg cgcgtgctcg      60

acatcggcgg cggcttcatg ccggacgcca cgttcgacga ggcggcagag gccatcaacg     120

atgcgctcgc gcagcacttc ccgcccgggt gcggcgtgga ggtgatcggc gagccggggc     180

agtacttcgc cgagaaggcc ttcacgctcg cggcgcgggt catcgggagg cgcgcgcgcg     240

gcgaggcgcg cgagtactgg atcgacgacg gcatctacgg cgccctcagc tgcaccatct     300

tgggcgacta cgtgccgcgc ccgaggccgc tggcgccccc gcgtcccggc ggcgagaaca     360

tgtgcagtac gtacgcgtcg acggtgttcg ggccgacgtg cgactcccgc gacaaggtgg     420

tgaccgggta ccagctgccg gagatgaacg tgggggactg gctcgtgttc gacggcatag     480

gcgcgtacgc cgcctcgtcg ggcaccaact tcaatggatt cttgatcgcc gacataaaga     540

cgcacttggc ctactccacc tagctccaaa cgtccaatgg ctccgggaag gaacatgcac     600

atgcacatgc acatgcacat ccatgcatgg gcacgtccga aattattggc gtcgattggt 660

gtttgtattg catgtacctt tcttctttgt tttgtttttt tttttgcagc gcggggtttc 720

ttcttgacag tctatatctt gttgtattct cacacataga aagaaaaatt g 771

<210> 22
<211> 526
<212> DNA
<213> Hordeum vulgare

<400> 22
cgccaagaag cattcccccg agccagcgat cttctcgccg cattccacac tgttgtccgt 60

gcacgtgcgc cggcccgagc taggctctca ttgtttaagt gtgaccatca gcacagtcgt 120

cgtcgtcgtc gagatggttg ggagcagccc gatgcaggcg gtgctggtgg cgccgggggt 180

gaaggacaag aaggtgctgg cgttcaggcg ggacgcgctc aaggagaagg acgccgtatc 240

cgcgctcatg cgcaccatcg cggccggcgg cgtgcggagc gccttctacg tcttcgacct 300

cgcccgggtc gtcgacctgt accgcggatg gcgccgcgcg ctccccggcg tgcggtcctg 360

ctacgccgtc aagtgcaacc cggagccggc gctgctcggc gcgctggccg cgctcggggc 420

gggcttcgac tgcgccagcc gcagggagat ggaggccgtg ctcgcgctcg gcgtgcagcc 480

cggcagcatt gtgtacgcca acccgtgcaa gcccgaggcg cacctc 526

<210> 23
<211> 717
<212> DNA
<213> Oryza sativa

<400> 23
atggtggaaa accgtcttgt tggcatgaaa tcgcacagtg tctatgagac ccttggtgac 60

accataatgg cagctgccat tcgtgagctc gagtccctga cccttgatag ggagatcatg 120

cagtggaaag acattcttgc cctcaaaata cgccaagttg gtttatgttg gccgctggtt 180

cgacccactc cagcaatcca tggatgcctt catggagaag agcccctaca gcctgtacgg 240

ggaggacatt tcctacagca tgggatcgga cgcattgata tggtggaaaa ccgtcttgtt 300

ggcatgaaat cgcacggtgt ctacaagacc cccggtgaca ccataatggc agctgccgtt 360

cgtgagctcg agtccctgac ccttgatagg gagatcatgc agtggaaaga cattcttgcc 420

ctcaagtaca ccaagttggt ttatgttggc cgctggttcg acccgctcca gcaatccatg 480

gatgccttca tggagaaggt taccgagaca acaactggtt cggttaccct caagttgtac 540

aagggttcag taaacattgc atcccggaag agcccctaca tcctgtacgg ggaggacatt 600

tcctcgttcg agaatggaaa gatatataac caggcaaaaa cgctgaggga ttcatcaggt 660

tgtatggtct ccccacacga gtgcgggcag tgctggacaa gggtctataa gatttag 717

<210> 24
<211> 1446
<212> DNA
<213> Oryza sativa

<400> 24
atggcgcacg ccctcggctg cggcgccgcc accgcccccg ccggggtggg tctcctccac 60

caccacgaga aggctggggc gttgttcagt gccgcaggct ccatcgttcg ggttcagcag 120

ctcagcggga aggggagcaa aagccaagct ataagatgtg caatgccaag tgcaagtgag 180

catggtggaa tttcaaccgc aacagctagt agtagtgatc aaaagaaagg tggactgcgt 240

ggcaaactga ataaagttgt tctagcttac agtggtggtt tggatacatc agttattgtt 300

ccatggctcc gggagaacta tggttgtgaa gttgtctgtt tcactgctga tgttggccag 360

ggtgacattg aattggaagg gctagagaag aaggcaaaag ctagtggtgc aagccagctt 420

gttgtgaagg acttgaaaga agagttcgtt agtgaataca tttacccctg cttgcgtgct 480

ggtgcagttt atgaaagaaa gtatctgctg gggacttcaa tggctaggcc tgttattgcc 540

aaggcaatgg ttgatgttgc aaaggaagtt ggtgcagatg cagttgctca tggatgcact 600

ggaaaaggca atgatcaggt tcgttttgag cttacattct atgctttaaa ccctgagctt 660

aaagtggtgg caccttggag ggagtgggat atcacaggac gtgaagatgc tattgagtat 720

gcaaagaaac acaatgtgcc aattcctgtt acaaagaaat caatatacag ccgtgatcga 780

aatttgtggc accttagcca tgagggtgac attttggaag atccagcaaa tgagccaaag 840

gaagatatgt atatgatgtc agttgcacca gaaaatgcac cttcaaaacc tgagtatttg 900

gagattggta ttattgcagg tgttcctgtt tccatcaatg gaaaagatct ctcaccagca 960

tctctccttg ccaagctcaa tgaaatcggt ggaaagcatg gtattgggcg cattgatatg 1020

gtggaaaacc gcctcgtagg catgaagtcc cgtggtgtat acgagacccc cggtggcacc 1080

atcatggcag ctgctgtccg tgagctcgag tccttgactt tggacaggga gacaatgcag 1140

tggaaggaca tgcttgccct caagtacgcc gagctggtct acgctggccg ttggttcgac 1200

ccgctccgac aatccattga tgcgttcatg gagaacatta ccgcgacaac aaccggctcc 1260

gtgactctca agctgtacaa gggctccgta aacgtcgcgt cccggaagag cccatacagc 1320

ctgtacaggg aagacatctc ctcatttgag aatggggaga tctacaacca ggctgatgct 1380

gaagggttca tcaggctata tggtctccct accagagtcc gggcaatgct ggagaagggc 1440

atctaa 1446

<210> 25

<211>  1745
<212>  DNA
<213>  Triticum aestivum


<220>
<221>  misc_feature
<222>  (1736)..(1736)
<223>  n is a, c, g, or t

<400>  25
ctaccccgct tgtagccgcc gcgtctgacc caagccttcc acttccaccc agccgcccgc      60

cccccatgcc gctcccgatg cgccgcctcg ccgccgctcc ctccctccca gccgtgcacc     120

cgcacgactc ccccgcgccg ccctccgttc ggctccggcg acagcatggc cgggtgtcga     180

tggagaggtc ctcgacgacg acggcccgag gtgttagttg cgtgatggca agtggaaagg     240

agcatgcagc aactactagt ggtgatgaaa agaaaggtgg actgcgtggc aaactgaaaa     300

aagttgttct agcttacagc ggtggcttgg atacatcagt aattgttcca tggcttaggg     360

agaactatgg ctgtgaagtc gtctgtttca ctgctgatgt tggtcagggt gacattgaat     420

tggaaggatt ggagaaaaag gcaaaagcta gcggtgcaag ccagcttgtt gtgaaggact     480

tgacagaaga atttgttggt gaatatatat acccctgctt gcgtgctggt gcagtttacg     540

aaagaaagta tctgcttggg acttccatgg ctaggcctgt tattgctaag gcaatggttg     600

atgttgccaa ggaagttggc gcagatgcag ttgctcatgg gtgcactggg aagggcaatg     660

atcaggttcg ctttgagctt acattttatg ccttaaatcc ggaacttaaa gtggtggcac     720

cctggaggga gtgggatatc acaggacgtg aagatgcaat tgagtatgca aagaaacaca     780

atgtgccaat tccggtttca aagaaatcaa tatacagccg agatagaaat ttgtggcacc     840

ttagccatga gggtgacatt ttggaagatc cagcgaatga gccaaaagaa gatatgtata     900

tgatgtctgt ttctccagaa aatgcacctt caaaacctga gtatctggag attggcatag     960

tcgctggcgt ccctacttct atcaacggta aagatctctc gccagcaact ctcctcgcca    1020

agctcaacga aatcggcgga aagcacggca tcgggcgcat cgacatggtg gagaaccgcc    1080

tggtggggat gaagtcccgt ggcgtctacg agacccccgg cggcaccatc atggcggccg    1140

cggtccgcga gctcgagtcc ctgacactgg accgggagac gatgcagtgg aaggacatgc    1200

tcgccctcaa gtacgcggag ctcgtctacg cggggcgctg gttcgacccg ctgcggctgt    1260

ccatggacgc cttcatggag accatcaccg cgacgacgac cgggtcggtg accctgaagc    1320

tgtacaaggg gtcggtgacc gtcgcgtccc ggaagagccc ctacagcctg tacagggagg    1380

acatctcgtc gttcgagaac ggggagatct atgaccaggc tgacgctgag ggtttcatca    1440

ggctgtacgg cctccccacc cgggtccgcg cgatgctgga gaagggcatc taatctaagg    1500

tcgtctttgc gtctcgaata aattgttttc tcattgaaaa ctgccgagtg tgtgtctttg 1560

agtaactctg gacaaatgga agatttccag gcgggtgggc tttgagcaca ttgtggagtt 1620

tctgttttgt ttggtagtat gtattgaaga gttgctccaa cttgcagtaa tatactgttt 1680

ggtgctgcgg ccgggtgtgt tcggttactc gcatcatttt tgggcttcct ggaccnaccc 1740

gtaga 1745

<210> 26
<211> 820
<212> DNA
<213> Triticum aestivum

<400> 26
tcgccagcaa ctctcctcac caagctcaac gaaatcggcg gaaagcacgg catcgggcgc 60

atcgacatgg tggagaaccg cctggtgggg atgaagtccc gcggcgtcta cgagaccccc 120

ggcggcacca tcatggcggc cgcggtccgc gagctcgagt ccctgacact ggacagggag 180

acgatgcagt ggaaggacat gctggccctc aagtacgcgg agctcgtcta cgcggggcgc 240

tggttcgacc cgctccggct gtccatggac gccttcatgg agaccatcac cgcgacaacg 300

accggctcgg tgaccctgaa gctgtacaag gggtcggtga cggtcgcgtc ccggaagagc 360

ccctacagcc tttacaggga ggacatctcg tcgttcgaga acggggagat ctacgaccag 420

gctgacgccg aaggtttcat caggctgtac ggcctcccca cccgggtccg cgcgatgctg 480

gagaagggca tctaaaggcc gtctttgcgt ctcgaataaa ttgttttctc attgaaaact 540

gccgagtgtg tctttgagta actgtagaca aatggaagat ttccaggcgg gtgggctttt 600

tgggcacatt gtggagtttc tgttttgtgt ggtagccttt tcatccggtg tgcgtttgaa 660

gagttgctcc aacttgcggt aatatactgt tcggtgctgc agtgcactcc gggtgtgttt 720

ggttacttgc atcattttag gcttcctgga actttgaccc ggtagagcct gtatgggcta 780

atacgaggta tcggtcaagc tgtgcagttt gtttggttgc 820

<210> 27
<211> 607
<212> DNA
<213> Triticum aestivum

<400> 27
tttggaacta aaatggcgga tgcttcgttt tagcctcaca tacttggatg attcgaaggc 60

accaagtgta aagtaaatag acaaggaatg cacggcagca ccaaacagta tattacggca 120

agttggagca actcctcaat acatatatcc aggcacaccg gatgaaaagg ctaccaaaca 180

aaacagaaaa ctccacaatg tgctcaaagc ccacccacct ggaaatctct tccatttgtt 240

cacagctact caaagacaca ctcggcagtt ttcaatgaga aaacaattta ttcgagacgc 300

aaagccgacc ttcagattcc cttctccagc atcgcgcgga cccgggtggg gaggccgtac 360

agcctgatga aaccttccgc gtcagcctgg tcgtagatct ccccgttctc gaacgacgag 420

atgtcctccc tgtacaggct gtaggggctc ttccgggacg cgaccgtcac cgaccccttg 480

tacagcttca gggtcaccga gccggtcgtc gtcgcggtga tggtctccat gaaggcgtcc 540

atggacagcc ggagcgggtc gaaccagcgc cccgcgtaga cgagctccgc gtacttgagg 600

gccagca 607

<210> 28
<211> 1800
<212> DNA
<213> Hordeum vulgare

<400> 28
cggcacgagg ccattagccg cctcgtctga cccaagtctt ccacccccgc cgcccaccat 60

gccgctcccg atgcgccgcc tcgccgctgc tccgtcgctc ccagccgtgc acccacacga 120

ctcccatgcg ccaccctccg ttcggctccg gcgacaacat ggccgggtgt cgatggagag 180

gacctcgacg acgacgggcc gaggtgttag ttgtgtaatg gcaagcggaa aagagcatgc 240

aacaactact actgctgatg aaaagaaagg tggattgcgt ggcaaactaa aaaaagttgt 300

tctagcttac agcggtggct tggatacatc agtaattgtt ccatggctta gggagaacta 360

tggctgtgaa gtcgtctgtt tcactgctga tgttggccag ggtgacattg aattggaagg 420

attggagaaa aaggcaaaag ctagcggtgc aagccagctt gttgtgaagg acttgacaga 480

agaatttgtt ggtgaatata tatacccctg cttgcgtgct ggtgcagttt acgaaagaaa 540

gtatctgctt gggacttcca tggctaggcc cgttattgct aaggcaatgg ttgatgttgc 600

caaggaagtt ggcgcagatg cagttgctca tgggtgcact ggaaagggaa atgatcaggt 660

tcgctttgag cttacatttt atgccttaaa tccggaactt aaagtggtgg caccctggag 720

ggagtgggat atcacaggac gtgaagatgc aattgagtat gcaaagaaac acaatgtgcc 780

aattccggtt tcaaagaaat caatatacag ccgagataga aatttgtggc accttagcca 840

tgagggtgac attttggaag atccagcaaa tgagccaaaa gaagatatgt atatgatgtc 900

tgtctctcca gaaagtgcgc cttcaaaacc tgagtatctg gagattggta tagtcgctgg 960

tgttcctacc tctatcaacg gaaaagatct ctcgccagca actctccttg ccaagctcaa 1020

cgaaatcggt ggaaagcacg ggatcgggcg catcgacatg gtggagaacc gcctcgtggg 1080

gatgaagtcc cgtggcgtgt acgagacccc cggcggcacc gtcatggcgg ccgcggtccg 1140

cgagctcgag tccctgacac tggaccggga gacgatgcag tggaaggaca tgctcgccct 1200

caagtacgcg gagctcgtct acgcggggcg ctggttcgac ccgctccggc tgtccatgga 1260

cgccttcatg gagaagatca ccgcgacgac gaccggctcc gtgaccctga agctgtacaa 1320

gggctcggtg accgtcgcgt cccggaagag cccctacagc ctgtacaggg aggacatctc 1380

atcgttcgaa aacggggaga tatatgacca ggctgatgct gaaggtttca tcaggctgta 1440

cggcctcccc acccgggtcc gcgcgatgct ggagaagggt atctaaggcc gttttcgtgt 1500

ctcggaataa aatgttctgt tttcgtgtct cgaataaaat gttcgcattg ggaactgccg 1560

agtgtatttt ctagtgattt caaggtgggc ttagagcact ttgtcgagtt tttgttttgt 1620

ttcatctggt gtgcgtggat atgtgtatcg aagggttgct ccagcttgca gtactatacg 1680

cattgtggtg ctgcggtgca gtccttgtct cattgcactt caggcgtgtt tggttactgg 1740

cgtcattttt ggcttccggt agtctgtatg ggttaatacc aagtctgtgc caagttgtgt 1800

<210> 29
<211> 3336
<212> DNA
<213> Zea mays

<400> 29
tgatttttaa attgtctttg cctagtgttg gagaaaaaac actcggcaaa gagctctttg 60

ccgagtgttg tatttgtggc actcggcaaa gagccctttg ccgagtgtca aaaaaagaca 120

ctcggcaaag aaactctttg ccgagtgtca aaaataaaac actcggcaaa gatcttcttt 180

gccgagtgtt ttattttacc gagtgttttt tgcgtgacac tcggcaaaga gcttctttgc 240

cgagtgcccg aaaaaaaaca ctcggcaaag atccaaattc cggtagtgtg aggcggagtt 300

tgctccgagg ttgaggtcgg cttcgggcaa ggcgtagttt cctcccgagg gccggaccat 360

gctttatcat acgcgcatcc cattcggagt tgtttgaact ctacgggcgg catgggagag 420

cagtagccat tattcccggg catgcgtcat cgtagttggt gcaacagagc aaattcaact 480

ttataattca tttcaattga tttataataa aagaaaatac ttagaaccat aattcgtgat 540

tctaagtcac tcaaatctta caaattaaac taaatattga aaaaattgtt ggttcgaaaa 600

ctagggtggt acattctaag tatatatgca gtccatctat ggaatgaata ataatacaga 660

tagatacgag tacccatctg taccatggtt ttttttaagt ggcattccac gttctatgta 720

agttccgttt cttgatcact ggatcgtttt gaaaacctgg ctgctaaggg ttgctgtgct 780

gtgcaaactg atctgaccat tcagccccgc gcgggcacca actaactaac acacgtgcct 840

acctaacgca gtcctccgtg gaccatggag gaggtgtgga cagcgtcgtc gtcttcccgc 900

cgatccaccg cccggccgcc cgtgctataa agaggaaggc caaccacgga cacccatgcc 960

aagcagacca agatttattt gttcgtcgca ttcgattcat tagcagctcc ctgcttgctt 1020

tgcacgtggt gactggtgag ttcattcact ttgccaagtc atcacgtcag cagcagacca 1080

agaccaacag agacatgggt ggaggcagcc gcatgcagac cgtgctgttg gccccgggcg 1140

ttaaggacaa gaagctgctc tcctacaccc gccgcaacgc gctcaaggaa aaggacgccg 1200

tcaccgacct catccgctcc atcatcgcca cgtccagccc cgtccagcgg accgccttcc 1260

acgtcttccg cattgacaac gtcctcgagc tctactcggc ctggcgccgc ggcctccccg 1320

acgtgcgccc ctactacgcc gtcaagtgca acccggagcc ggcgctcctc ggcgccctcg 1380

cggagctcgg cgccgggttc gactgcgcca gccgcgccga gatcgaggcc gtgctcgcgc 1440

tcggcgtccc gcccagcagc atcatctacg ccaacccctg caagtccgag ccgcacctag 1500

agtacgccgc cgcggccggc gtcaacctca ccacctacga caccgaggag gaggtcgcca 1560

aggtcaagcg ctgccacccg aggtgcgagc tcctgctccg cctcaagggc cccgacggca 1620

gcgagaccac gtgcgaccta ggcaccaagt acggcgcgcg cgccgacgag gtggtgccgc 1680

tcctgcgcgc cgtccagcgc gcgggggtca ccgtggccgg cgtctccttc cacgtcggga 1740

gccccgtggc ccgcgtcggc gtgtaccgcg ccgccatcga ggccgcccgc gccgcgttcg 1800

acgaggccgc cgccctcggc atgcccccca tgcgcgtcct cgacatcggc ggcgggttca 1860

cggccggcgc cagcttcgac gacgccgcgg ccgtcatcaa cgacgccctc gcgcgctcct 1920

tcggcgacct gccgtgcgtc gaggtcatcg gcgagccggg ccgctacttc tgcgagaccg 1980

cgttcaccct cgccgcgcgc gtcatcggca agcgcacgcg cggcgaggtg cgcgagtact 2040

ggatcgacga cggcctctac ggctcgctca acaacatact catggaccac aaggtcccgc 2100

gcccgaggcc gctggccacc ccgcgccccg gcgacaggac gcacacgtcc acggttttcg 2160

gcccgacgtg cgactcccag gacgaggtgg tcaccggcta ccagctgccc gagatgagcg 2220

tcggcgactg gctcgtcttc gacaacatgg gcgcctactc cacaggttcc ggctccaagt 2280

tcaacggctt cgacacgtcg gagattaaga tctacgtcgc ctactccagc tgagctagca 2340

atgcatatat cgacggcgcc cgccatcata tgcatgcatt attacacgtc tatatacata 2400

tagcccctcg ttaaaaaaac ataactacgt accgacgagt agtacaccgt cgatcggtaa 2460

agatgtctta caaataatga gtcggtaaat ccacgtttac cagttattac ctgtcagttg 2520

atggtaaatc cacgtttacc ctacttctac cttccaaatt tctgtcaata tattcgagca 2580

ttgtttttcc ttaattcagg tttacttgtg tcatgtgatt ttatatttat acatgtacgt 2640

atgtatcaga taaaaggggt gatccacatt acatggaaat ccgtatttga atttcagatt 2700

aattaaataa atgaattggt tacactgttc caaagatgag ccctcgtcat gaactcatgt 2760

gattgtattg tatcatcagt acttacaggc attcttatga tccagtggag atcaataatt 2820

aattcaagaa cctgactttt cttttgttaa gagggctgtg gcytttttat tttcctggtg 2880

gtgtgctctt tagaattcag atttccgaga acttagttag cagcactgtc aaacagaacg 2940

gttttggaag tgtagcatgc atgctggttt ataaaaaaag gttttaggag gcatggattt 3000

tactaatagg gacagacatc ttcacctacc acctttatca atcatgttgg gacctaatag 3060

catatgcatg tctctaacaa tcgatttgta tacatcaaaa taagtcgttt acgtaagtgg 3120

tttatttttt ggaggtgtca taggttcgga atcaataggc aagccgcaag catcgtgttg 3180

ctcgttggag gtcagtgtgt ggacatctga tggctcgaac actcaaaaca cacaaacgac 3240

acaaccaatt tatcttgttt tggacttctc gggaacagta tggttgttcc tcatgttagg 3300

aatgtctggt cgaggttttc tacagtcagg ggcgac 3336

<210> 30
<211> 29
<212> DNA
<213> artificial

<220>
<223> primer

<400> 30
ggcctcgagg ctcacgggct acagtgaat 29

<210> 31
<211> 30
<212> DNA
<213> artificial

<220>
<223> primer

<400> 31
tgctcaccat gatggcaagg ttgtactggt 30

<210> 32
<211> 29
<212> DNA
<213> artificial

<220>
<223> primer

<400> 32
ttgccatcat ggtgagcaag ggcgaggag 29

<210> 33
<211> 34
<212> DNA
<213> Artificial

<220>
<223> primer

<400>  33
ggcctcgagc gccttaagat acattgatga gttt                                    34


<210>  34
<211>  20
<212>  DNA
<213>  Artificial

<220>
<223>  primer

<400>  34
aggtttcaac ctccgaggaa                                                    20


<210>  35
<211>  20
<212>  DNA
<213>  Artificial

<220>
<223>  primer

<400>  35
tatctggcat gcctgtcatc                                                    20


<210>  36
<211>  20
<212>  DNA
<213>  Artificial

<220>
<223>  primer

<400>  36
acagctacgc caaggacgac                                                    20


<210>  37
<211>  21
<212>  DNA
<213>  Artificial

<220>
<223>  primer

<400>  37
cgcgtcctaa tctaagggca g                                                  21


<210>  38
<211>  23
<212>  DNA
<213>  Artificial

<220>
<223>  primer

<400>  38

gagattccac agatgcaaac gag                                              23

<210>  39
<211>  21
<212>  DNA
<213>  Artificial

<220>
<223>  primer

<400>  39
ggaggccctt gtttctgaat g                                                21

<210>  40
<211>  20
<212>  DNA
<213>  Artificial

<220>
<223>  primer

<400>  40
ttggaaaatt gttggtgttg                                                  20

<210>  41
<211>  19
<212>  DNA
<213>  Artificial

<220>
<223>  primer

<400>  41
cccattcata tggagcatc                                                   19

<210>  42
<211>  20
<212>  DNA
<213>  Artificial

<220>
<223>  primer

<400>  42
atgaagccct gatggagatg                                                  20

<210>  43
<211>  20
<212>  DNA
<213>  Artificial

<220>
<223>  primer

<400>  43
atggcaccgt ctgttacctc                                                  20

<210> 44
<211> 20
<212> DNA
<213> Artificial

<220>
<223> primer

<400> 44
acgctgaagg tttcatcagg 20


<210> 45
<211> 20
<212> DNA
<213> Artificial

<220>
<223> primer

<400> 45
tagatgccct tctccagcat 20


<210> 46
<211> 20
<212> DNA
<213> Artificial

<220>
<223> primer

<400> 46
aggaggagga gctcgacatt 20


<210> 47
<211> 18
<212> DNA
<213> Artificial

<220>
<223> primer

<400> 47
gccgaacttg cccttctc 18


<210> 48
<211> 20
<212> DNA
<213> Artificial

<220>
<223> primer

<400> 48
gggaagagat ttggtgtgga 20

<210>  49
<211>  20
<212>  DNA
<213>  Artificial

<220>
<223>  primer

<400>  49
tcacaccttc cccaagtttc                                                    20


<210>  50
<211>  20
<212>  DNA
<213>  Artificial

<220>
<223>  primer

<400>  50
ggtacgcgag tactggatcg                                                    20


<210>  51
<211>  20
<212>  DNA
<213>  Artificial

<220>
<223>  primer

<400>  51
tcgacgtgta cgtcttctcg                                                    20


<210>  52
<211>  421
<212>  PRT
<213>  Triticum aestivum

<400>  52

Met Pro Ala Leu Ala Val Asp Ala Ala Ala Pro Val Ala His Ala Phe
1               5                   10                  15


Ala Ala Ser Cys Asp Ala Ser Pro Arg Phe Ala Ala Pro Leu Leu Gly
            20                  25                  30


Pro Ala Ala Ala Val Val Ala Ala Thr Ala Ala Glu Lys Pro Glu Met
        35                  40                  45


Ala Ala Trp Ser Ala Asp Leu Ser Ser Ala Leu Tyr Asn Val Asp Gly
    50                  55                  60


Trp Gly Ala Pro Tyr Phe Phe Val Asn Asp Asp Gly Asp Val Ala Val

65                  70                  75                  80

Arg Pro His Gly Ala Ala Thr Leu Pro Gly Gln Glu Ile Asp Leu Ala
                85                  90                  95

Lys Val Val Ala Arg Ala Ala Gly Ala Arg Ala Gly Gly Gly Leu Gly
            100                 105                 110

Leu Ala Leu Pro Leu Leu Val Arg Phe Pro Asp Val Leu Arg His Arg
        115                 120                 125

Val Glu Thr Leu Asn Ala Ala Phe Ala Tyr Ala Val Arg Ser Thr Gly
    130                 135                 140

Tyr Ala Gly Arg Tyr Gln Gly Val Tyr Pro Val Lys Cys Asn Gln Asp
145                 150                 155                 160

Arg Tyr Val Val Glu Asp Ile Val Glu Phe Gly Ala Pro Phe Gly Phe
                165                 170                 175

Gly Leu Glu Ala Gly Ser Lys Pro Glu Leu Leu Leu Thr Met Ser Cys
            180                 185                 190

Leu Val Ala Arg Gly Ser Pro Asp Ala Leu Leu Ile Cys Asn Gly Tyr
        195                 200                 205

Lys Asp Leu Glu Tyr Val Ser Leu Ala Leu Ile Ala Arg Thr Met Gly
    210                 215                 220

Leu Asn Thr Val Ile Val Leu Glu Gln Glu Glu Glu Leu Asp Ile Val
225                 230                 235                 240

Val Glu Ala Ser Arg Arg Leu Asn Ile Arg Pro Val Val Gly Met Arg
                245                 250                 255

Ala Lys Leu Arg Thr Lys His Ala Gly His Phe Gly Ala Thr Ser Gly
            260                 265                 270

Glu Lys Gly Lys Phe Gly Leu Asn Ala Ala Gln Ile Leu Ser Val Val
        275                 280                 285

Ala Lys Leu Lys Ala Leu Ala Met Leu Asp Cys Leu Gln Leu Leu His
    290                 295                 300

Phe His Ile Gly Ser Gln Ile Pro Thr Thr Ala Leu Leu Ala Asp Gly
305                 310                 315                 320

Val Gly Glu Ala Ala Gln Ile Tyr Cys Glu Leu Ala Arg Leu Gly Ala
                325                 330                 335

Ala Met Arg Val Ile Asp Val Gly Gly Gly Leu Gly Ile Asp Tyr Asp
            340                 345                 350

Gly Thr His Ser Ala Gln Thr Asp Met Ser Val Ala Tyr Ser Leu Glu
        355                 360                 365

Glu Tyr Ala Ala Ala Val Val Ala Ala Val Gly Arg Val Cys Asp Arg
    370                 375                 380

Lys Gly Arg Ala Ala Pro Cys His Leu Ala Ala Arg Ala Gly Arg Pro
385                 390                 395                 400

Leu Val Ser His His Ser Val Leu Ile Phe Arg Ala Phe Pro Glu Gly
                405                 410                 415

Gly Arg Val Pro Lys
            420

<210>  53
<211>  450
<212>  DNA
<213>  Artificial

<220>
<223>  fragment from pUC57 TaADC2

<400>  53
gacgcgtcgc cgcgcttcgc cgcgccgctg ctgggccccg ccgccgccgt cgtcgctgcc      60

acggccgccg agaagcccga gatggccgcg tggtcggccg acctctcgtc ggcgctctac     120

aacgtggacg gctggggcgc gccctacttc ttcgtcaacg acgacggcga cgtcgcggtg     180

cgcccgcacg gcgccgccac gctgcccggc caggagatcg acctcgccaa ggtcgtcgcc     240

cgggcggccg gcgcgcgcgc cggcggcggg ctcggcctcg cgctgccgct gctcgtgcgc     300

ttccccgacg tgctgcgcca ccgcgtcgag accctcaacg ccgccttcgc ctacgccgtg     360

cgctccacgg gctacgccgg gcggtaccag ggcgtctacc ccgtcaagtg caaccaggac     420

cggtacgtgg tcgaggacat cgtcgagttc                                      450

<210>  54
<211>  470
<212>  DNA
<213>  Artificial

<220>
<223>  fragment from pUC57 TaODC

<400>  54
tatatctaga gggcgccatc gaggccgcgc gcggggtctt cgacgcggca gccgggctcg      60

gcatgccgcc catgcgcgtg ctcgatatcg gcggcgggtt catggccggc ccggcgttcg     120

acgaggcggc cgcggtcatc aacgcggcgc tcgagcgcta cttcggggag cttccgtgcg     180

tggaggtgat aggcgagccg gggaggtact tcgccgagac cgccttcacg atggcggcgc     240

gggtcatcgg gaagcgcact cgcggtgagg tacgcgagta ctggatcgac gacggcctct     300

acggcaccct cagctgcatc cccatggacc actacgtgcc gcacccgagg ccgctcgccg     360

tgccgcgcgc cggcgagaag acgtacacgt cgacggtgtt cgggccgacg tgcgactccc     420

tcgacacggt ggtgaccggg taccagctgc cagagatgag gagctctata                470


<210>  55
<211>  1624
<212>  DNA
<213>  Zea mays

<400>  55
atcctatttc tgtagcagtg ctatatagaa cgacatacat tgaaagaaca ccttattttt      60

atatcgaaag atagataata cagggttggg tcgattatag aataaataaa aaaagaacat     120

atcaattagt ccaggcccga cgtcgtggag acgttcgcat tcagctggag taggctacat     180

agatctttat atctgacgtc gcgtagccgt tgaagttgga gccggctccg gtagtgtagg     240

cgcccatgtc gtcgaagacg agccagtcgc cgacgctcat ctctggcaga aggtacccgg     300

tgagcaccgt gtcgagggag tcgcacgttg ggccgaagac cgtggaagtg taggttttat     360

cgccggggcg cgggctggcg agcggcctcg gggtgggcac gtagtggtcc atgatgatgc     420

agttgagcga gccgtagagg ccgtcgtcga tccagtactc gcgcacctct ccgcgcgtgc     480

gcttcccgat gacgcgcgcg gcgagcgtga acgcggtctc ggcgaagtag cggccgggct     540

cggcgatcac ctcgacgcac ggcaggtcgc cgaagtggtg cgcgagggcg tcgttgatga     600

cggccgcggc gtcgtcgaaa gtggggccgg acacgaagcc gccgccgatg tcgaggacgc     660

gcatgggcgg catccccagg gcagcggccg cgtcgaacgc ggcgcgcgcg gcctggatgg     720

ccccgcggta gacgtggagg ttggacgcgc cgctgccgac gtggaaggag acgccggcca     780

cgccgacccc cgcgcgctgg gcggcgcgca ggagcggcac gacctcgtcg gggtgcgcgc     840

cgtacttggt ccccaggtcg acccggacgt cgccgccgtc ggggcccttg aggcggagca     900

ggagctcgca gccggggtgg cagcgcctga ccttggccac ctcctcctcg gagtcgtagg     960

tggtgaggtt gacgccgacc tccgcggcgt actcgaggtg cgcctcgggc ttgcaggggt    1020

tggcgtagac gatgtccccg ggccggacgc cgagcgcgag cacggcctcg atctcggcgc 1080

ggctggcgca gtcgaagccc gcgccgagcg cggcgagcgc gccgagcagc gccgggtccg 1140

ggttgcactt gacggcgtag cagggccgcg cgtccggcag cgcccggcgc cacgccctga 1200

agaggtcgac gaccttggcc aggtcgaaga cgtagaaggc gctgcgctgc gccgggctgg 1260

acgcgaggat ggagtggatg aggccggaga cggcgtcctt gtccttgagc gcgtcgcgct 1320

tgaacgcgag caccttcctg tccttgacgc cgggggccac cagcacggcc tgcagggggc 1380

tgcttccaac catggtgatg acgagacgat tgacgacgcg cgcgatagat gagcagcggg 1440

atcgaacttg ttcgatggag aactcgtgtt gactgaaaac caagatatac aagggaaaaa 1500

aggagcacgt tattattaat aaacgatgac gatgtaaatg ttgcagcttc cttcgtgtct 1560

ggtagtgtca ttatgcaggg tcgatcactc gcgtctggta gatagataga tatagataga 1620

taga 1624

<210> 56
<211> 1062
<212> DNA
<213> Zea mays

<400> 56
cgattctgac cggtcagccg caccccggca ccacaccgaa ccacatcgcc ctcccgcggt 60

gcctctctcc ggcttccggc ccacctaacc cattccagat tccaccgatg aaacgtgagc 120

acctccccca tggccagccg cgggcactcc tatatatggc gccagactca tgccgagcaa 180

ccccgcacac acaccaagag acgcgtctca ccagacgacc ttctcgcctc agggcctgct 240

tggcatcact tgccaggtcc gttggatcgg tcatcgtctc gtcaacacga gttctccatc 300

gaacaagttc gatcccgctg ctcatctatc gcgcgcgtcg tcaatcgtct cgtcatcacc 360

atggttggaa gcagccccct gcaggccgtg ctggtggccc ccggcgtcaa ggacaggaag 420

gtgctcgcgt tcaagcgcga cgcgctcaag gacaaggacg ccgtctccgg cctcatccac 480

tccatcctcg cgtccagccc ggcgcagcgc agcgccttct acgtcttcga cctggccaag 540

gtcgtcgacc tcttcagggc gtggcgccgg gcgctgccgg acgcgcggcc ctgctacgcc 600

gtcaagtgca acccggaccc ggcgctgctc ggcgcgctcg ccgcgctcgg cgcgggcttc 660

gactgcgcca gccgcgccga gatcgaggcc gtgctcgcgc tcggcgtccg gcccggggac 720

atcgtctacg ccaacccctg caagcccgag gcgcacctcg agtacgccgc ggaggtcggc 780

gtcaacctca ccacctacga ctccgaggag gaggtggcca aggtcaggcg ctgccacccc 840

ggctgcgagc tcctgctccg cctcaagggc cccgacggcg gcgacgtccg ggtcgacctg 900

gggaccaagt acggcgcgca ccccgacgag gtcgtgccgc tcctgcgcgc cgcccagcgc 960

gcgggggtcg gcgtggccgg cgtctccttc cacgtcggca gcggcgcgtc caacctccac 1020

gtctaccgcg gggccatcca ggccgcgcgc gccgcgttcg ac 1062

<210> 57
<211> 1018
<212> DNA
<213> Zea mays

<400> 57
ttgaacccga cggccattct tcagtccaca atcggcggag ctcctccttc taccgccgca 60

gctgcggcgg agaacggtga caccaccaga aaagttgttc ctctatcaaa agatgccctt 120

caggatttca tggtttccat cataacccag aagttacaag gtgaaaagaa gccgttttat 180

gtgttagatt tgggagaagt tgtttccctg atggatcaat ggaacgttgc gttgccgaat 240

gttcgtccgt tttatgctgt gaagtgtaat cctgaacctt cattcctttc tatgttggct 300

gctatgggtt cgaattttga ttgtgctagc cgtgctgaaa ttgagtacgt tttgtctctt 360

ggtatctcgc cggagaggat tgtattcgct aacccatgta agccggaatc cgatatcatt 420

ttcgccgaaa aagttggagt caatctcaca acttacgatt cagaggacga ggtttacaag 480

atcaaaaagc atcacccaaa atgcgagctt ttgctccgta tcaaaccgat gaacgatggt 540

aatgctagat gtccaatggg tccaaaatac ggcgctttgc ctgaagaaat cgagccgctt 600

ctccgtatag ctcaagcttc ccggctcact gtctccggcg tctccttcca taacggcagc 660

ggcgacgctg attccaacgc ttatttaggc gccatagccg cagccaagca agttttcgaa 720

acggcagcca aattcggcat gtcgaaaata aatgttctag acatcggcgg cgggtttacc 780

tccggccacc agttcaccac cgcagcaacc gccgtgaaat cagctttaca acaacacttc 840

tctaacgaac cggagctcac catcatagct gaaccgggtc ggtttttcgc agaaacggcg 900

ttcacattgg caacaaccat catcggaaaa agagtacgtg gagatttacg tgaatactgg 960

atcaacgacg ggttatacgg gtcgatgaac tgtgtacttt acgacaatgc gactagtg 1018

<210> 58
<211> 4650
<212> DNA
<213> Zea mays

<400> 58
   gcttattcgt gccgggccgg cccgggccag catataaccg ggtcgtgccg ggctcggacc 60
   gggcccaaaa aacgggcttc gtgccgtgct cgcgggcttt gtgcttattg gacatctata 120
   gaggcacttg tcacatcatg gcagagtcag agacgccagt gtcacgcaca attacataag 180
   agatctacaa gaatataacg acggtaattc acggcatagc atgatccctc caagacgatg 240
   cgtggaggat tgggggaagg ggcttcctga cgacgcctcc aaggaggcta gtggcgccga 300
   gggcgtcgtc gcctacacag acttcgtatg gcatggcttt cgcctgggac cccaaaaccc 360
   ctgctcaagc taccggttat accacaacga acacaacaac atacaacagg agtgggggtt 420
   ataggagtgg ctccgaactt ctaacgtcga tgatggagac acaggttgtg cttcacctag 480
   gggctcgagg ccagctatgc aaccaatggt ggtggcgctg ccttcaaagg gaggggtgcg 540
   agggcccaag gtgagcctga tccaatgaac ccgatgaccg ccgcacgcta ctgccaaggt 600
   caagccgcgc accgcgggcg cactacgaga cgcagacgtt gtcccttagt ggttagctgc 660
   ggccaccatt gggagccagc gtcggaaacc gcccaagcgt gaaaggggcg ccaaatccga 720
   tcgtgcctag ggtagtggag ccaaatcccc tcgcggcaca agtacagcgc gttgaggtgg 780
   gctcaaaggg aaggcacaaa gaagagtgtg gtgatacctc tacatctaag gccatcgaat 840
   gaggaaagtc aatatgagcc cgggtagcca catccggtcg cactgtcact ggtcgactac 900
   cggcggacgc tccggcgaaa gaacacacgt ccggtgggag agagatgagg gaggaagaag 960
   aaagcaatcg agagagagag ggaggaggaa gaagaagaac ctagctgtcg ccttctttgc 1020
   cctccacctg accaccgatg tcgtgcttcg gtgacggctg ggcagcgaat gcgtgaggga 1080
   aggggctggg ggcggcgacg gtggagtgcc gctcgaggcg ctagcttaat ggtgagtccc 1140
   ttgtccttgc ccatccctag caatcatgcc ggcgacagcc tctaggggct tatgatatcg 1200
   gcaaggctcc accgctggta agacccgagt acgaacaaac agagaagaag agaggataat 1260
   gattcatttt gagatcttga ttgatttgtt gggagggtat ccacaagaat aacataagac 1320
   aaaactaggt aagtcacttt tttgatttca ccattccaat ataaagggga aaagtgtttt 1380
   tttagcaaaa ctcatttccc aaactactgt ttgatacgac ttaaggaaaa aacgaaacca 1440
   aagtaaaagc cgatgctaaa aagccatacc aaaggacccc taactcctgt cggtaaactt 1500
   ggcagccaac gaaagttagt taattaccgt tgacaaagct aggttagctg acgaaagtta 1560
   atggaatttt tatgggcttg gagttaatta attcttgttg gcttgatcaa agtccgatga 1620
   gaattatcga agccaacgga aatggccccg ggaattatcc aaacctatgg gaatgtctcc 1680
   gattccttta gtgtttagaa ataaatattc ttaaaaaaac atgctgaaat atataatctg 1740
   gattaaattg ttagaaaatg gtagaaacag acatgcacat atagcaatta aacacaccag 1800
   tgaagcttgt tttatttatt ggataacacg tacaataaca agatgcaagc gagcagggta 1860
   aactcaaaaa agggaaacat acatacgtag gctgctttcg ttatctgctt atccatcatc 1920
   aaccaggcag ttcttattgt atatatgatc gcagtcgcag ccaaatccgg agactctgca 1980
   gtgcagaagt tcaagggatg ggttcaggtt caggttcagg ttcaggacct ttgtattcgt 2040
   tgaggtaggg catggtggtg agcgctttgg tcatgagtgg ctccaccatc tcccacgccc 2100
   acacacgttc ttcttcttct gtccccttgg ctttcttctc gatgacggtg ataatgtctt 2160
   cctcgacgct gtacctcatg ctgccgatga tctcatccgc agctgggccc aggtcaacgc 2220
   gttcaatctc gaagccgcca tcttttgtag tgctactatt gacacgaacg agggttgggc 2280
   cactgaacag gttgtgcttg ccagcaatgg cctcctggta cgccccggag acgagtacgg 2340
   ccacgtagta gccaccgagg ctcggctcga acaggtgcag cggcaatgtg ttttcgcggc 2400
   tgatgaagtt ctcgatcttc ccgtcactgt cgcaggtgat gtccaccagc gtgcccttga 2460
   gagtcggctc ctcgttgagg cggctcaccg gcatcatcgg gaagagctgc cctatgcccc 2520
   agaagtcagg caacagcgag aagacggaga ggttcatgtg gtagttatag acggtgttgg 2580
   catcggcgac gaccctcttg ccgagcttct tgccaagctt gtacatctcg atgccgtgct 2640
   tcttgaggtc tgcggcgtga gagtaaacgg ccgccgtggc gccgccaacc ggagctctcg 2700
   gctgcttgta agataggtcc tggatcctgt tcaacagctg ctcggaggtg tcctcgtcct 2760
   gcggctccgg gatcgccgag agcgtctcga ggatgatcat ggagtggtgc gacgccatgg 2820
   cgcgcccgct ctcggtgcac acgaccgggt gcgcgacgcc gttgtagtcg cacttgagcc 2880
   gcaccgcctg cacgacggcg gaggcgtact cctgtatccc gtacgccacc gacatgtcgg 2940
   agtcgcctga acgggtcccg tcgtagtcga ccccgagccc gccgccgcag tccagcgtgg 3000
   ccaaccactt ggcgccgtgc tcgttcacca ggttgcagta gatgccagcg gcctcgctca 3060
   ccgcgtcgta cactttcccc gtcgtcggga tcatggagcc gatgtggaag tgcagcagct 3120
   ggagccagtg caacttgccc tccttgccga gcgtctcggc cacctcgtag atctggtcgg 3180
   cgagcatccc gaacttgccg tgcttgccgg ccgtcgaacc gaagtggcct ggtagcttgg 3240
   tgagaagctt ggcgcggatg ccgatgatcg gctcgaactt cttgcgcacg gcatcgtcgt 3300
   ctggcagctt ctgctgcagc ttcctgtact ggtcgaggac gatggcaagc tcctcctcca 3360
   tctcgagcac gatgatgggg ttcaggccca tggcccgcgc cgagagcgcg agcgcgatgt 3420
   agtccgcgtc cttgtagccg ttgcagatca agtacggctt gggctcggct tcggtgacgg 3480
   ggaggcagct catggcgatg agcagttctg gcttggagcc agcctcgagc ccatagttcc 3540
   attgctggcc gaacctaacc atgtcctgca cgacgtgctt gttctggttc accttgatcg 3600
   ggaacacgcc ttggtacctg gacgtgtacc cggtggactc catggcgctg ttgaatgcgt 3660
   tatggagcgt ctcgatacgg ttcttgagca cgtcggggaa gcgaaggatc atggggaact 3720
   gagatttgct ggtgctgtcg ttgggttggg tcgctagagc ttccacgatg accgagtgca 3780
   cgtcgatctc ctgcccgggc tccgtccagc ggccgaaagg cttcacgctg aggtgcccct 3840
   ccgtgttcac cgcgaagtac tcctcacccc atccgaggat gttgtagagt tcaccgtagt 3900
   tcttatccat gatgggaaaa agtgctctcc tatatccaaa caatataatg cgatataata 3960
   tggggaaaag tgtctacagt gaaaaacgtt ttttttaaaa aaaatgtccg accggatcca 4020
   taaatatgca tgcatgagat gttgtctgct agttttaagc tagttacgat gttcaatata 4080
   taatgaagct tgttaaacta tatagtgaat gtcgtagtaa tggtatgtta cgtactttca 4140
   ttttttgaga agtgaatggc ctcacaaaag ctgctctttt tgaagcaata ttagtggtca 4200
   tcttagattt taatcctaaa tatgaatagt tatctatgta gtgtttaata tgcacatcat 4260
   actacattgt caagcagtaa taatgttatt tacttatgta gagatcacta ttggtgcgat 4320
   ggcagcttga tcgatatgaa gtagggtctg tgcctatcta atgacaagct tcttaaatac 4380
   tctctccgtc caaaaatatt aatcatttta actcttgatt tttatgttta tatttaaata 4440
   aataatgata aatctagaca catatataaa aaaataaatt aattattgta tgaatcaact 4500
   aaaatggata aaacaaattt tattttggga ctagtatgtc tcaacatttt aacgtgtggt 4560
   gtcatagcca tattccatat tgcacacaca attaaacttt gcaaagcttc accatgcatg 4620
   tgttcataat atcggtaaga attatgtgaa 4650

<210> 59
<211> 2968
<212> DNA
<213> Zea mays

<400> 59
ccaagcacgc cggccacttc ggctccacgt ccggggagaa gggcaagttc ggcctcagcg 60

ccgcgcagat actgtccgtg gtcaccaagc tcaaggccat cggtatgctc gactgcctcc 120

agctgctgca cttccacatc ggctcccaga tcccgaccac cgccctgctc tccgacggcg 180

tcggcgaggc cgcgcagatc tactgcgagc tcgcccgcct cggcgcggac atgcgcgtca 240

tcgacgtcgg cggcggcctc ggcattgact acgacgggac ccactcggcg cagaccgaca 300

tgtcggtggc gtacagcctg gaggagtacg cgggcgccgt ggtggccgcc gtgggccgcg 360

tgtgtgaccg caagggggtg cagcacccca tcatctgcag cgagagcggc cgcgcgctgg 420

tgtcgcacca ctcggtcctg gtgttcgagg ccttctcggc tacggcgccg ggacgcctgg 480

acgcggccac ggcctacctc ctcgacgagc tcacggacga ctgccgcgcc gactaccgca 540

acgtgatggc cgccgccgtg cgcggcgact acgacacgtg cggcctgtac gccgaccagc 600

tgaagcggcg gtccgcggag cagttcaagg agggggtcct tggcctggag cacctcgccg 660

cggtggacgc cttctgcgag ctcgtcgccc gcggcatggg cgcgcccgag ccgccgcgca 720

cctaccacat caacctgtcc gtgttcacct ccctgccgga catgtgggcc atcgggcagc 780

agttccccat catcccgatc cagcggctgc aggagcggcc cgccgtcgac ggcgtgctct 840

cggacctcac ctgcgacagc gacggcaagg tgagcgagtt catcggcggg aggcacagcc 900

tgccgctgca cgagctgccc acccacgcca ctcgcggcta ctacctgggc atgttcctcg 960
ggggcgccta ccaggaggcc ctcggcgggc tgcacaacct tttcggcggg cccagcgtgg 1020
tgcgcgtgtc ccagtgcgac gggccccact gctttgccgt gacgcgcgcc gccgcgggcc 1080
cgtcctgcgc cgacgtgctc cgcgcgatgc agcacgagcc cgaggtgatg tacgaggtgc 1140
tgaagcagag gaccgacggc gccacggccg ccgccctcgc ccgggccttc ggcgcaatgc 1200
cgtacctggt gttcgacccc gaggccgccg tgatgtccgg cggcgagagc agcgactcgg 1260
agcggtcggc cgccggcgcc gccgaggagg aagacgatga ggagtgggag ttcatgcgcg 1320
ggctcaccgt ctgatgacgt gcccgcccgc ccgccccggg tgaaacaagg cgagtccgtc 1380
gccaggaagt tgtcagtggc cggccggcgt tggtttacat aataaccccc accgcccggt 1440
gttgtatcgt gtcgtggtcg tagtcgtcgg gtgtctcgtc gtcgccggag ttgtttggcg 1500
ccaccttttt attccgaacg cgtcagtgtc cactgttcgc ctttcctttt ttttctctct 1560
ctttcatggg aataatctga aggaaactaa tagcaagttt ctctttaact ggaaatttcg 1620
ttttttcaag aattcattct gtctgttgct gtgtcggttc cgctctcccg ttgcgtggtg 1680
cgctgccacc ggagaacacc ttcctctcgt ctcgacgtcg tcgctttctc ttttcttctc 1740
catcgtcgcg tcgtgggcag ggggcaggtg tcacggcccc gagcgccgga atctctctgg 1800
ataaaccccg agatattcgg acgtcacgcg ggtgggccgc ggcggatttg aaacgcagct 1860
ctgggacgct acctgacgcg cgtgtcagcg tcatcggagc ggtaggtgtg gggacgctgg 1920
cgggctgggc tttgcttcct tccttgctgt gcgtgctcga ctgcgtctgc ctttgccgca 1980
tgtgttgcga gatgacgagg acgaaaaggt cttccagaat ttatgagcag tttttggatt 2040
tggtacagat gctgtgagct cgagctggag caccgagacc ggggctgcct gcgacgcggt 2100
aggtgagcgc cgcccgggag tcggttatac tacgactttc gccgcataca ttcagccgcc 2160
ggccgccgca tacggcatgg attagattat tagataaaga tcggatgagt aataagcagt 2220
gcaaagttca tgaacctgaa atatgcatgc atacacctgc tggcgatctc ggccgttgac 2280
ggagcatgca tgtggtgtgt ccccgttccg gatggctcgc acagatctac tgtctactac 2340
tagctcccag agcagagcag gcagagatgg ttggcggttg gcacaggtag agaggcagcc 2400
acgcagtgca gagccgtggt agcgatgcgc gaggcggatg gacaggatag ggaggggata 2460
gcgtaggggc caagtgacca ctcgtcggct ctggcctttc ggcgcccatc tcctcctcat 2520
ctcatctccc tccacggtcc acttgtttcg ttcgctgtgt gacagtagtg tgagagattc 2580
cagatccaac acctgccgac cccgtcgtga tcctgtccag cggctccaga gacagagagg 2640
cacgaacaca tgccaagctc ccagctcctc cctccccccc tcacctcacc tcaggccctc 2700

actcccccag gtactcaatg ctctttctct ctttcagccg ccgctgctgc gttgcgtcgt 2760

gtgggagctg agctagtggc actgggtgct ctcgaccgca ctaggcagac cagacgttgc 2820

gtcgtgcccc atattcgccg tcgccgtgcc tttcagcatc tatctgggag tgaatgactt 2880

gggctgggtg acggatagct gaccatctca gctctctcaa agaacaagat cgtaggagaa 2940

cctcctagtc ctttgcccct gtcgacaa 2968

<210> 60
<211> 2968
<212> DNA
<213> Zea mays

<400> 60
ccaagcacgc cggccacttc ggctccacgt ccggggagaa gggcaagttc ggcctcagcg 60

ccgcgcagat actgtccgtg gtcaccaagc tcaaggccat cggtatgctc gactgcctcc 120

agctgctgca cttccacatc ggctcccaga tcccgaccac cgccctgctc tccgacggcg 180

tcggcgaggc cgcgcagatc tactgcgagc tcgcccgcct cggcgcggac atgcgcgtca 240

tcgacgtcgg cggcggcctc ggcattgact acgacgggac ccactcggcg cagaccgaca 300

tgtcggtggc gtacagcctg gaggagtacg cgggcgccgt ggtggccgcc gtgggccgcg 360

tgtgtgaccg caagggggtg cagcacccca tcatctgcag cgagagcggc cgcgcgctgg 420

tgtcgcacca ctcggtcctg gtgttcgagg ccttctcggc tacggcgccg ggacgcctgg 480

acgcggccac ggcctacctc ctcgacgagc tcacggacga ctgccgcgcc gactaccgca 540

acgtgatggc cgccgccgtg cgcggcgact acgacacgtg cggcctgtac gccgaccagc 600

tgaagcggcg gtccgcggag cagttcaagg agggggtcct tggcctggag cacctcgccg 660

cggtggacgc cttctgcgag ctcgtcgccc gcggcatggg cgcgcccgag ccgccgcgca 720

cctaccacat caacctgtcc gtgttcacct ccctgccgga catgtgggcc atcgggcagc 780

agttccccat catcccgatc cagcggctgc aggagcggcc cgccgtcgac ggcgtgctct 840

cggacctcac ctgcgacagc gacggcaagg tgagcgagtt catcggcggg aggcacagcc 900

tgccgctgca cgagctgccc acccacgcca ctcgcggcta ctacctgggc atgttcctcg 960

gggcgcccta ccaggaggcc ctcggcgggc tgcacaacct tttcggcggg cccagcgtgg 1020

tgcgcgtgtc ccagtgcgac gggccccact gctttgccgt gacgcgcgcc gccgcgggcc 1080

cgtcctgcgc cgacgtgctc cgcgcgatgc agcacgagcc cgaggtgatg tacgaggtgc 1140

tgaagcagag gaccgacggc gccacggccg ccgccctcgc ccgggccttc ggcgcaatgc 1200

cgtacctggt gttcgacccc gaggccgccg tgatgtccgg cggcgagagc agcgactcgg 1260

agcggtcggc cgccggcgcc gccgaggagg aagacgatga ggagtgggag ttcatgcgcg 1320

ggctcaccgt ctgatgacgt gcccgcccgc ccgccccggg tgaaacaagg cgagtccgtc 1380

gccaggaagt tgtcagtggc cggccggcgt tggtttacat aataaccccc accgcccggt 1440

gttgtatcgt gtcgtggtcg tagtcgtcgg gtgtctcgtc gtcgccggag ttgtttggcg 1500

ccaccttttt attccgaacg cgtcagtgtc cactgttcgc ctttcctttt ttttctctct 1560

ctttcatggg aataatctga aggaaactaa tagcaagttt ctctttaact ggaaatttcg 1620

ttttttcaag aattcattct gtctgttgct gtgtcggttc cgctctcccg ttgcgtggtg 1680

cgctgccacc ggagaacacc ttcctctcgt ctcgacgtcg tcgctttctc ttttcttctc 1740

catcgtcgcg tcgtgggcag ggggcaggtg tcacggcccc gagcgccgga atctctctgg 1800

ataaaccccg agatattcgg acgtcacgcg ggtgggccgc ggcggatttg aaacgcagct 1860

ctgggacgct acctgacgcg cgtgtcagcg tcatcggagc ggtaggtgtg gggacgctgg 1920

cgggctgggc tttgcttcct tccttgctgt gcgtgctcga ctgcgtctgc ctttgccgca 1980

tgtgttgcga gatgacgagg acgaaaaggt cttccagaat ttatgagcag tttttggatt 2040

tggtacagat gctgtgagct cgagctggag caccgagacc ggggctgcct gcgacgcggt 2100

aggtgagcgc cgcccgggag tcggttatac tacgactttc gccgcataca ttcagccgcc 2160

ggccgccgca tacggcatgg attagattat tagataaaga tcggatgagt aataagcagt 2220

gcaaagttca tgaacctgaa atatgcatgc atacacctgc tggcgatctc ggccgttgac 2280

ggagcatgca tgtggtgtgt ccccgttccg gatggctcgc acagatctac tgtctactac 2340

tagctcccag agcagagcag gcagagatgg ttggcggttg gcacaggtag agaggcagcc 2400

acgcagtgca gagccgtggt agcgatgcgc gaggcggatg gacaggatag ggaggggata 2460

gcgtaggggc caagtgacca ctcgtcggct ctggcctttc ggcgcccatc tcctcctcat 2520

ctcatctccc tccacggtcc acttgtttcg ttcgctgtgt gacagtagtg tgagagattc 2580

cagatccaac acctgccgac cccgtcgtga tcctgtccag cggctccaga gacagagagg 2640

cacgaacaca tgccaagctc ccagctcctc ccctcccccc tcacctcacc tcaggccctc 2700

actcccccag gtactcaatg ctctttctct ctttcagccg ccgctgctgc gttgcgtcgt 2760

gtgggagctg agctagtggc actgggtgct ctcgaccgca ctaggcagac cagacgttgc 2820

gtcgtgcccc atattcgccg tcgccgtgcc tttcagcatc tatctgggag tgaatgactt 2880

gggctgggtg acggatagct gaccatctca gctctctcaa agaacaagat cgtaggagaa 2940

cctcctagtc ctttgcccct gtcgacaa 2968

<210> 61
<211> 924
<212> DNA
<213> Zea mays

<400>  61
cacagcacta aagtctattc atctctggct ccccaaattc agctggagag cactttttcc      60

catcatggat aagaactacg gtgaactcta caacatcctc ggatggggtg aggagtactt     120

cgcggtgaac acggaggggc acctcagcgt gaagcctttc ggccgctgga cggagcccgg     180

gcaggagatc gacgtgcact cggtcatcgt ggaagctcta gcgacccaac ccaacgacag     240

caccagcaaa tctcagttcc ccatgatcct tcgcttcccc gacgtgctca agaaccgtat     300

cgagacgctc cataacgcat tcaacagcgc catggagtcc accgggtaca cgtccaggta     360

ccaaggcgtg ttcccgatca aggtgaacca gaacaagcac gtcgtgcagg acatggttag     420

gttcggccag caatggaact atgggctcga ggctggctcc aagccagaac tgctcatcgc     480

catgagctgc ctccccgtca ccgaagccga gcccaagccg tacttgatct gcaacggcta     540

caaggacgcg gactacatcg cgctcgcgct ctcggcgcgg gccatgggcc tgaaccccat     600

catcgtgctc gagatggagg aggagcttgc catcgtcctc gaccagtaca ggaagctgca     660

gcagaagctg ccagacgacg atgccgtgcg caagaagttc gagccgatca tcggcatccg     720

cgccaagctt ctcaccaagc taccaggcca cttcggttcg acggccggca agcacggcaa     780

gttcgggatg ctcgccgacc agatctacga ggtggccgag acgctcggca aggagggcaa     840

gttgcactgg ctccagctgc tgcacttcca catcggctcc atgatcccga cgacggggaa     900

agtgtacgac gccgtgagcg aggc                                            924

<210>  62
<211>  1858
<212>  DNA
<213>  Zea mays

<400>  62
cccgggatcg acgcgcagtc gcccattccg catctcgccc tcccgtcccg tccgtccgtt      60

ctccgattgg ccgccgcccg cctcgccctc ctcgtcaaca aaagcccacc gcaagcgagc     120

gagcgagcca ccccgcccga tagccgccgc cgccgtcccg cccccacccg actcctcctg     180

accgccggcg tggggtgccc gggctccacc tccggcgacg acaccctccc cgccgcgcgc     240

gcccccaccg cctgctccgt ttgcgggggt agccggggct ccggcctcgg cggctttcaa     300

agccccctcc ctcacaacag gatccaaaat cccgtcctag acttcttcta cctccccttc     360

cagaacttcc tcttttggca tcccggacgg tcgatctcga cccccttcga tttcgattca     420

tctcttctta tcgattgatc tttcttctag cttatttcgt atactgtcat tttgattcct     480

tccttcttcc cgccgccggt agctaatccc aaatcccaat cgaatcgact cccccctgtt     540

cgtcggattc tcttcctccg tccgcaccca gctaacgcca ccgaaaacag aaaatttacc     600

cgccgcccaa aaaaaaacag aaaggctaac ctcctttttt ctctctctat ctttctctcc 660

atctgtggac ttctccggtt ttgtttgacg agatgcctgc gctcgccgtc gacgccgcga 720

cacccgtggc gcacgccttc gcgtcctgcg acgcggcgcg cttccccgcc ccgcccgtgg 780

tgggccccgc cgccgcggcc gaggccgccc cctggtcggc cgacctctcc gcggcgctct 840

acaacgtgga cggctggggc gcgccctact tcttcgtcaa cgacgacggc gacgtggccg 900

tgcgcccgca cggcgccgcg actctcccgg gccaggagat cgacctggcc aaggtcgtgg 960

ccaaggccgc gggcccgcgc gccggaggcg ggctcggcct gccgctgccg ctgctcgtgc 1020

gcttccccga cgtcctgcgc caccgcgtcg agacgctcaa cgccgccttc ggctacgccg 1080

tccgctccac cggctacggc tcaaggtacc agggcgtgta cccggtcaag tgcaaccagg 1140

acaggtacgt cgtcgaggac attgtggagt tcggcgcgcc ctttgggttc ggcctcgagg 1200

ccggctccaa gcccgagctg ctgctcgcca tgagctgcct cgccgcgcgc ggcggcctgg 1260

acgccctgct catctgcaac gggtacaagg acgacggcta cgtctcgctc gcgctcatgg 1320

cgcgctccat gggcctcaac accgtcatcg tgctcgagca ggaggaggag ctcgacattg 1380

tcgtcgaggc cagccgccgc ctcggcgtgc gccccgtcgt cggcatgcgc gccaagctgc 1440

gcaccaagca cgccggccac ttcggctcca cgtccgggga gaagggcaag ttcggcctca 1500

gcgccgcgca gatactgtcc gtggtcacca agctcaaggc catcggtatg ctcgactgcc 1560

tccagctgct gcacttccac atcggctccc agatcccgac caccgccctg ctctccgacg 1620

gcgtcggcga ggccgcgcag atctactgcg agctcgcccg cctcggcgcg gacatgcgcg 1680

tcatcgacgt cggcggcggc ctcggcattg actacgacgg gacccactcg gcgcagaccg 1740

acatgtcggt ggcgtacagc ctggaggagt acgcgggcgc cgtggtggcc gccgtgggcc 1800

gcgtgtgtga ccgcaagggg gtgcagcacc ccatcatctg cagcgagagc gaacacta 1858

<210> 63
<211> 874
<212> DNA
<213> Zea mays

<400> 63
gccgcgtcga ccaagaagca cggcatcgag atgtacaagc ttggcaagaa gctcggcaag 60

agggtcgtcg ccgatgccaa caccgtctat aactaccaca tgaacctctc cgtcttctcg 120

ctggtgcctg acttctgggg catcgggcag ctcttcccga tgatgccggt gagccgcctc 180

aacgaggagc cgactctcaa gggcacgctg gtggacatca cctgcgacag tgacgggaag 240

atcgagaact tcatcagccg cgaaaacaca ttgccgctgc acctgttcga gccgagcctc 300

ggtggctact acgtggccgt actcgtctcc ggggcgtacc aggaggccat tgctggcaag 360

cacaacctgt tcagtggccc aaccctcgtt cgtgtcaata gtagcactac aaaagatggc 420

ggcttcgaga ttgaacgcgt tgacctgggc ccagctgcgg atgagatcat cggcagcatg 480

aggtacagcg tcgaggaaga cattatcacc gtcatcgaga agaaagccaa ggggacagaa 540

gaagaagaac gtgtgtgggc gtgggagatg gtggagccac tcatgaccaa agcgctcacc 600

accatgccct acctcaacga atacaaggtc cctgaacctg aacctgaacc tgaacccatc 660

ccttgaactt ctgcactgca gagtctccgg atttggctgc gactgcgatc atatatacaa 720

taagaactgc ctggttgatg atggataagc agataacgaa agcagcctac gtatgtatgt 780

ttcccttttt tgagtttacc ctgctcgctt gcatcttgtt attgtacgtg ttatccaata 840

aataaaacaa gcttcactgg tgtgtttaat tgct 874

<210> 64
<211> 1346
<212> DNA
<213> Zea mays

<400> 64
gacgagacgg gacccactcg gcgcagaccg acatgtcggt ggcgtacagc ctggaggagt 60

acgcgggcgc cgtggtggcc gccgtgggcc gcgtgtgtga ccgcaagggg gtgcagcacc 120

ccatcatctg cagcgagagc ggccgcgcgc tggtgtcgca ccactcggtc ctggtgttcg 180

aggccttctc ggctacggcg ccgggacgcc tggacgcggc cacggcctac ctcctcgacg 240

agctcacgga cgactgccgc gccgactacc gcaacgtgat ggccgccgcc gtgcgcggcg 300

actacgacac gtgcggcctg tacgccgacc agctgaagcg gcggtccgcg gagcagttca 360

aggagggggt ccttggcctg gagcacctcg ccgcggtgga cgccttctgc gagctcgtcg 420

cccgcggcat gggcgcgccc gagccgccgc gcacctacca catcaacctg tccgtgttca 480

cctccctgcc ggacatgtgg gccatcgggc agcagttccc catcatcccg atccagcggc 540

tgcaggagcg gcccgccgtc gacggcgtgc tctcggacct cacctgcgac agcgacggca 600

aggtgagcga gttcatcggc gggaggcaca gcctgccgct gcacgagctg cccacccacg 660

ccactcgcgg ctactacctg ggcatgttcc tcgggggcgc ctaccaggag gccctcggcg 720

ggctgcacaa ccttttcggc gggcccagcg tggtgcgcgt gtcccagtgc gacggccccc 780

actgctttgc cgtgacgcgc gccgccgcgg gcccgtcctg cgccgacgtg ctccgcgcga 840

tgcagcacga gcccgaggtg atgtacgagg tgctgaagca gaggaccgac ggcgccacgg 900

ccgccgccct cgcccgggcc ttcggcgcaa tgccgtacct ggtgttcgac cccgaggccg 960

ccgtgatgtc cggcggcgag agcagcgact cggagcggtc ggccgccggc gccgccgagg 1020

aggaagacga tgaggagtgg gagttcatgc gcgggctcac cgtctgatga cgtgcccgcc 1080

cgcccgcccc gggtgaaaca aggcgagtcc gtcgccagga agttgtcagt ggccggccgg 1140

cgttggttta cataataacc cccaccgccc ggtgttgtat cgtgtcgtgg tcgtagtcgt 1200

cgggtgtctc gtcgtcgccg gagttgtttg gcgccacctt tttattccga acgcgtcagt 1260

gtccactgtt cgcctttcct tttttttctc tctctttcat gggaataatc tgaaggaaac 1320

taatagcaag tttctcttta actggg 1346

<210> 65
<211> 1336
<212> DNA
<213> Zea mays

<400> 65
cgcagatcta ctgcgagctc gcccgcctcg gcgcggacat gcgcgtcatc gacgtcggcg 60

gcggcctcgg cattgactac gacgggaccc actcggcgca gaccgacatg tcggtggcgt 120

acagcctgga ggagtactct tttcgccgtg gtggccgccg tgggccgcgt gtgtgaccgc 180

aagggggtgc agcaccccat catctgcagc gagagcgggc gcgcgctggt gtcgcaccac 240

tcggtcctgg tgttcgaggc cttctcggct acggcgccgg gacgcctgga cgcggccacg 300

gcctacctcc tcgacgagct cacggacgac tgccgcgccg actaccgcaa cgtgatggcc 360

gccgccgtgc gcggcgacta cgacacgtgc ggcctgtacg ccgaccagct gaagcggcgg 420

tccgcggagc agttcaagga gggggtcctt ggcctggagc acctcgccgc ggtggacgcc 480

ttctgcgagc tcgtcgcccg cggcatgggc gcgcccgagc cgccgcgcac ctaccacatc 540

aacctgtccg tgttcacctc cctgccggac atgtggccca tcgggcagca gttccccatc 600

atcccgatcc agcggctgca ggagcggccc gccgtcgacg gcgtgctctc ggacctcacc 660

tgcgacagcg acggcaaggt gagcgagttc atcggcggga ggcacagcct gccgctgcac 720

gagctgccca cccacgccac tcgcggctac tacctgggca tgttcctcgg gggcgcctac 780

caggaggccc tcggcgggct gcacaacctt ttcggcgggc ccagcgtggt gcgcgtgtcc 840

cagtgcgacg ggccccactg ctttgccgtg acgcgcgccg ccgcgggccc gtcctgcgcc 900

gacgtgctcc gcgcgatgca gcacgagccc gaggtgatgt acgaggtgct gaagcagagg 960

accgacggcg ccacggccgc cgccctcgcc cgggccttcg gcgcaatgcc gtacctggtg 1020

ttcgaccccg aggccgccgt gatgtccggc ggcgagagca gcgactcgga gcggtcggcc 1080

gccggcgccg ccgaggagga agacgattag gagtgggagt tcatgcgcgg gctcaccgtc 1140

tgatgacgtg cccgcccgcc cgccccgggt gaaaccaggc gagtccgtcg ccaggaagtt 1200

gacaatggca gaaaaacgtt ggtttacata ataaccccca ccgcccggtg ttgtatagaa 1260

tcgtggtcgt agtcgtcggg tgtctcgtcg tcgccggagt tgtttggcgc caccttttta 1320

ttccgaacgc gtcagt 1336

<210> 66
<211> 5474
<212> DNA
<213> Zea mays

<400> 66
ggacaatgta aacggatggt aaaacagcat ggtgaactcc ccaccaaaca cccatctgtc 60

tgcaatcact gggaaacaga tgtttttatt tctaaatcaa gcaggcaact actcagatac 120

ccttctccag catcgcccgg acccgtgtcg gtaggccgta cagcctgatg aacccctcgg 180

cgtcagcttg gttgtagatc tccccgttct caaacgagga gatatcttcc ctgtacaggc 240

tgtaggggct cttccgggac gcgacgttca cggaaccctt gtacagcttc agggtcactg 300

aaccagtggt cgtcgcggta accttctcca tgaaggcatc aaaggactgc cggagcgggt 360

caaaccagcg gccagcatag accagctcgg cgtacttgag ggcaatgatg tccttccact 420

gcatggtctc cctgtccagg gtcagggact ccaactcgcg taccgcggct gccatgatgg 480

tgccaccggg ggtctcatag accccacgtg acttcatgcc gaccagcctg ttttccacca 540

tgtcgatacg cccaatccca tgctttccac cgatttcatt gagctccgcg aggagggatg 600

ctggtgagag gtctcggcca ttgattgaaa caggaacacc agcaatgata ccaatctcca 660

aatacctgaa tgtatgcggc tcggcattaa tctatcacgg tatgcataca tatgcatcta 720

gtgtacaaat ggaaaaaaaa cctatgttta tactgatcat attaagttcc aggccaatcc 780

tgctttggat ggatccaact ctggaatacc aggtgcagta atgctcaatt gtgcatacac 840

agtaaactgt gctacagatt gcagcaaaaa tgaaaaaaaa aagagcggaa agatttaata 900

ttgatcactc actcaggttt cgagggcgca ttttctggag caatcgacat catatacata 960

tcttcctttg gctcatttgc tgggtcctcc aggatgtcac cctgcaatgt agagaatgat 1020

tcacttcatc tcgcatcaaa gatgggcgtc atgttgtttg gttaaaggtg tcgacaagtg 1080

caggacatta attcagaaca ctaatgtggc attttgcata tattggctta aaagttggaa 1140

cgtaaccatc ttgtaaagtt ctggtaacta agattctgtc ctacatgtaa agaaaaagat 1200

attaaactac aaaaaatgct ttgataaaac ttgaagcaat aaacatgaaa aatggacaat 1260

acctcatggc taaggtgcca caagtttcgg tctcggctgt atattgattt ctttgaaact 1320

ggaacaggta cattatgttt ctttgcatat tcaatagcat cttcacgtcc tgtgatatcc 1380

cactccctcc aaggtgccac cactttaagt tcaggattta aagcatagaa tgtaagctca 1440

aagcgaacct gcagaataag ggtagcatta caagatgacc agacacatta atatacaggc 1500

taacagcgtc actacctgat cattgccttt tccagtgcaa ccatgggcaa ctgcatccgc 1560

accaacttcc ttggcaacat caaccattgc ctgcagtgga gatataatgc cataaaactt 1620

ggagcattca aaagagtaag agtgtattaa tgctgccaac acaatcccac ctacagatcc 1680

taaagaatat atgcagcatt cagaacatta tttgaagcat atttcaatat tttatttcaa 1740

ctaaataaaa gaaacagcac gccctacccc atgatattct tgctccacta gttctgcatg 1800

atgcccataa gattcacagt aggttaactg gacagatcat tccaagccaa acaacagctg 1860

agccacctac tctacagggc tagcagacca aatcaacaaa acaaagatga tcatccaaag 1920

cttctaaggc ttgtacctga gacttttatg aaacaaatat gtaaagacaa atttgccaag 1980

tctatgtaca ccaagaagca taccatgtac tattggaaca gatttgatat atgctataaa 2040

agagtaaatg gctaccaaaa gttacgtcaa gtttacatca gcaactttgg tgctgcagta 2100

gcgattataa ttcaaatgtt atctctacca tcatgtgcat ttaatttaca gtacactgta 2160

aaccatgaca tcaatcaaat ctcaaggaat tatgcaaaca tcagaaatta cttttcaaga 2220

aattcatgca gattatttgt gattatacaa taacaagatg cagctattgt attaatttca 2280

ccttagcaat aacaggccta gccatcgaag tcccaagcag atactttctt tcataaactg 2340

cacctgcacg caggcagggg tatatatatt cactaacaaa ttcttctttg aggtccttca 2400

caacaagctg acatgcacca ctggcttttg ccttcttctc caacccttcc aattcaatgg 2460

caccctaggg agggttaagt aaatgtattt aaacagatgg ttagatacaa gaaaatggat 2520

agaaaatcca agccatgaca ctgcctattt acatattgac aaccaaaaaa taataaatca 2580

ctaggaagat taaaaatata aaaaggattc acaacttcat aaattaggac aatacaaagc 2640

catagaaaca ataggtgaag aaactattag ttttggaact cctggcagga atgcaaccag 2700

cagaaacatt atgcaagtat gtgaaataag aaagagacat gggagggaaa gaaacaggaa 2760

tatgttggct agacattaca agcttcagaa taggccaagc aataatatta ggaaacatac 2820

ctggccaaca tcagcagtga aacaaacgac ttcacaacca tagttctccc ttgaattagt 2880

aaaccaaaaa aaataaagct tgcagaagtt agacaaatgt agacttcaaa cttcgtaaaa 2940

gacaattaaa ataaggaata tatatacctg agccatggaa caataacgga tgtatccaaa 3000

ccaccactat aagctaaaac aactttgttt aattttccac gcaaaccgcc cttggtttga 3060

tcaccgctac tggcagtgga aattgcaccc tgctcttttc cagttgccat tgcacacctg 3120

atagctgtgc atcgttcata aagatacaga agttattatc atagtaatag aaaattgcta 3180

tttgaggtag cagttacaaa attagttttt tttctcgaag gcgcagggga actgcgcctt 3240

aaatatatta agaagaccag aggttacagc aaggaactcc ttacggaggc cagcaatgga 3300

aaccggactc aaaaaactac tacagaaaca gggggggggt gtcagacaaa ggagctgcca 3360

aggagaccct tggccccagc taattcccaa agtcttctcc cgtcgtcggc ctgccgaagg 3420

atagaggaga gactgggagc ctgaccatca aacacacatc tattgcggtg attccaaatc 3480

atccaggccy ccgaagagat cagagatctc aaacctttct tgaccattaa ggatgtcagg 3540

cccagctggg gagcaaggct atgcaaccca aactttctga gcaatttata ccagaagatc 3600

cttgcaaaca cacatgagac cagcaggtgg ttaatagatt caggttcttg gtcacataac 3660

ggacagctgc ttggatgcat gcccctcttt gccggtctgt cagcagtcca gcacctgtta 3720

tgggccacaa gccataggaa gaaacgacat tttgatggag cccaagtttt ccagaccctc 3780

ttgtattgac cagatgtaca ggatcctagg aaaagaccct tgtaagctgc tttcgccgaa 3840

tactttccat ttggggctac actaaagata tgtctatctt caatgtgagg gtgcagctca 3900

atttcagaca gacaggccca gagctttaag caatctacca aaaccccaac agtaagacca 3960

cctctaatgt cagatattca tcttctgttt agcaaacctc tgatggtctc caggacctcc 4020

tgttctgcaa agggggagtc caagacagcc aggttgtgtc taggaaagcc aagagcttcc 4080

aggtcaactg tcatatcctc tgtccaccct gcccaatgag atttttgtag aagaggtcca 4140

ctaccgctgc tttttcacca tgctcagtta gaatatggtc tccataaatc agcctggcaa 4200

caaagttttt cctcttgcga ataggttgtc actaaaattt cctattacaa gcagtaagaa 4260

gctatttagt cgtacagtta agttaagtta acgaagaaca gcaatattgc aggaccaccc 4320

ttccagaaac tctttcaatc cacccttttt gcctaaaatg gttggtccat atactccatt 4380

agatgagaat aagccagagt ccaccaaagt gaaaaataca aagattgaac aaaagatgtg 4440

ttcaattaga ttcttagtca tgtcccgtta aggcccatat ggccccatga ggctctctat 4500

atacacatag cttatggtaa ttaagtcaag aattatggta agggttaaac taatttgacc 4560

cacccgatcc attttttccc cttgagggga tccttcctct ggcatttctg gtttggcccg 4620

tcgccatgct ttctttcttg tgccycatca ccatccatcc acagtctttg tcgcctgaac 4680

ttcatccacg ccaaccccac tacatctagt gtggtccgcc tctgtctgtg cggccattgt 4740

caagcgcgga cttcattcat gtggtccatc tccgtccgca ctgcttcatc acagattatc 4800

tgccttcatc cactcatcgc cttcacaatg tggacctgtc cacaccccgg cttccaggga 4860

cccgccttca cgcaggatgc gtccatttgc atttttcctc catgccaccc gccagtcgcc 4920

ccctgcttcg tgggacactg cacgcacgcc tgcagtccgg aggccatgat cttgctaaaa 4980

ctgtagactt ttggagtttt agaaactcaa ccctagacct cttttttcta aactatggtt 5040

ttgcacattc tagattctaa aattacaatt ttctaatacc aaagtttctg aaaactacag 5100

cattaaaaaa ggccataagt tttcaacatg ccatcaattc atattagatg acttacaatg 5160

attttgttgt ttatcatttc ctcttttctt tttggctgaa gactataaaa aatgaactaa 5220

agagaagaca gtccatgtgg aattctgtac actgggaact ggtggagcct gctgttagta 5280

tcagaacttt gagggctcgc atggtaagct acttctgttt gactgtatat ctgttccaat 5340

cttcaataga cgaattaaca tactgtggta tgtgcggtgt acatgctaaa tttttgaact 5400

tcaatataat ctctctgata aatttatatt tgttgtacta tgctttcctg attggataca 5460

gttggaatgt tgtt 5474

<210> 67
<211> 1838
<212> DNA
<213> Zea mays

<400> 67
attcggcacg accctctccg tagtcctact cttgtccgac ccgcgcggcc gccgccagcc 60

cgcgccctcg gagccccctt ccgcttctcc tctccgggaa tggcgtcggc gcgaggacct 120

tgcggaatta gtgtccgttc tgacttcagt tcagcctgcc ttattcacaa ggctggagcc 180

ccatcgaatt ctgtttctgg cgtgatggct gttcaggttc accgccaccg taacaaggtc 240

cgcaatggcc aagctatcag gtgtgcaatg gcaactggaa aagagcaggg tgcaatttcc 300

actgccagta gcggtgatca aaccaagggc ggtttgcgtg gaaaattaaa caaagttgtt 360

ttagcttata gtggtggttt ggatacatcc gttattgttc catggctcag ggagaactat 420

ggttgtgaag tcgtttgttt cactgctgat gttggccagg gtgccattga attggaaggg 480

ttggagaaga aggcaaaagc cagtggtgca tgtcagcttg ttgtgaagga cctcaaagaa 540

gaatttgtta gtgaatatat atacccctgc ctgcgtgcag gtgcagttta tgaaagaaag 600

tatctgcttg ggacttcgat ggctaggcct gttattgcta aggcaatggt tgatgttgcc 660

aaggaagttg gtgcggatgc agttgcccat ggttgcactg gaaaaggcaa tgatcaggtt 720

cgctttgagc ttacattcta tgctttaaat cctgaactta aagtggtggc accttggagg 780

gagtgggata tcacaggacg tgaagatgct attgaatatg caaagaaaca taatgtacct 840

gttccagttt caaagaaatc aatatacagc cgagaccgaa acttgtggca ccttagccat 900

gagggtgaca tcctggagga cccagcaaat gagccaaagg aagatatgta tatgatgtcg 960

attgctccag aaaatgcgcc ctcgaaacct gagtatttgg agattggtat cattgctggt 1020

gttcctgttt caatcaatgg ccgagacctc tcaccagcat ccctcctcgc ggagctcaat 1080

gaaatcggtg gaaagcatgg gattgggcgt atcgacatgg tggaaaacag gctggtcggc 1140

atgaagtcac gtggggtcta tgagaccccc ggtggcacca tcatggcagc cgcggtacgc 1200

gagttggagt ccctgaccct ggacagggag accatgcagt ggaaggacat cattgccctc 1260

aagtacgccg agctggtcta tgctggccgc tggtttgacc cgctccggca gtcctttgat 1320

gccttcatgg agaaggttac cgcgacgacc actggttcag tgaccctgaa gctgtacaag   1380

ggttccgtga acgtcgcgtc ccggaagagc ccctacagcc tgtacaggga agatatctcc   1440

tcgtttgaga acggggagat ctacaaccaa gctgacgccg aggggttcat caggctgtac   1500

ggcctaccga cacgggtccg ggcgatgctg gagaagggta tctgagtagt tgcctgcttg   1560

atttagaaat aaaaacatct gtttcccagt gattgcagac agatgggtgt ttggtgggga   1620

gttcaccatg ctgttttacc atccgtttac attgtccaaa ccaaaatatt atcattgcta   1680

catcagctgc ttttctcatt ggtttatatt gagcagacca ttctggtcga ttatcgtcat   1740

ctattgatct gcgataacgt tggagcgatg tctatctatg acttaagata ccttgacgat   1800

tcgaactgat acaccgttca tgccattaca caaatttc                           1838

<210>  68
<211>  803
<212>  DNA
<213>  Zea mays

<400>  68
tacccgcctc gaagatccct acatcatcat cgacaccgag ctccatcgga cagacgccga     60

ccgaggcccc aacctcctcc tccacccagc gtcgcgtcca gtgtggcccc gccgctgatt    120

atcgaagcca agggcttgag cgcagcgcgg gaacaggagg gcgagctgag gtggcgacga    180

attctcccac ctctcgtcgc ccacgcccgc tggatcccac tgtcgccgtc tctgcctcct    240

ctctgtatcc tcgcccgcag agaggaatta cttgagcagg gagatgagga gggcaggagg    300

cccatggctc gccggagcaa cccatgggca agaaggcaca cgtcgattag gtggatgggg    360

ttctagggag gcgcttcctc aggagctatc caaaatccta ctagatggag aagatcatgg    420

caacaaccat cgtatcgatg accctgaaga cgctggttta acttgctcca acagtcctac    480

tagatggaga agatcatgac aacaaccatt ggatcggtga ccctaaagtt gtacaagggc    540

ttggtgagtg tcgcatccct gaagagccct ttcatccatg caaggagaca tttcctcgtt    600

tgaggacgag gaaatctata tccaggctaa cgacgagggg tcatcttgct ctggtctccc    660

aaccggggtc tgggcgatgc tagagaagag catctgactc tttcttctct gttggattta    720

gaaatgaaat tcgttcttgg gaaactgttg aagtgagtga tcactgagga atgggtataa    780

ttatggaaat ttgtgcttcc ccc                                            803

<210>  69
<211>  1266
<212>  DNA
<213>  Zea mays

<400>  69
ttaagccgcc tccgctacgc gcaaggacgc cgccgccgct cagcagagag cagaccgcac     60

tccgccccct cccaaattcc cactcccacc tctggttctc ctccgcagct cggatcatct 120

gctataatgg cgtctgctgc tggagacgca tcccgattcg tcggtaatcc gcagctagat 180

cgccccactt cgttccaatc cgtcctgtct tttcttctcg ttccgcccat gtttggcatt 240

gcgtgttttc cggacagagc cgtgttattt agacgcttag atatttctag gctttcgtac 300

gtgttcgcac tcgcacggct tccttgacaa aaccctaaac cctccgccgc gctgcagctc 360

cgacgtgcgc cttaggttct agaacaaggc gagtagcacg cctttacctc cccggctcgc 420

cttactacct aagcgtctcc taggcgacgc ttgaatacta tgtatatact ttgtttcaat 480

agttaacctg gtagtttgga ttttttttta aattctagct aacaatttgt ttaatacata 540

tgctgaactc ttgttaaggt gccattaaac tggaaggatt agagacggca aaagctagtg 600

gtgcatgcca gcttgttgtg aagtatctga aagaataatt tgttggtgaa tatacatacc 660

cctgcttgcg tgctggtgca atttatgaaa gaaagtattt gctggggact tcaatggcta 720

ggcctgttat tgctaaggca atggttgatg ttgccaagga agttgatgca gattgttgct 780

tgtggatgca ccgagaaagg caatgatcag gtatcagaag attggcatcg tcgcaggtgt 840

tcctgtttgc atcaacggat aagacgtgtc gcccacatcc gtctcctcgc tgagctcaat 900

gaaattggtg ggaagcatgg gaggagactt cctcgtttga tgacggggaa atctataacc 960

aggctgacgc cgaggggggg tcatcaggct ctggtctccc gaccggggtc cgggcgatgc 1020

tggagaaggg tatttgaatc tttctttgtt tgattcagag acaaaatccg ttcttgggaa 1080

atcgttgttg ggtcggtgat cacagacgga tgggaatcat gtggaatttt gtttacatgt 1140

tgactaacta tagttagttg accgtgcatc tgtttctata tatatcatat agctatacat 1200

tggtttgatg ggttatctaa aatatctatc ttttatatat agtacaaacc aacacaggct 1260

tggttt 1266

<210> 70
<211> 625
<212> DNA
<213> Zea mays

<400> 70
aattcggacg aggcttgtcc gacccgcgcg gccgccgcca gcccgcgccc tcggagcccc 60

cttccgcttc tcctctccgg gaatggcgtc ggcgcgagga ccttgcggaa ttagtgtccg 120

ttctgacttc agttcagcct gccttattca caaggctgga gccccatcga attctgtttc 180

tggcgtgatg gctgttcagg ttcaccgcca ccgtaacaag gtccgcaatg gccaagctat 240

caggtgtgca atggcaactg gaaaagagca gggtgcaatt tccactgcca gtagcggtga 300

tcaaaccaag ggcggtttgc gtggaaaatt aaacaaagtt gttttagctt atagtggtgg 360

tttggataca tccgttattg ttccatggct cagggagaac tatggttgtg aagtcgtttg 420

tttcactgct gatgttggcc aggcaatggt tgatgttgcc aaggaagttg gtgcggatgc 480

agttgcccat ggttgcactg gaaaaggcaa tgatcaggtt cgctttgagc ttacattcta 540

tgctttaaat cctgaactta aagtggtggc accttggagg gagtgggata tcacaggacg 600

tgaagatgct attgaatatg caaag 625

<210> 71
<211> 808
<212> DNA
<213> Zea mays

<400> 71
gttctgactt cagttcagcc tgccttattc acaaggctgg agccccatcg aattctgttt 60

ctggcgtgat ggctgttcag gttcaccgcc accgtaacaa ggtccgcaat ggccaagcta 120

tcaggtgttt tttttttact ggaaaagagc agggtgcaat ttccactgcc agtagcggtg 180

atcaaaccaa gggcggtttg cgtggaaaat taaacaaagt tgttttagct tatagtggtg 240

gtttggatac atccgttatt gttcgcatgg ctcaggaaga actatggttg tgaagtcgtt 300

tgtttcactg ctgatgttgg ccagggtgcc attgaattgg aagggttgga gaagaaggca 360

aaagccagtg gtgcatgtca gcttgttgtg aaggacctca aaaaaaaatt tgttagtgaa 420

tatatatacc cctgcctgcg tgcaggtgca gtttatgaaa aaaagtatct gcttgggact 480

tcgatggcta ggcctgttat tgctaaggca atggttgatg ttgccaagga agttggtgcg 540

gatgcagttg cccatggttg cactggaaaa ggcaatgatc aggtagtgac gctgttaccc 600

tgtatattaa tgtgtctggt catcttgtaa tgctaccctt attctgcagg ttcgctttga 660

gcttacattc tatgctttaa atcctgaact taaagtggtg gcaccttgga gggagtggga 720

tatcacagga cgtgaagatg ctattgaata tgcaaagaaa catagtgtac ctgttccagt 780

ttcaaagaaa tcaatataca gccgagac 808